# EUROPEAN PATENT SPECIFICATION

(11) **EP 3 397 758 B1**
(45) Date of publication and mention of the grant of the patent: **25.11.2020**
(21) Application number: 16826360.6
(22) Date of filing: 29.12.2016
(51) Int. Cl.: C12N 9/10

(54) **MODIFIED THIOLASES CAPABLE OF PRODUCING BRANCHED COMPOUNDS AND USES THEREOF**
MODIFIZIERTE THIOLASEN ZUR HERSTELLUNG VON VERZWEIGTEN VERBINDUNGEN UND VERWENDUNGEN DAVON
THIOLASES MODIFIES CAPABLES DE PRODUIRE DES COMPOSES RAMIFIES ET LEURS UTILISATIONS

(30) Priority: 29.12.2015 EP 15382676
(43) Date of publication of application: 07.11.2018
(73) Proprietor: Repsol, S.A., 28045 Madrid (ES)
(72) Inventor: BERNAL SÁNCHEZ, Vicente, 28935 Móstoles (ES); TORRES SALAS, Pamela, 28935 Móstoles (ES); MORREALE DE LEÓN, Antonio, 28935 Móstoles (ES); BARRERA BURGOS, Víctor, 28935 Móstoles (ES); GONZÁLEZ BARROSO, María del Mar, 28935 Móstoles (ES); LÓPEZ GALLEGO, Fernando, 20009 San Sebastián (ES); GAGO BADENAS, Federico, 28801 Alcalá de Henares (ES); SÁNCHEZ MURCIA, Pedro Alejandro, 28801 Alcalá de Henares (ES)
(74) Representative: ABG Intellectual Property Law, S.L.
(86) International application number: PCT/EP2016/082840
(87) International publication number: WO 2017/114897

(56) References cited:
- WO-A1-2008/124523
- WO-A1-2014/006203
- DATABASE UniProt [Online] 24 March 2009 (2009-03-24), "SubName: Full=Acetyl-coa acetyltransferase, putative {ECO:0000313|EMBL:CAX43676.1}; EC=2.3.1.9 {ECO:0000313|EMBL:CAX43676.1};", XP002757410, retrieved from EBI accession no. UNIPROT:B9WBA6 Database accession no. B9WBA6
- DATABASE UniProt [Online] 19 September 2003 (2003-09-19), "RecName: Full=Acetyl-CoA acetyltransferase IA; EC=2.3.1.9; AltName: Full=Peroxisomal acetoacetyl-CoA thiolase; AltName: Full=Thiolase IA;", XP002757411, retrieved from EBI accession no. UNIPROT:Q12598 Database accession no. Q12598
- DATABASE Geneseq [Online] 29 September 2011 (2011-09-29), "Plant cytoplasm localized yield increasing sequence SEQ:3392.", XP002757412, retrieved from EBI accession no. GSP:AZL81227 Database accession no. AZL81227 & WO 2011/061656 A1 (BASF PLANT SCIENCE CO GMBH [DE]; SCHOEN HARDY [DE]; THIMM OLIVER [DE];) 26 May 2011 (2011-05-26)
- DATABASE Protein [Online] 13 June 2014 (2014-06-13), "Acetyl-CoA acetyltransferase [Salinimicrobium xinjiangense].", XP002757413, retrieved from NCBI Database accession no. WP_029036843
- DATABASE UniProt [Online] 29 October 2014 (2014-10-29), "SubName: Full=Uncharacterized protein {ECO:0000313|EMBL:CDS03077.1};", XP002757414, retrieved from EBI accession no. UNIPROT:A0A077W6P1 Database accession no. A0A077W6P1
- DATABASE Geneseq [Online] 1 April 2010 (2010-04-01), "Ralstonia eutropha H16 variant beta-ketothiolase protein, SEQ ID:2.", XP002757415, retrieved from EBI accession no. GSP:AXV25529 Database accession no. AXV25529
- DATABASE Geneseq [Online] 1 April 2010 (2010-04-01), "Ralstonia eutropha H16 variant beta-ketothiolase protein, SEQ ID:1.", XP002757416, retrieved from EBI accession no. GSP:AXV25528 Database accession no. AXV25528
- DATABASE UniProt [Online] 3 October 2012 (2012-10-03), "SubName: Full=Acetyl-CoA acetyltransferase {ECO:0000313|EMBL:AJQ25424.1}; EC=2.3.1.9 {ECO:0000313|EMBL:AJQ25424.1};", XP002757417, retrieved from EBI accession no. UNIPROT:I9NK82 Database accession no. I9NK82
- DATABASE UniProt [Online] 9 December 2015 (2015-12-09), "SubName: Full=Beta-ketoadipyl CoA thiolase {ECO:0000313|EMBL:KPC64084.1};", XP002757418, retrieved from EBI accession no. UNIPROT:A0A0N0XYH7 Database accession no. A0A0N0XYH7
- WRENSFORD L V ET AL: "An acyl-coenzyme a chain length dependent assay for 3-oxoacyl-coenzyme a thiolases employing acetyldithio-coenzyme A", ANALYTICAL BIOCHEMISTRY, ELSEVIER, AMSTERDAM, NL, vol. 192, no. 1, 1 January 1991 (1991-01-01), pages 49-54, XP024823619, ISSN: 0003-2697, DOI: 10.1016/0003-2697(91)90181-R [retrieved on 1991-01-01]
- ALSTON ET AL: "Suicide substrates for mitochondrial enzymes", PHARMACOLOGY AND THERAPEUTICS, ELSEVIER, GB, vol. 12, no. 1, 1 January 1981 (1981-01-01), pages 1-41, XP025484680, ISSN: 0163-7258, DOI: 10.1016/0163-7258(81)90074-7 [retrieved on 1981-01-01]
- MIDDLETON B ET AL: "The synthesis and characterisation of 2-methylacetoacetyl coenzyme A and its use in the identification of the site of the defect in 2-methylacetoacetic and 2-methyl-3-hydroxybutyric aciduria", CLINICA CHIMICA ACTA, ELSEVIER BV, AMSTERDAM, NL, vol. 128, no. 2-3, 14 March 1983 (1983-03-14), pages 291-305, XP023397002, ISSN: 0009-8981, DOI: 10.1016/0009-8981(83)90329-7 [retrieved on 1983-03-14]
- YAMAGAMI SETSU ET AL: "Isolation and characterization of acetoacetyl-CoA thiolase gene essential for n-decane assimilation in yeast Yarrowia lipolytica", BIOCHEMICAL AND BIOPHYSICAL RESEARCH COMMUNICATIONS, ELSEVIER, AMSTERDAM, NL, vol. 282, no. 3, 6 April 2001 (2001-04-06) , pages 832-838, XP002236254, ISSN: 0006-291X, DOI: 10.1006/BBRC.2001.4653

## Description

### FIELD OF THE INVENTION

The invention relates to the field of bioengineering and particularly to enzymes with thiolase activity capable of generating precursors of 2-branched derivative compounds for industrial use.

### BACKGROUND OF THE INVENTION

Thiolases are a superfamily of enzymes catalyzing the transfer of a non-amino acid acyl group (for example an acetyl) by means of a Claisen condensation reaction to form a carbon-carbon bond. The thioester of a cysteine residue present in the active center thereof intervenes in the mechanism of reaction in all cases. These enzymes are involved in a number of metabolic pathways, including both biosynthesis (anabolic) and degradation (catabolic) pathways in eukaryotic and prokaryotic organisms.

The thiolase superfamily has been classified into three families according to functionality: (a) β-ketoacyl:acyl-carrier-protein synthases (KASs), (b) polyketide synthases (PKSs) and (c) the synthetic and degradative thiolase family. The two first groups are involved in fatty acid and complex polyketide metabolism, respectively. These two groups of proteins are mostly dependent on acyl carrier protein and involve carboxylation processes for substrate activation.

The third group is divided into two families: biosynthetic thiolases (EC 2.3.1.9) and degradative thiolases (EC 2.3.1.16), although both groups of enzymes are capable of catalyzing both the forward and reverse reactions under specific conditions.

Although their substrate specificity is different, the thiolase superfamily conserves one and the same type of thiolase folding, in which each monomer is formed by two similar halves having a sandwich-type βαβαβαββ motif. In terms of their quaternary structure, they are usually organized as dimers or dimers of dimers in biological medium. In the latter, they are dimers of dimers interacting through four β6/β7 loops, one for each monomer, and where the interactions are mostly hydrophobic-type interactions.

Enzymes belonging to the thiolase family have about 400 residues with an extensive loop of about 20 residues connecting two similar domains at the N- and C-terminal ends with βαβαβαββ folding. It is precisely this region which folds outwardly from the active center, forming a large part of the ligand interaction site. Catalytically relevant residues in these enzymes are located in the contiguous loops of the so-called α-3 helixes of respective N- and C-terminal domains (one histidine and two cysteines).

The general biosynthetic mechanism of reaction accepted for thiolases consists of two consecutive steps in which a covalent acylcysteine (Cys(Ac)) intermediate is generated (Scheme 1). Therefore, in a first step an acyl group (acetyl if substrate 1 is acetyl-CoA) is transferred to the catalytic cysteine (acceptor). In a second step a Claisen condensation step occurs between this catalytic cysteine and a new substrate 2 to obtain this substrate 2 acetylated in position 2.
(i) **Acyl transfer** 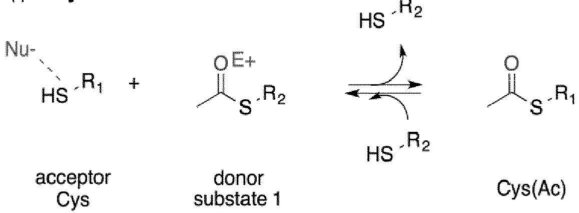
(ii) **Claisen condensation**

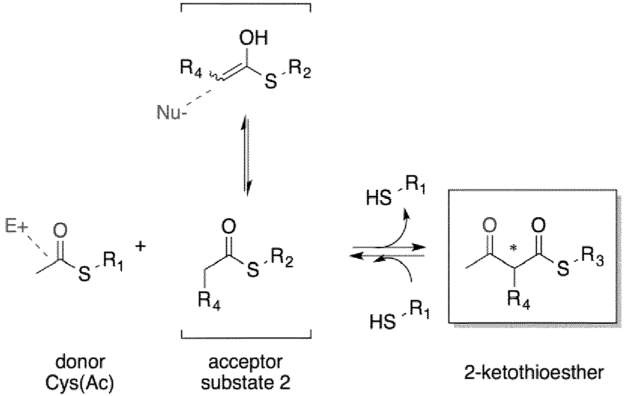

### Scheme 1.- Mechanism of the reaction catalyzed by thiolases.

The active center of thiolases is formed by two oxyanion holes (I and II) where the negative charges generated are stabilized throughout the catalytic cycle.

Compared to lineal products, branched products serve as a basis for obtaining a new family of compounds of industrial interest. In particular, branched products exhibit advantages as bio-fuels compared with the linear counterparts. However, most thiolases are not able to synthesize branched products.

Sequences provided in the GeneSeq database with accession numbers GSP:AXV25528 and GSP:AXV25529 and in the UniProt database with accession number A0A0N0XYH7 correspond to mutant thiolases capable using branched products as substrates.

Thus, there is a necessity to provide thiolases capable of synthesizing branched products to yield derivatives of interest in the industry.

### BRIEF DESCRIPTION OF THE INVENTION

In a first aspect, the invention relates to a mutant thiolase capable of catalyzing the formation of a compound of formula (I) 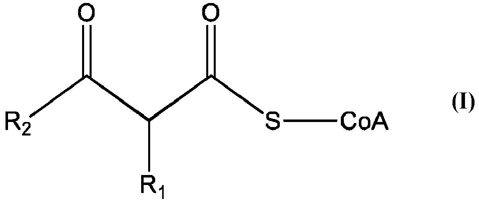 by the condensation of a compound of formula (II) 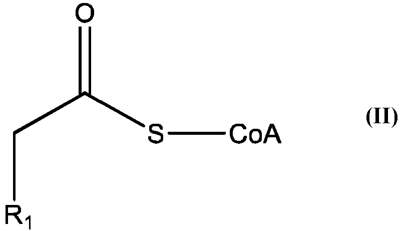 with a compound of formula (III) 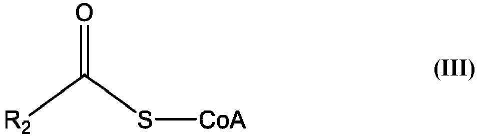
wherein R₁ and R₂ are independently selected from the group consisting of linear or branched C₁-C₆ alkyl, linear or branched C₂-C₆ alkenyl, linear or branched C₂-C₆ alkynyl, aryl, aryl-alkyl and benzyl, and S-CoA is coenzyme A,
said mutant thiolase having at least an amino acid substitution at a position corresponding to position 293 in the polypeptide of SEQ ID NO: 1 wherein the thiolase shows at least a 75% sequence identity with a thiolase selected from the group consisting of SEQ ID NO:1, 2, or 3, with the proviso that said thiolase is not a thiolase consisting of the sequence SEQ ID NO: 7 or SEQ ID NO: 8, and wherein said mutant thiolase displays a specific thiolase activity of at least 0,05mU/mg for the catalysis of a branched product of formula (I) when compound of formula (II) is butyryl-CoA and compound of formula (III) is acetyl-CoA,.

In a second aspect the invention relates to a nucleic acid encoding a thiolase according to the first aspect of the invention, or to a vector comprising a nucleic acid encoding the thiolase according to the first aspect of the invention, or to ahost cell comprising the vector comprising the nucleic acid encoding the thiolase according to the first aspect of the invention.

In a third aspect, the invention relates to a process for the production of a compound of formula (I) comprising contacting a compound of formula (II) and a compound of formula (III) in the presence of a mutant thiolase capable of catalyzing the formation of a compound of formula (I) 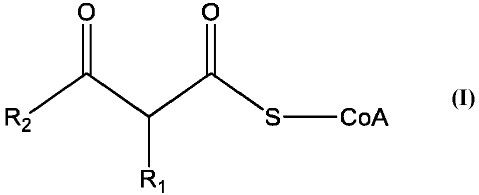 by the condensation of a compound of formula (II) 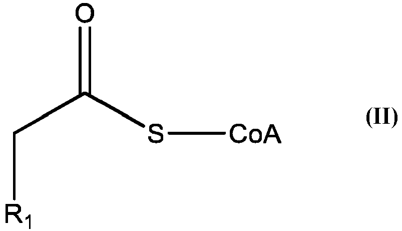 with a compound of formula (III) 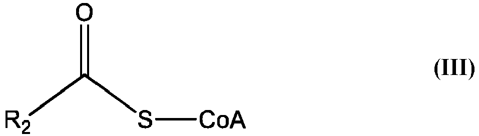
wherein R₁ and R₂ are independently selected from the group consisting of linear or branched C₁-C₆ alkyl, linear or branched C₂-C₆ alkenyl, linear or branched C₂-C₆ alkynyl, aryl, aryl-alkyl and benzyl, and S-CoA is coenzyme A,
said mutant thiolase having at least an amino acid substitution with respect to the naturally-occurring thiolase, wherein said amino acid substitution is located at a position corresponding to position 293 in the polypeptide of SEQ ID NO: 1;
wherein said thiolase has at least a 75% sequence identity with a thiolase selected from the group consisting of SEQ ID NO:1, 2, or 3 and displays a specific thiolase activity of at least 0,05mU/mg for the catalysis of a branched product of formula (I) when compound of formula (II) is butyryl-CoA and compound of formula (III) is acetyl-CoA, and wherein said contacting is carried out under conditions adequate for the condensation of compounds of formula (II) and (III) into a compound of formula (I).

### BRIEF DESCRIPTION OF THE DRAWINGS

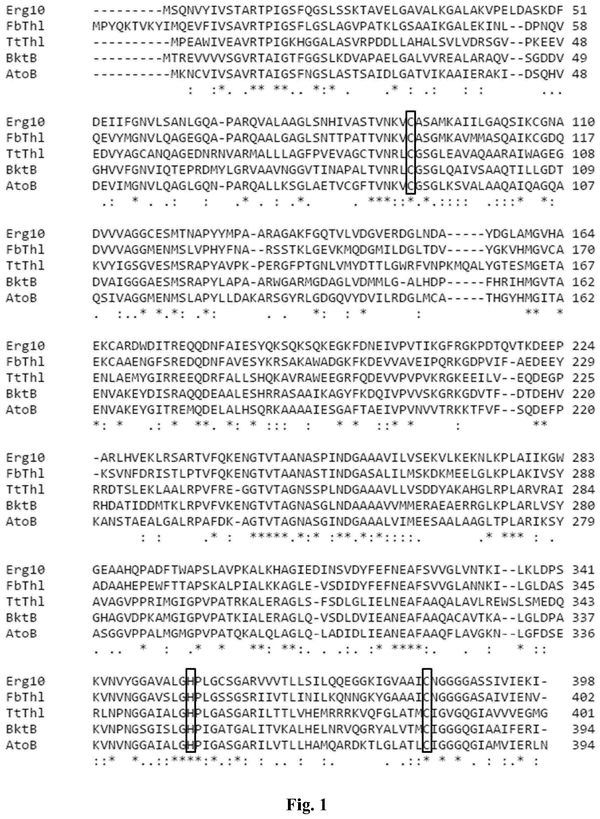

**Figure 1****.** Alignment of amino acid sequences of thiolases from *Saccharomyces cerevisiae* (SEQ ID NO:1, Erg10), *Flavobacterium bacteriaceae* (SEQ ID NO:5, *Fb*Thl), *Thermus thermophilus* (SEQ ID NO:4, *Tt*Tlh), *Ralstonia eutropha* (SEQ ID NO:2, BktB) and *Escherichia coli* (SEQ ID NO:3, AtoB). Highly conserved regions are marked with an asterisk; colon indicates conservation between groups of strongly similar properties; period indicates conservation between groups of weakly similar properties. Amino acids with a key role in the catalytic cycle are framed

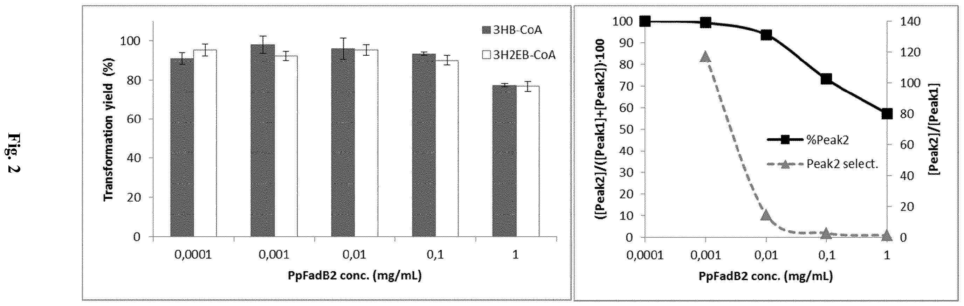

**Figure 2****.** (A) Yield of the reduction reaction of acetoacetyl-CoA and 3-keto-2-ethylbutyryl-CoA to 3-hydroxybutyryl-CoA (3HB-CoA) and 3-hydroxy-2-ethylbutyryl-CoA (3H2EB-CoA), respectively, as a function of PpFadB2 (SEQ ID NO:6) concentration. (B) Effect of PpFadB2 concentration on the stereoselectivity of the reduction reaction (expressed as [Peak2]/[Peak1] ratio) and on the production of the major "Peak 2" 3-hydroxy-2-ethylbutyryl-CoA isomer (expressed as [Peak2]/([Peak1]+[Peak2])·100). At the lowest protein concentration assayed (0.0001 mg/mL), only Peak2 isomer was detected.

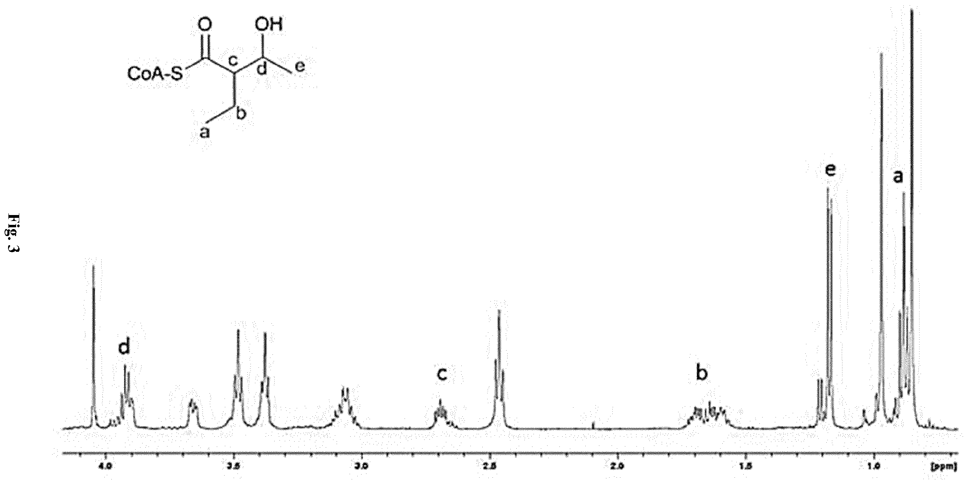

**Figure 3****.** ¹H-NMR spectrum of 3-hydroxy-2-ethylbutyryl-CoA.

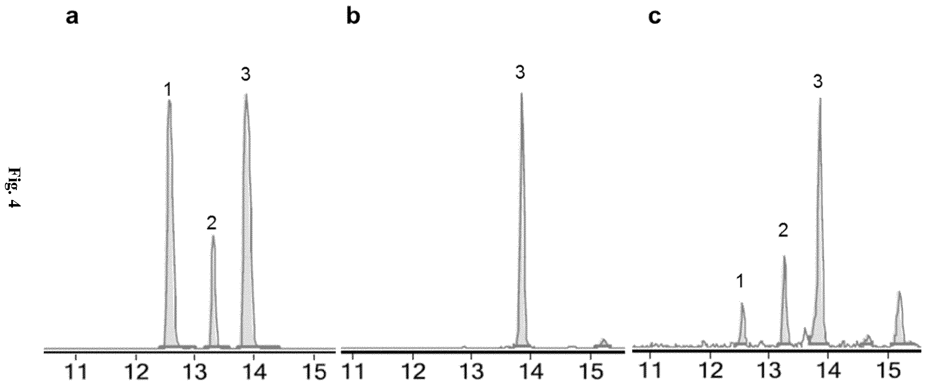

**Figure 4****.** Analysis of acetyl-CoA/butyryl-CoA condensation capacity of thiolases from *Ralstonia eutropha* (BktB). Total Ion Chromatogram (TIC) of (a) the standard mixture, (b) wild type BktB and (c) BktB_Var10. **Peak legend: 1:** 3H2EB-CoA Peak 1; **2:** 3H2EB-CoA Peak 2; **3:** 3HHB-CoA

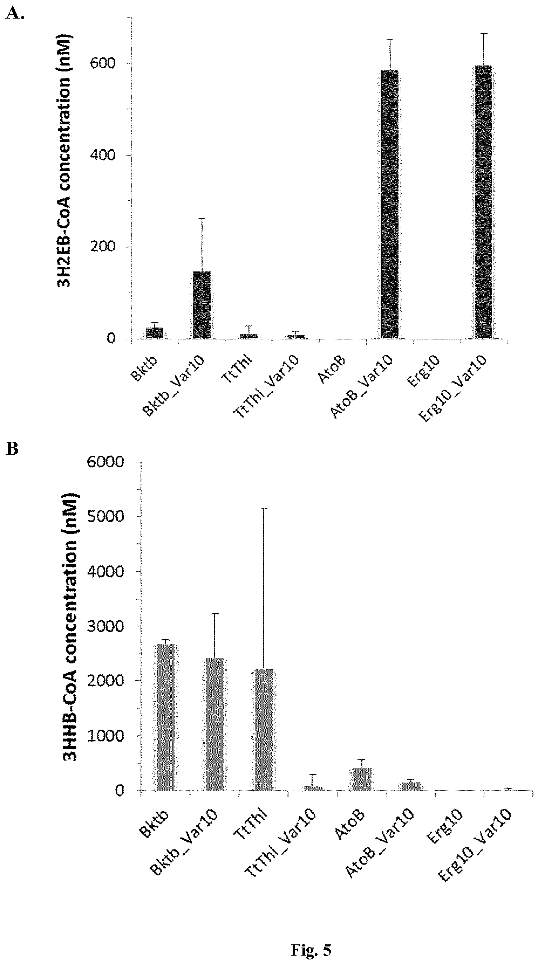

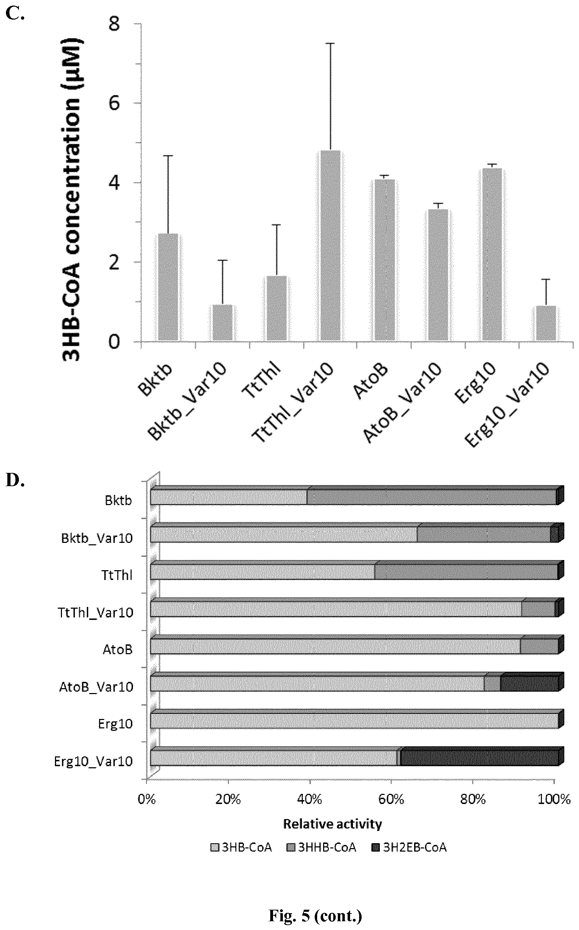

**Figure 5****.** Comparison of 3HB-CoA production yields of thiolases from *Saccharomyces cerevisiae* (Erg10), *Flavobacterium bacteriaceae* (*Fb*Thl), *Thermus thermophilus* (*Tt*Tlhl), *Ralstonia eutropha* (BktB), *Escherichia coli* (AtoB) and their corresponding VarlO (triple mutants). Reactions were carried out at 37°C in 100 mM MES buffer pH 6.0 using purified thiolase and FadB2 at a concentration of 0.19 and 0.15 mg/ml, respectively. Product formation was analyzed after 1h and quantified by HPLC-MS. A) 3-hydroxy-2-ethylbutyryl-CoA yield, B) 3-hydroxyhexanoyl-CoA yield C) 3-hydroxybutyryl-CoA yield. D) Product selectivity of thiolase variants in acetyl-CoA/butyryl-CoA condensation assays.

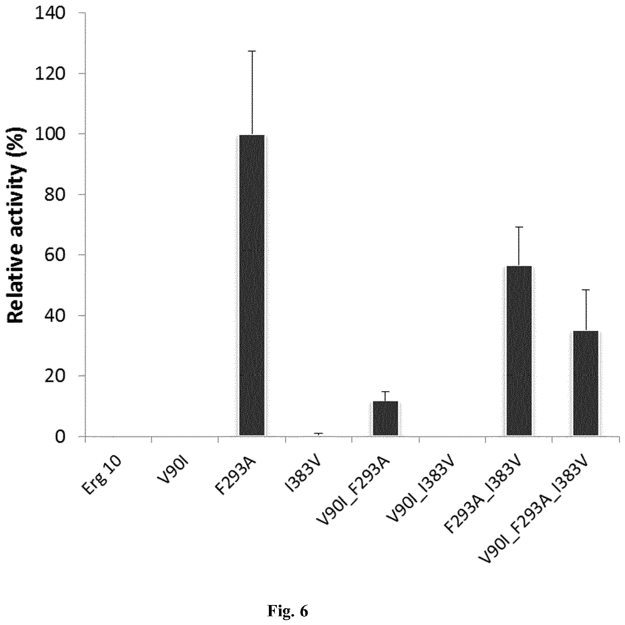

**Figure 6****.** Comparison of variants of Erg10 thiolase from *Saccharomyces cerevisiae.* (SEQ ID NO:1) Reactions were carried out at 37°C in 100 mM MES buffer pH 6.0 using purified thiolase and *Pp*FadB2 at a concentration of 0.19 and 0.15 mg/ml, respectively. Product formation was analyzed and quantified by HPLC-MS after 1h of reaction incubation.

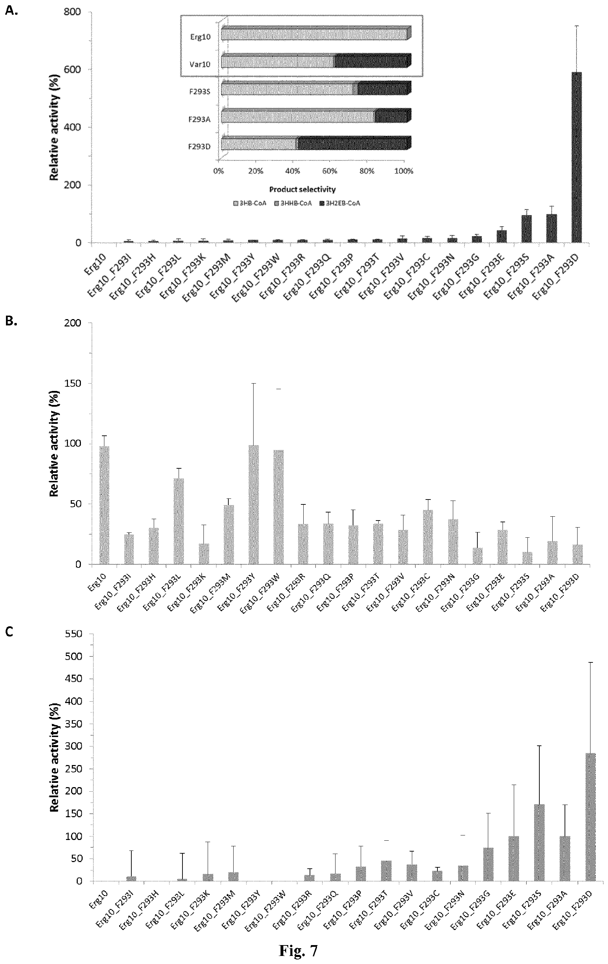

**Figure 7****.** Product yields of thiolases from *Saccharomyces cerevisiae* (SEQ ID NO:1, Erg10) modified at position F293. Reactions were carried out at 37°C in 100 mM MES buffer pH 6.0 using purified thiolases and *Pp*FadB2 at a concentration of 0.19 and 0.15 mg/ml, respectively. Product formation was analyzed and quantified by HPLC-MS after 1h of reaction incubation. (A) 3-hydroxy-2-ethylbutyryl-CoA (insert represents the product selectivity of selected mutants), (B) 3-hydroxybutyryl-CoA, (C) 3-hydroxyhexanoyl-CoA.

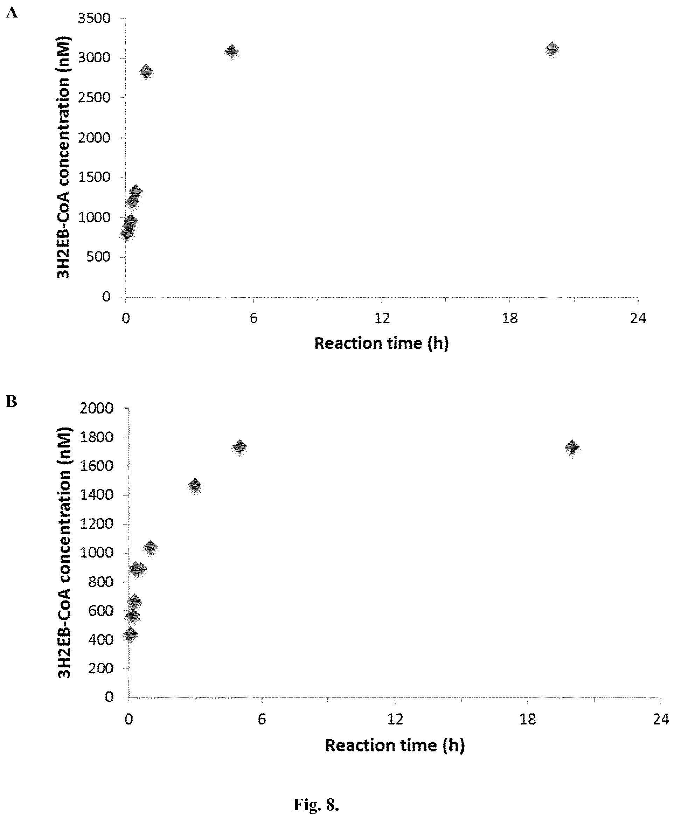

**Figure 8****.** Time course of product formation using (a) Erg10_F293D and (b) Erg10_F293A as biocatalyst. Reactions were carried out at 37°C in 100 mM MES buffer pH 6.0 using purified thiolases and *Pp*FadB2 at a concentration of 0.1 and 0.15mg/ml, respectively. Product formation was quantified by HPLC-MS.

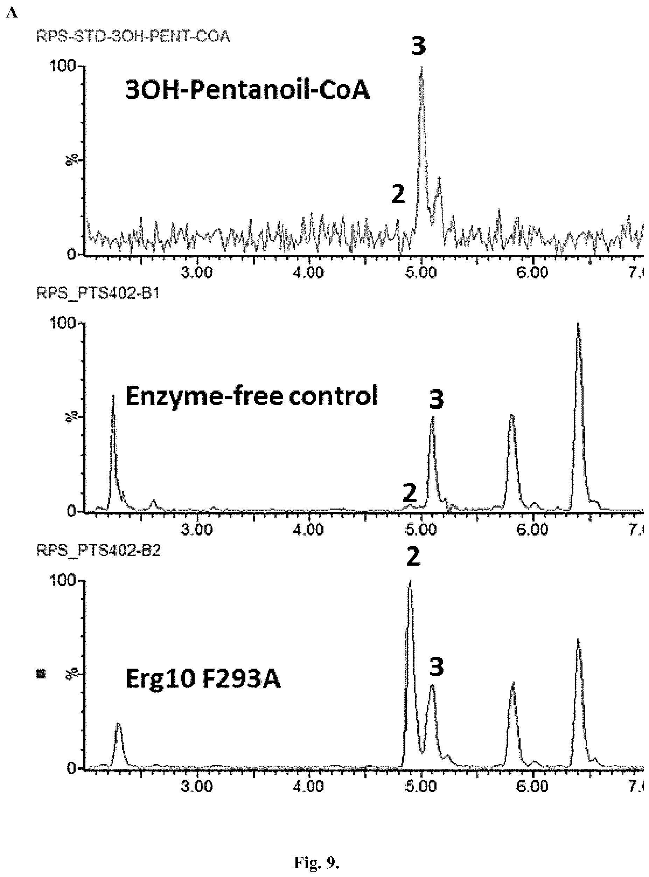

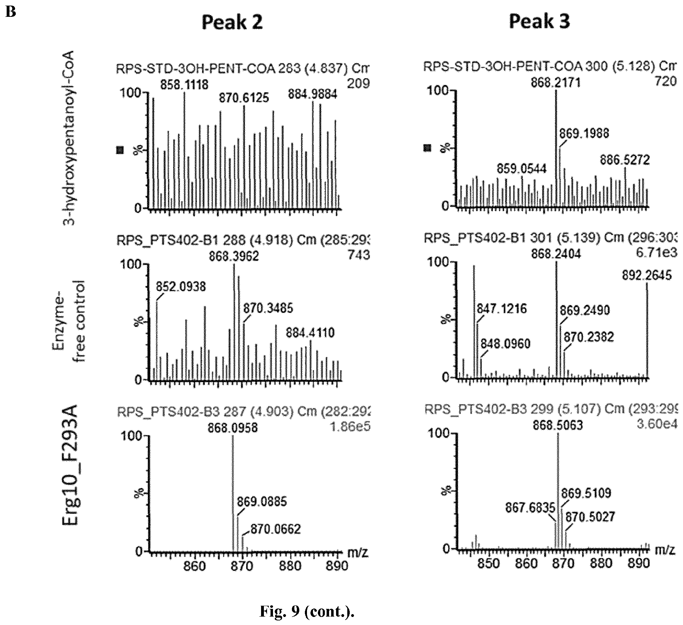

**Figure 9****.** A) LC-MS analysis of standard 3-hydroxy-pentanoyl-CoA (top), enzyme free control (middle) and Erg10-F293A reaction sample (bottom). B) MS analysis of peak 2 and peak 3.

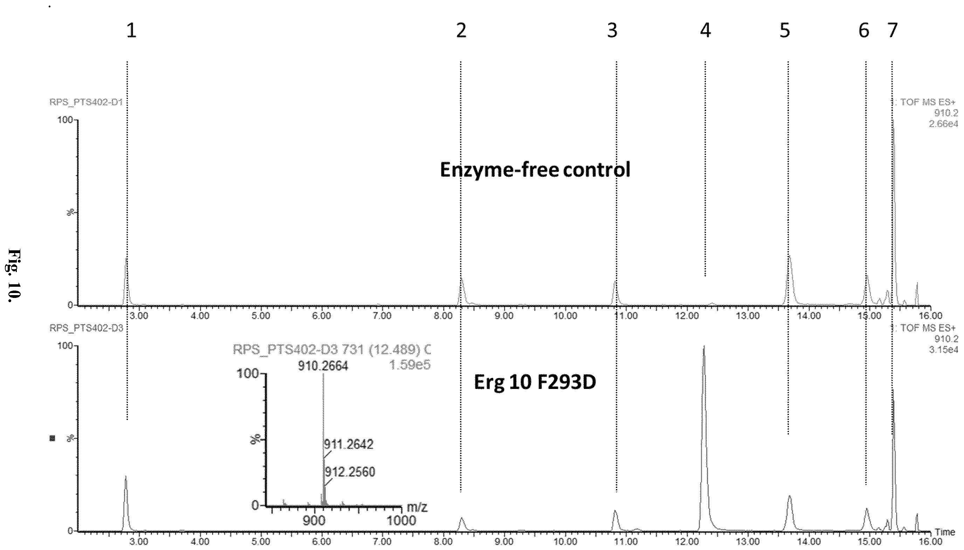

**Figure 10****.** LC-MS analysis of enzyme free control (top) and Erg10-F293D reaction sample (bottom). In the insert, MS analysis of peak 4.

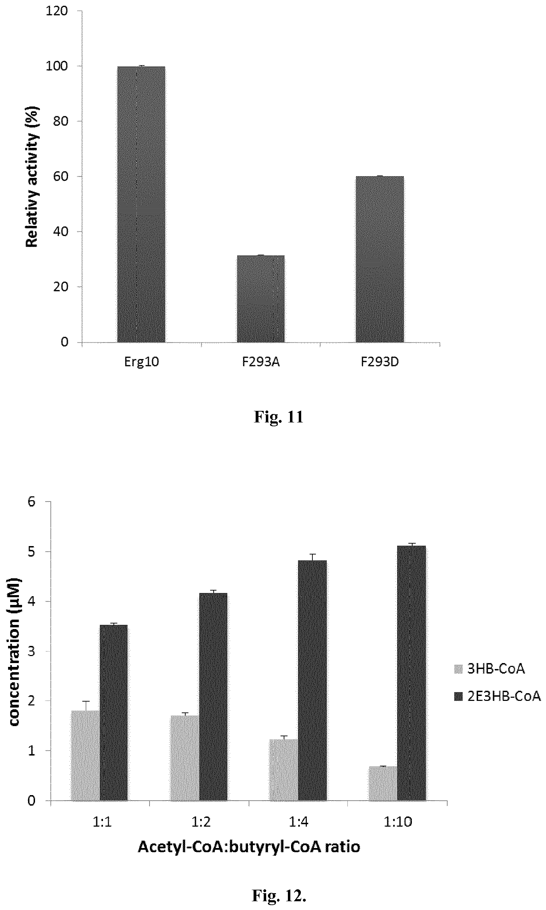

**Figure 11****.** Acetyl-CoA/acetyl-CoA condensation product yield of variants of Erg10 thiolase from *Saccharomyces cerevisiae* modified at position F293. Reactions were carried out at 37°C in 100 mM MES buffer pH 6.0 using purified thiolases and *Pp*FadB2 at a concentration of 0.19 and 0.15 mg/ml, respectively. Product formation was analyzed and quantified by HPLC-MS after 1h of reaction incubation. 3-hydroxybutyryl-CoA product yields were normalized by comparison with the product yield of wild type Erg10.
**Figure 12****.** Effect of acetyl-CoA:butyryl-CoA ratio on the product profile of Erg10_F293D variant as biocatalyst after 1h of reaction. Reactions were carried out at 37°C in 100 mM MES buffer pH 6.0 using purified thiolases and *Pp*FadB2 at a concentration of 0.19 and 0.15 mg/ml, respectively. Product formation quantified by HPLC-MS.

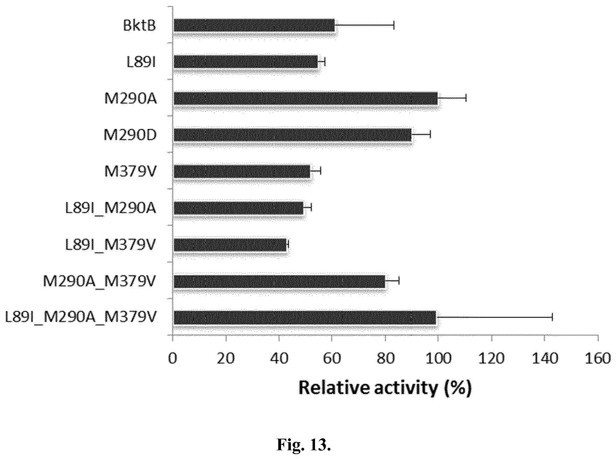

**Figure 13****.** Comparison of variants of BktB thiolase from *Ralstonia eutropha* (SEQ ID NO:2) Reactions were carried out at 37°C in 100 mM MES buffer pH 6.0 using purified thiolase and *Pp*FadB2 at a concentration of 0.19 and 0.15 mg/ml, respectively. Product formation was analyzed and quantified by HPLC-MS after 1h of reaction incubation.

### DETAILED DESCRIPTION OF THE INVENTION

The inventors of the present invention have identified mutations in thiolase enzymes which improve their biosynthetic activity and, more in particular, are capable of generating branched products.

Based on these findings, the inventors have developed the processes of the present invention in their different embodiments that will be described now in detail.

### MUTANT THIOLASES OF THE INVENTION

In a first aspect, the invention relates to a mutant thiolase capable of catalyzing the formation of a compound of formula (I) 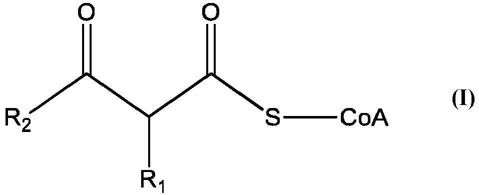 by the condensation of a compound of formula (II) 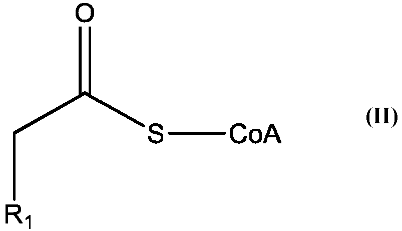 with a compound of formula (III) 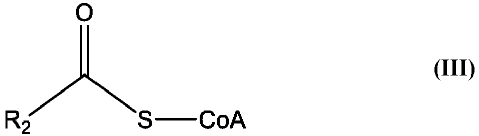
wherein R₁ and R₂ are independently selected from the group consisting of linear or branched C₁-C₆ alkyl, linear or branched C₂-C₆ alkenyl, linear or branched C₂-C₆ alkynyl, aryl, aryl-alkyl and benzyl, and S-CoA is coenzyme A,
said mutant thiolase having at least an amino acid substitution at a position corresponding to position 293 in the polypeptide of SEQ ID NO: 1 wherein the thiolase shows at least a 75% sequence identity with a thiolase selected from the group consisting of SEQ ID NO:1, 2, or 3, with the proviso that said thiolase is not a thiolase consisting of the sequence SEQ ID NO: 7 or SEQ ID NO: 8, and wherein said mutant thiolase displays a specific thiolase activity of at least 0,05mU/mg for the catalysis of a branched product of formula (I) when compound of formula (II) is butyryl-CoA and compound of formula (III) is acetyl-CoA.

As it is used herein, the term "thiolase" refers to polypeptides capable of condensing 2 molecules comprising acetyl-CoA (AcCoA) to yield molecules comprising acetoacetyl-CoA. The term includes both, those thiolases which naturally catalyze the condensation of, for example, 2 acetyl-CoA molecules (biosynthetic thiolases, also known as acetoacetyl-CoA thiolase or acetyl-CoA acetyltransferase, with EC number 2.3.1.9) and thiolases which naturally catalyze the formation of acetyl-CoA from acetoacetyl-CoA and coenzyme A (degradative thiolases, also known as 3-ketoacyl-CoA thiolases or 3-oxoacyl-CoA thiolases, with EC number 2.3.1.16), but which can catalyze both the direct and the reverse reactions under suitable conditions. Preferred thiolases according to the present invention include, without limitation, *Saccharomyces cerevisiae* acetoacetyl-CoA thiolase (Erg10, SEQ ID NO: 1), *Ralstonia eutropha* acetyl-CoA acetyltransferase (BktB, SEQ ID NO: 2), *Escherichia coli* acetoacetyl-CoA thiolase (AtoB, SEQ ID NO: 3) and the *Thermus thermophilus* acetoacetyl-CoA thiolase (*Tt*Thl, SEQ ID NO: 4).

As it is used herein, the term "mutant" refers to any variant of a thiolase as defined above which is capable of condensing two molecules comprising acetyl-coenzyme A (AcCoA) with butyryl-coenzyme A (BuCoA) giving rise to the formation of 3-keto-2-ethylbutyryl-CoA), which differs from acetoacetyl-CoA thiolase due to the presence of an addition, substitution or deletion of one or more amino acids and which shows a sequence identity with native thiolase that is at least 75%, at least 80%, at least 85%, at least 90%, at least 95%, at least 96%, at least 97%, at least 98%, at least 99% or higher with respect to any of the sequences defined as SEQ ID NO:1 to 4. In a preferred embodiment, the sequence identity is determined over the entire length of the sequence. The person skilled in the art appreciates that there are many established algorithms available to align two sequences. Optimal alignment of sequences for comparison can be conducted, e.g., by the local homology algorithm of Smith and Waterman, 1981, Adv. Appl. Math. 2:482, by the homology alignment algorithm of Needleman and Wunsch, 1970, J. MoI. Biol. 48:443, by the search for similarity method of Pearson and Lipman, 1988, Proc. Natl. Acad. Sci. USA 85:2444, by computerized implementations of these algorithms (e.g., GAP, BESTFIT, FASTA, and TFASTA in the GCG Wisconsin Software Package), or by visual inspection (see generally, Current Protocols in Molecular Biology, F. M. Ausubel et al., eds., Greene Publishing Associates, Inc. and John Wiley and Sons, Inc., (1995 Supplement)). Examples of algorithms that are suitable for determining percent sequence identity and sequence similarity are the BLAST and BLAST 2.0 algorithms, which are described in Altschul et al., 1990, J. MoI. Biol. 215: 403-410 and Altschul et al., 1977, Nucleic Acids Res. 3389-3402, respectively as well as the BLASTP algorithm (BLAST Manual, Altschul, S. et al., NCBI NLM NIH Bethesda, Md. 20894, Altschul, S., et al., J., 1990, Mol. Biol. 215:403-410). Software for performing BLAST analyses is publicly available through the National Center for Biotechnology Information website. This algorithm involves first identifying high scoring sequence pairs (HSPs) by identifying short words of length W in the query sequence, which either match or satisfy some positive-valued threshold score, T, when aligned with a word of the same length in a database sequence. T is referred to as, the neighbourhood word score threshold (Altschul et al, *supra*)*.* These initial neighbourhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4, and a comparison of both strands. For amino acid sequences, the BLASTP program uses as defaults a wordlength (W) of 3, an expectation (E) of 10, and the BLOSUM62 scoring matrix (see Henikoff and Henikoff, 1989, Proc Natl Acad Sci USA 89:10915). Exemplary determination of sequence alignment and percent sequence identity can employ the BESTFIT or GAP programs in the GCG Wisconsin Software package (Accelrys, Madison WI), using default parameters provided, or the multiple alignment program (available from the Swiss Institute of Bioinformatics, Lausanne, Switzerland), using, in some embodiments, the parameters below.

For purposes of the present disclosure, in some embodiments, the degree of percent amino acid sequence identity can be obtained by multiple alignment analysis, counting the number of identical matches in the alignment and dividing such number of identical matches by the length of the reference sequence, and using the following default parameters to achieve slow/accurate pairwise optimal alignments Output format: clustal w/o numbers; dealing input sequences: No; MBED-like clustering guide-tree: yes; Number of combined Iterations: default (0); Max guide tree iterations: default; max HMM iterations: default; Order: aligned.

The person skilled in the art will understand that the mutants according to the present invention showing a thiolase activity such as of acetyl-coenzyme A (AcCoA) condensation with butyryl-coenzyme A (BuCoA) can have reduced activity of acetyl-CoA condensation with respect to native sequences, such that the mutants according to the present invention can have a thiolase activity of acetyl-CoA condensation that is 99%, 98%, 97%, 96%, 95%, 94%, 93%, 92%, 91%, 90%, 85%, 80%, 75%, 70%, 65%, 60%, 55%, 50%, 45%, 40%, 35%, 30%, 25%, 20%, 15%, 10% or less with respect to native thiolase activity.

Thiolase mutants according to the present invention have specific thiolase activity of condensing compounds of formulas (II) and (III) which is of at least 0.01, at least 0.05, at least 0.1, at least 0.5, at least 1, at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 75, at least 80, at least 85, at least 90, at least 95 or at least 100 mU/mg when the compounds of formula (II), preferably butyryl-coenzyme A (BuCoA), and the compounds of formula (III), preferably acetyl-CoA, are used as substrates.

As it is used herein, the term "C₁-C₆ alkyl group" refers to a linear- or branched-chain saturated non-cyclic hydrocarbon having 1 to 6 carbon atoms. Representative linear C₁-C₆ alkyl groups include, without limitation, methyl, ethyl, *n*-propyl, *n*-butyl, *n-*pentyl, *n*-hexyl; whereas branched C₁-C₆ alkyl groups include, without limitation, isopropyl, *sec*-butyl, isobutyl, *tert*-butyl, isopentyl, 2-methylbutyl, 3-methylbutyl, 2-methylpentyl, 3-methylpentyl, 4-methylpentyl, 2,3-dimethylbutyl, 2,2-dimethylbutyl, 3,3-dimethylbutyl and the like. Alkyl groups included in this invention can be optionally modified with one or more substituents.

As it is used herein, the term "C₂-C₆ alkyl group" refers to a linear- or branched-chain saturated non-cyclic hydrocarbon having 2 to 6 carbon atoms. Representative linear C₂-C₆ alkyl groups include, without limitation, ethyl, *n*-propyl, *n*-butyl, *n*-pentyl, *n*-hexyl; whereas branched C₂-C₆ alkyl groups include, without limitation, isopropyl, *sec*-butyl, isobutyl, *tert*-butyl, isopentyl, 2-methylbutyl, 3-methylbutyl, 2-methylpentyl, 3-methylpentyl, 4-methylpentyl, 2,3-dimethylbutyl, 2,2-dimethylbutyl, 3,3-dimethylbutyl and the like. Alkyl groups included in this invention can be optionally modified with one or more substituents.

As it is used herein, the term "C₂-C₆ alkenyl group" refers to a linear- or branched-chain acyclic hydrocarbon having 2 to 6 carbon atoms and at least one carbon-carbon double bond. Representative linear and branched C₂-C₆ alkenyl groups include, without limitation, vinyl, allyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, 1-hexenyl, 2-hexenyl, 3-hexenyl and the like. The alkenyl groups can be optionally modified by one or more substituents.

As it is used herein, the term "C₂-C₆ alkynyl group" refers to a linear- or branched-chain acyclic hydrocarbon having 2 to 6 carbon atoms and at least one carbon-carbon triple bond. Representative linear and branched C₂-C₆ alkynyl groups include, without limitation, ethynyl, propynyl, butynyl, pentynyl, hexynyl and the like.

"Aryl" refers to aromatic hydrocarbon groups such as phenyl, naphthyl or anthracyl. The aryl group can be optionally substituted by one or more groups such as hydroxy, mercapto, halo, alkyl, phenyl, alkoxy, haloalkyl, nitro, cyano, carboxy, amino, dialkylamino, aminoalkyl, acyl and alkoxycarbonyl.

As it is used herein, "aryl-alkyl" is defined as an alkyl group as defined above associated with an aryl group as defined above.

As it is used herein, "benzyl" is defined as the -CH₂-phenyl group, wherein the phenyl group can be substituted or unsubstituted.

In a particular and preferred embodiment of the invention, R₁ is an ethyl group. In another particular and preferred embodiment of the invention, R₂ is a methyl group.

As it is used herein, the expression "position corresponding to position X in the polypeptide Y" refers to a position in the thiolase sequence coinciding with position X when said thiolase sequence overlaps with polypeptide Y sequence in a multiple sequence alignment including the mutant thiolase sequence, the polypeptide Y sequence and a variable number of sequences. In a preferred embodiment, the multiple sequence alignment is formed by the aforementioned thiolase sequences (sequences having SEQ ID NO:1 to 4). In a preferred embodiment, the multiple sequence alignment is carried out using at least 10 thiolase sequences, at least 15 sequences, at least 20 sequences, at least 25 sequences or more. The multiple sequence alignment can be obtained by using any standard method for that purpose as mentioned above and as it is shown in Example 1 of the present patent application. In a preferred embodiment, the multiple sequence alignment is carried out by means of the algorithm implemented in the CLUSTALW2 program (Larkin et al., Bioinformatics, 2007, 23:2947-2948) using standard parameters (Alignment type: slow; matrix: Gonnet; gap open: 10; gap extension: 0.1; KTUP: 1; Window length: 5; Score type: percent; Top Diags: 5 and Pair Gap: 3). In another preferred embodiment, the multiple sequence alignment is carried out by means of the algorithm implemented in the CLUSTAL OMEGA program (Sievers F. et al., Biology 7 Article number: 539 doi:10.1038/msb.2011.75) using standard parameters (HHalign algorithm with default parameters and the default transition matrix is Gonnet with a gap opening penalty of 6 bits and gap extension of 1 bit.

According to the present invention, the mutant thiolase has at least one mutation with respect to the naturally occurring thiolase wherein said mutation is located at a position corresponding to positions 293 in the polypeptide of SEQ ID NO: 1 with the proviso that said thiolase is not a thiolase consisting of the sequence SEQ ID NO: 7 or SEQ ID NO: 8.

In a particular embodiment of the invention, the sequence in said thiolase corresponding to the thiolase signature having the accession number PS00098 in the Prosite database has an identity of at least 35% to the sequence corresponding to said signature in the thiolase of SEQ ID NO:1, the sequence in said thiolase corresponding the thiolase signature having the accession number PS00737 in the Prosite database has an identity of at least 50% to the sequence corresponding to said signature in the thiolase of SEQ ID NO:1 and/or the sequence in said thiolase corresponding the thiolase signature having the accession number PS00099 in the Prosite database has an identity of at least 25% to the sequence corresponding to said signature in the thiolase of SEQ ID NO:1.

The expression "the sequence in said thiolase corresponding to the thiolase signature having the accession number X in the Prosite database" refers to the sequence of the thiolase of the invention coinciding with the signature X in the Prosite database in a sequence alignment including the mutant thiolase sequence of the invention (i.e. the thiolase comprising the sequences SEQs ID NO: 1-4) and the signature having the accession number X in the Prosite database. The sequence identity can be determined by using any suitable algorithm available to align two sequences previously detailed.

The signature having the accession number PS00098 is a conserved thiolase signature which has the following consensus pattern: [LIVM]-[NST]-{T}-x-C-[SAGLI]-[ST]-[SAG]-[LIVMFYNS]-x-[STAG]-[LIVM]-x(6)-[LIVM]. The sequence corresponding to signature having the PS00098 accession number in the Prosite database is found in the polypeptide of SEQ ID NO:1 at positions 87 to 105 and corresponds to the subsequence
VNKVCASAMKAIILGAQSI

The signature having the accession number PS00737 is a conserved thiolase signature which has the following consensus pattern: N-x(2)-G-G-x-[LIVM]-[SA]-x-G-H-P-x-[GAS]-x-[ST]-G. The sequence corresponding to signature having the PS00737 accession number in the Prosite database is found in the polypeptide of SEQ ID NO:1 at positions 344 to 360 and corresponds to the subsequence
NVYGGAVALGHPLGCSG

The signature having the accession number PS00099 is a conserved thiolase signature which has the following consensus pattern: [AG] - [LIVMA] - [STAGCLIVM]-[STAG]-[LIVMA]-C-{Q}-[AG]-x-[AG]-x-[AG]-x-[SAG] The sequence corresponding to signature having the PS00099 accession number in the Prosite database is found in the polypeptide of SEQ ID NO:1 at positions 379 to 392 and corresponds to the subsequence
GVAAICNGGGGASS

Thus, according with this embodiment, the sequence in the thiolase of the invention corresponding the thiolase signature having the accession number PS00098 in the Prosite database has an identity of at least 35% to the sequence corresponding to said thiolase signature of SEQ ID NO: 1. In a more particular embodiment, the sequence in the thiolase of the invention corresponding to the thiolase signature having the accession number PS00098 in the Prosite database has an identity of at least 40% to the sequence corresponding to said thiolase signature of SEQ ID NO: 1. In another particular embodiment, said identity is of at least 45%, at least 50%, at least 55%, at least 60%, at least 65%, at least 70%, at least 75%, at least 80%, at least 85%, at least 90%, at least 95%, at least 99% or at least 100%. In a preferred embodiment, said sequence of the thiolase of the invention corresponding the thiolase signature having the accession number PS00098 in the Prosite database has an identity of about 37% to the sequence corresponding to said thiolase signature of SEQ ID NO: 1. In another preferred embodiment, said sequence of the thiolase of the invention corresponding the thiolase signature having the accession number PS00098 in the Prosite database has an identity of about 47% to the sequence corresponding to said thiolase signature of SEQ ID NO: 1. In a preferred embodiment, said sequence of the thiolase of the invention corresponding the thiolase signature having the accession number PS00098 in the Prosite database has an identity of about 58% to the sequence corresponding to said thiolase signature of SEQ ID NO: 1. In a preferred embodiment, said sequence of the thiolase of the invention corresponding the thiolase signature having the accession number PS00098 in the Prosite database has an identity of about 63% to the sequence corresponding to said thiolase signature of SEQ ID NO: 1.

Alternatively or additionally, the sequence of the thiolase of the invention corresponding the thiolase signature having the accession number PS00737 in the Prosite database has an identity of at least 50% to the sequence corresponding to said thiolase signature of SEQ ID NO: 1. Said signature having the accession number PS00737 is a conserved thiolase signature which has the following consensus pattern: N-x(2)-G-G-x-[LIVM]-[SA]-x-G-H-P-x-[GAS]-x-[ST]-G. In a more particular embodiment, said sequence of the thiolase of the invention corresponding the thiolase signature having the accession number PS00737 in the Prosite database has an identity of at least 55% to the sequence corresponding to said thiolase signature of SEQ ID NO: 1. In another particular embodiment, said identity is of at least 60%, at least 65%, at least 70%, at least 75%, at least 80%, at least 85%, at least 90%, at least 95%, at least 99% or at least 100%. In a preferred embodiment, said sequence of the thiolase of the invention corresponding the thiolase signature having the accession number PS00737 in the Prosite database has an identity of about 55% to the sequence corresponding to said thiolase signature of SEQ ID NO: 1. In another preferred embodiment, said sequence of the thiolase of the invention corresponding the thiolase signature having the accession number PS00737 in the Prosite database has an identity of about 76% to the sequence corresponding to said thiolase signature of SEQ ID NO: 1. In a preferred embodiment, said sequence of the thiolase of the invention corresponding the thiolase signature having the accession number PS00737 in the Prosite database has an identity of about 82% to the sequence corresponding to said thiolase signature of SEQ ID NO: 1. In a preferred embodiment, said sequence of the thiolase of the invention corresponding the thiolase signature having the accession number PS00737 in the Prosite database has an identity of about 86% to the sequence corresponding to said thiolase signature of SEQ ID NO: 1.

Alternatively or additionally, the sequence of the thiolase of the invention corresponding the thiolase signature having the accession number PS00099 in the Prosite database has an identity of at least 25% to the sequence corresponding to said thiolase signature of SEQ ID NO: 1. Said signature is a conserved thiolase signature which has the following consensus pattern: [AG]-[LIVMA]-[STAGCLIVM]-[STAG]-[LIVMA]-C-{Q}-[AG]-x-[AG]-x-[AG]-x-[SAG]. In a more particular embodiment, said sequence of the thiolase of the invention corresponding the thiolase signature having the accession number PS00099 in the Prosite database has an identity of at least 30% to the sequence corresponding to said thiolase signature of SEQ ID NO: 1. In another particular embodiment, said identity is of at least at least 35%, at least 40%, at least 45%, at least 50%, at least 55%, at least 60%, at least 65%, at least 70%, at least 75%, at least 80%, at least 85%, at least 90%, at least 95%, at least 99% or at least 100%. In a preferred embodiment, said sequence of the thiolase of the invention corresponding the thiolase signature having the accession number PS00099 in the Prosite database has an identity of about 35% to the sequence corresponding to said thiolase signature of SEQ ID NO: 1. In another preferred embodiment, said sequence of the thiolase of the invention corresponding the thiolase signature having the accession number PS00099 in the Prosite database has an identity of about 42% to the sequence corresponding to said thiolase signature of SEQ ID NO: 1. In a preferred embodiment, said sequence of the thiolase of the invention corresponding the thiolase signature having the accession number PS00099 in the Prosite database has an identity of about 82% to the sequence corresponding to said thiolase signature of SEQ ID NO: 1. In a preferred embodiment, said sequence of the thiolase of the invention corresponding the thiolase signature having the accession number PS00099 in the Prosite database has an identity of about 92% to the sequence corresponding to said thiolase signature of SEQ ID NO: 1.

In one embodiment, the mutant thiolase is selected from the group consisting of:
(i) The polypeptide of SEQ ID NO: 1, wherein the at least one mutation is located at position Phe293;
(ii)The polypeptide of SEQ ID NO: 2 wherein the at least one mutation is located at position Met290,
(iii)The polypeptide of SEQ ID NO: 3, wherein the at least one mutation is located at position Met289 and

In another embodiment, the mutant thiolase is selected from the group consisting of:
(i) The polypeptide of SEQ ID NO: 1, wherein the residue at position 293 is not Phe;
(ii) The polypeptide of SEQ ID NO: 2 wherein the residue at position 290 is not Met,
(iii)The polypeptide of SEQ ID NO: 3, wherein the residue at position 289 is not Met and

In another embodiment, the mutant thiolase is the polypeptide of SEQ ID NO: 1 wherein the at least one mutation located at position Phe293 is selected from the group consisting of Phe293Asp, Phe293Ala, Phe293Ser, Phe293Gly and Phe293Glu.

In another embodiment, the mutant thiolase is the polypeptide of SEQ ID NO: 2 wherein the at least one mutation at position Met290 is selected from the group consisting of Met290Asp, Met290Ala, Met290Ser, Met290Gly and Met290Glu.

In another embodiment, the mutant thiolase is the polypeptide of SEQ ID NO: 3, wherein at least one mutation located at position Met289 is selected from the group consisting of Met289Asp, Met289Ala, Met289Ser, Met289Gly and Met289Glu.

In another disclosure, the mutant thiolase is the polypeptide of SEQ ID NO: 4 wherein the at least one mutation located at position Met294 is selected from the group consisting of Met294Asp, Met294Ala, Met294Ser, Met294Gly, and Met294Glu.

In another embodiment, the thiolase according to the invention further comprises at least one additional mutation at a position selected from the group consisting of the residues corresponding to positions 90 and 383 in the polypeptide of SEQ ID NO: 1.

In one embodiment, the mutant thiolase is the polypeptide of SEQ ID NO: 1, containing mutations at positions Val90 and Phe293.

In one embodiment, the mutant thiolase is the polypeptide of SEQ ID NO: 1, containing mutations at positions Phe293 and Ile383.

In one embodiment, the mutant thiolase is the polypeptide of SEQ ID NO: 1, containing mutations at positions Val90, Phe293 and Ile383.

In one embodiment, the mutant thiolase is the polypeptide of SEQ ID NO: 1, containing mutations at positions Val90 and Phe293, wherein the mutations are such that the amino acid at position 90 is not Val and the amino acid at position 293 is not Phe.

In one embodiment, the mutant thiolase is the polypeptide of SEQ ID NO: 1, containing mutations at positions Phe293 and Ile383, wherein the mutations are such that the amino acid at position 293 is not Phe and the amino acid at position 383 is not Ile.

In one embodiment, the mutant thiolase is the polypeptide of SEQ ID NO: 1, containing mutations at positions Val90, Phe293 and Ile383, wherein the mutations are such that the amino acid at position 90 is not Val, the amino acid at position 293 is not Phe and the amino acid at position 383 is not Ile.

In one embodiment, the mutant thiolase is the polypeptide of SEQ ID NO: 1, containing mutations at positions Val90 and Phe293, wherein the mutation located at position Phe293 is selected from the group consisting of Phe293Asp, Phe293Ala, Phe293Ser, Phe293Gly and Phe293Glu and/or wherein the mutation located at position Val90 is a V90I mutation.

In one embodiment, the mutant thiolase is the polypeptide of SEQ ID NO: 1, containing mutations at positions Phe293 and Ile383 wherein the mutation located at position Phe293 is selected from the group consisting of Phe293Asp, Phe293Ala, Phe293Ser, Phe293Gly and Phe293Glu and/or wherein the mutation located at position Ile383 is a I383V mutation.

In one embodiment, the mutant thiolase is the polypeptide of SEQ ID NO: 1, containing mutations at positions Val90, Phe293 and Ile383, wherein the mutation located at position Phe293 is selected from the group consisting of Phe293Asp, Phe293Ala, Phe293Ser, Phe293Gly and Phe293Glu, wherein the mutation located at position Val90 is a V90I mutation and/or wherein the mutation located at position Ile383 is a I383V mutation.

In another embodiment, the mutant thiolase is selected from the group consisting of:
(i) The polypeptide of SEQ ID NO:1 containing the V90I and the F293A mutations,
(ii) The polypeptide of SEQ ID NO:1 containing the V90I and the F293D mutations,
(iii) The polypeptide of SEQ ID NO:1 containing the F293A and the I383V mutations,
(iv) The polypeptide of SEQ ID NO:1 containing the F293D and the I383V mutations,
(v) The polypeptide of SEQ ID NO:1 containing the V90I, the F293D and the I383V mutations and
(vi) The polypeptide of SEQ ID NO:1 containing the V90I, the F293A and the I383V mutations.

In another embodiment, the mutant thiolase is the thiolase of SEQ ID NO:2 containing mutations at positions Leu89 and Met290.

In another embodiment, the mutant thiolase is the thiolase of SEQ ID NO:2 containing mutations at positions Met290 and Met379.

In another embodiment, the mutant thiolase is the thiolase of SEQ ID NO:2 containing mutations at positions Leu89, Met290 and Met379.

In another embodiment, the mutant thiolase is the thiolase of SEQ ID NO:2 containing mutations at positions Leu89 and Met290, wherein the mutation is such that the amino acid at position 89 is not Leu and/or the amino acid at position 290 is not Met.

In another embodiment, the mutant thiolase is the thiolase of SEQ ID NO:2 containing mutations at positions Met290 and Met379, wherein the mutation is such that the amino acid at position 290 is not Met and the amino acid at position 379 is not Met.

In another embodiment, the mutant thiolase is the thiolase of SEQ ID NO:2 containing mutations at positions Leu89, Met290 and Met379, wherein the mutation is such that the amino acid at position 89 is not Leu, the amino acid at position 290 is not Met and/or the amino acid at position 379 is not Met.

In one embodiment, the mutant thiolase is the polypeptide of SEQ ID NO: 2, containing mutations at positions Leu89 and Met290, wherein the mutation located at position Met290 is selected from the group consisting of Met290Asp, Met290Ala, Met290Ser, Met290Gly and Met290Glu and/or the mutation at position Leu89 is a L89I mutation.

In one embodiment, the mutant thiolase is the polypeptide of SEQ ID NO: 2, containing mutations at positions Met290 and Met379, wherein the mutation located at position Met290 is selected from the group consisting of Met290Asp, Met290Ala, Met290Ser, Met290Gly and Met290Glu and/or the mutation at position Met379 is a Met379Val mutation.

In one embodiment, the mutant thiolase is the polypeptide of SEQ ID NO: 2, containing mutations at positions Leu89, Met290 and Met379, wherein the mutation located at position Met290 is selected from the group consisting of Met290Asp, Met290Ala, Met290Ser, Met290Gly and Met290Glu, the mutation at position Leu89 is a Leu89Ile mutation and/or the mutation at position Met379 is a Met379Val mutation.

In another embodiment, the mutant thiolase is selected from the group consisting of
(i) The polypeptide of SEQ ID NO:2 containing the L89I and the M290A mutations,
(ii) The polypeptide of SEQ ID NO:2 containing the L89I and the M290D mutations,
(iii) The polypeptide of SEQ ID NO:2 containing the M290D and the M379V mutations,
(iv) The polypeptide of SEQ ID NO:2 containing the M290A and the M379V mutations,
(v) The polypeptide of SEQ ID NO:2 containing the L89I, the M290A and the M379V mutations and
(vi) The polypeptide of SEQ ID NO:2 containing the L89I, the M290D and the M379V mutations.

In another embodiment, the mutant thiolase is the thiolase of SEQ ID NO:3 containing mutations at positions Val87 and Met289.

In another embodiment, the mutant thiolase is the thiolase of SEQ ID NO:3 containing mutations at positions Met289 and Leu378.

In another embodiment, the mutant thiolase is the thiolase of SEQ ID NO:3 containing mutations at positions Val87, Met289 and Leu378.

In another embodiment, the mutant thiolase is the thiolase of SEQ ID NO:3 containing mutations at positions Val87 and Met289, wherein the mutation is such that the amino acid at position 87 is not Val and/or the amino acid at position 289 is not Met.

In another embodiment, the mutant thiolase is the thiolase of SEQ ID NO:3 containing mutations at positions Met289 and Leu378, wherein the mutation is such that the amino acid at position 289 is not Met and the amino acid at position 378 is not Leu.

In another embodiment, the mutant thiolase is the thiolase of SEQ ID NO:3 containing mutations at positions Val87, Met289 and Met379, wherein the mutation is such that the amino acid at position 87 is not Val, the amino acid at position 289 is not Met and/or the amino acid at position 379 is not Met.

In another embodiment, the mutant thiolase is the thiolase of SEQ ID NO:3 containing mutations at positions Val87 and Met289, wherein the mutation at position 289 is Met289Asp, Met289Ala, Met289Ser, Met289Gly and Met289Glu and/or wherein the mutation at position Val87 is a Val87Ile mutation.

In another embodiment, the mutant thiolase is the thiolase of SEQ ID NO:3 containing mutations at positions Met289 and Leu378 wherein the mutation at position 289 is Met289Asp, Met289Ala, Met289Ser, Met289Gly and Met289Glu and/or wherein the mutation at position Leu378 is a Leu378Val mutation.

In another embodiment, the mutant thiolase is the thiolase of SEQ ID NO:3 containing mutations at positions Val87, Met289 and Leu378, wherein the mutation at position 289 is Met289Asp, Met289Ala, Met289Ser, Met289Gly and Met289Glu, wherein the mutation at position Val87 is a Val87Ile mutation and wherein the mutation at position Leu378 is a Leu378Val mutation.

In another embodiment, the mutant thiolase is selected from the group consisting of
(i) The polypeptide of SEQ ID NO:3 containing the V87I, the M289A and the L378V mutations and
(ii) the polypeptide of SEQ ID NO:3 containing the V87I, the M289D and the L378V mutations.

In another disclosure, the mutant thiolase is the thiolase of SEQ ID NO:4 containing mutations at positions Leu88 and Met294.

In another disclosure, the mutant thiolase is the thiolase of SEQ ID NO:4 containing mutations at positions Met294 and Met385.

In another disclosure, the mutant thiolase is the thiolase of SEQ ID NO:4 containing mutations at positions Leu88, Met294 and Met385.

In another disclosure, the mutant thiolase is the thiolase of SEQ ID NO:4 containing mutations at positions Leu88 and Met294, wherein the mutation is such that the amino acid at position 88 is not Leu and/or the amino acid at position 294 is not Met.

In another disclosure, the mutant thiolase is the thiolase of SEQ ID NO:4 containing mutations at positions Met294 and Met385, wherein the mutation is such that the amino acid at position 294 is not Met and the amino acid at position 385 is not Met.

In another disclosure, the mutant thiolase is the thiolase of SEQ ID NO:4 containing mutations at positions Leu88, Met294 and Met385, wherein the mutation is such that the amino acid at position 88 is not Leu, the amino acid at position 294 is not Met and the amino acid at position 385 is not Met.

In another disclosure, the mutant thiolase is the thiolase of SEQ ID NO:4 containing mutations at positions Leu88 and Met294, wherein the mutation located at position Met294 is selected from the group consisting of Met294Asp, Met294Ala, Met294Ser, Met294Gly, and Met294Glu and/or the mutation located at position Leu88 is a Leu88Ile mutation.

In another disclosure, the mutant thiolase is the thiolase of SEQ ID NO:4 containing mutations at positions Met294 and Met385, wherein the mutation located at position Met294 is selected from the group consisting of Met294Asp, Met294Ala, Met294Ser, Met294Gly, and Met294Glu and/or the mutation located at position Met385 is a Met385Val mutation.

In another disclosure, the mutant thiolase is the thiolase of SEQ ID NO:4 containing mutations at positions Leu88, Met294 and Met385, wherein the mutation located at position Met294 is selected from the group consisting of Met294Asp, Met294Ala, Met294Ser, Met294Gly, and Met294Glu, the mutation located at position Leu88 is a Leu88Ile mutation and/or the mutation located at position Met385 is a Met385Val mutation.

In another disclosure, the mutant thiolase is selected from the group consisting of
(i) The polypeptide of SEQ ID NO:4 containing the L88I, the M294A and the M385V mutations,
(ii) The polypeptide of SEQ ID NO:4 containing the L88I, the M294D and the M385V mutations,

In a second aspect, the invention relates to a nucleic acid encoding a thiolase according to the first aspect of the invention, or to a vector comprising a nucleic acid encoding the thiolase according to the first aspect of the invention, or to a host cell comprising the vector comprising the nucleic acid encoding the thiolase according to the first aspect of the invention

The term "nucleic acid", as used herein, refers to a deoxyribonucleotide or ribonucleotide polymer in either single or double stranded form and, unless otherwise limited, encompasses natural nucleotides and analogues of natural nucleotides that hybridize to nucleic acids in a manner similar to naturally occurring nucleotides. The term "nucleotide" includes, but is not limited to, a monomer that includes a base (such as a pyrimidine, purine or synthetic analogs thereof) linked to a sugar (such as ribose, deoxyribose or synthetic analogs thereof), or a base linked to an amino acid, as in a peptide nucleic acid (PNA). A nucleotide is one monomer in an oligonucleotide or in a polynucleotide. A "nucleotide sequence" or "nucleic acid sequence" refers to the sequence of bases in an oligonucleotide or in a polynucleotide.

The term polynucleotide refers to a sequence of at least 10 nucleotides, preferably at least 16 nucleotides, preferably at least 20 nucleotides, preferably at least 25 nucleotides, preferably at least 100 nucleotides or more, including both polyribonucleotides and polydeoxyribonucleotides or combinations thereof. The term "oligonucleotide" also refers to molecules formed by conventional nucleotides bound by conventional phosphodiester bonds as well as variants thereof, including modifications in the purine or pyrimidine residues and modifications in the ribose or deoxyribose residues designed for increasing biological stability of the oligonucleotide or for stabilizing the physical stability of the hairpin-shaped structure. Examples of modified nucleotides that can be used in the present invention include, but are not limited to, nucleotides having at position 2' of the sugar a substituent selected from the fluoro, hydroxyl, amino, azido, alkyl, alkoxy, alkoxyalkyl, methyl, ethyl, propyl, butyl group or a functionalized alkyl group such as ethylamino, propylamino and butylamino. Alternatively, the alkoxy group is methoxy, ethoxy, propoxy or a functionalized alkoxy group according to the formula -O(CH₂)q-R, where q is 2 to 4 and R is an amino, methoxy or ethoxy group. Suitable alkoxyalkyl groups are methoxyethyl and ethoxyethyl. Oligonucleotides in which different nucleotides contain different modifications at position 2' are also part of the invention. Alternatively or additionally, the oligonucleotides of the invention can contain modified bonds such as phosphodiester-, phosphotriester-, phosphorothioate-, phosphorodithioate-, phosphoroselenoate-, phosphorodiselenoate-, phosphoroanilothioate-, phosphoramidate-, methylphosphonate-, boranephosphonate-type bonds as well as combinations thereof, or they are peptide nucleic acids (PNAs), in which the different nucleotides are bound by amide bonds.

The term "vector", as used herein, refers to a construct capable of delivering, and preferably additionally expressing, one or more polynucleotides of interest into a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells. This term also relates to targeting constructs which allow for random or site-directed integration of the targeting construct into genomic DNA. Such targeting constructs, preferably, comprise DNA of sufficient length for either homologous recombination or heterologous integration.

Transformation of a host cell with recombinant DNA can be carried out by conventional techniques that are well known to those of ordinary skill in the art. Where the host is prokaryotic, such as *E. coli,* competent cells which are capable of DNA uptake can be prepared from cells harvested after exponential growth phase and subsequently treated by the CaCl₂ method using procedures well known in the art. Alternatively, MgCl₂ or RbCl can be used. Transformation can also be performed after forming a protoplast of the host cell if desired, or by electroporation.

When the host is a eukaryote, such methods of transfection of DNA as calcium phosphate coprecipitates, conventional mechanical procedures such as microinjection, electroporation, insertion of a plasmid encased in liposomes, or virus vectors can be used. Eukaryotic cells can also be co-transformed with polynucleotide sequences encoding the mutant thiolase of the invention, and a second foreign DNA molecule encoding a selectable phenotype, such as the herpes simplex thymidine kinase gene. Another method is to use a eukaryotic viral vector, such as simian virus 40 (SV40) or bovine papilloma virus, to transiently infect or transform eukaryotic cells and express the protein. *See* Gluzman Y, Ed., "Eukaryotic Viral Vectors" (Cold Spring Harbor Laboratory Press, Cold Spring Harbor, NY, US, 1982).

### IN VITRO PROCESS FOR THE PRODUCTION OF THE COMPOUND OF FORMULA (I)

In a third aspect, the invention relates to a process, hereinafter "the first process of the invention" for the production of a compound of formula (I) comprising contacting a compound of formula (II) and a compound of formula (III) in the presence of a mutant thiolase capable of catalyzing the formation of a compound of formula (I) 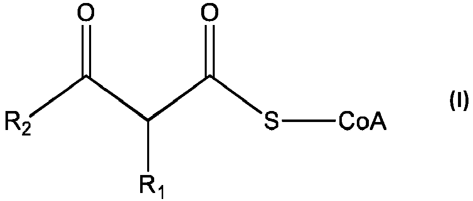 by the condensation of a compound of formula (II) 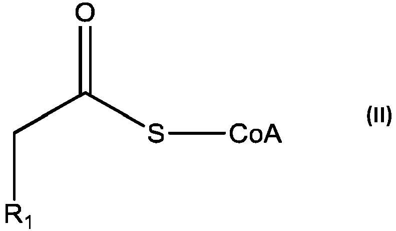 with a compound of formula (III) 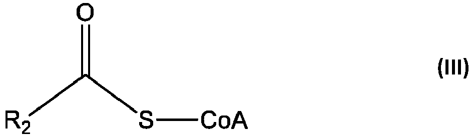
wherein R₁ and R₂ are independently selected from the group consisting of linear or branched C₁-C₆ alkyl, linear or branched C₂-C₆ alkenyl, linear or branched C₂-C₆ alkynyl, aryl, aryl-alkyl and benzyl, and S-CoA is coenzyme A,
said mutant thiolase having at least an amino acid substitution with respect to the naturally-occurring thiolase, wherein said amino acid substitution is located at a position corresponding to position 293 in the polypeptide of SEQ ID NO: 1, wherein said thiolase has at least a 75% sequence identity with a thiolase selected from the group consisting of of SEQ ID NO:1, 2, or 3 and displays a specific thiolase activity of at least 0,05mU/mg for the catalysis of a branched product of formula (I) when compound of formula (II) is butyryl-CoA and compound of formula (III) is acetyl-CoA, and wherein said contacting is carried out under conditions adequate for the condensation of compounds of formula (II) and (III) into a compound of formula (I).

The terms "mutant", "thiolase", "compound of formula I", "compound of formula II", and "compound of formula III" and their particulars have been defined in the context of the first embodiment of the invention.

In a particular embodiment of the first process of the invention, the sequence in said thiolase corresponding the thiolase signature having the accession number PS00098 in the Prosite database has an identity of at least 35% to the sequence corresponding to said signature in the thiolase of SEQ ID NO:1, the sequence in said thiolase corresponding the thiolase signature having the accession number PS00737 in the Prosite database has an identity of at least 50 % to the sequence corresponding to said signature in the thiolase of SEQ ID NO:1 and/or the sequence in said thiolase corresponding the thiolase signature having the accession number PS00099 in the Prosite database has an identity of at least 25% to the sequence corresponding to said signature in the thiolase of SEQ ID NO:1.

In one embodiment, the mutant thiolase is selected from the group consisting of:
(i) The polypeptide of SEQ ID NO: 1, wherein the at least one mutation is located at position Phe293;
(ii)The polypeptide of SEQ ID NO: 2 wherein the at least one mutation is located at position Met290,
(iii)The polypeptide of SEQ ID NO: 3, wherein the at least one mutation is located at position Met289 and

In another embodiment, the mutant thiolase is selected from the group consisting of:
(i) The polypeptide of SEQ ID NO: 1, wherein the residue at position 293 is not Phe;
(ii) The polypeptide of SEQ ID NO: 2 wherein the residue at position 290 is not Met,
(iii)The polypeptide of SEQ ID NO: 3, wherein the residue at position 289 is not Met and

In another embodiment, the mutant thiolase is the polypeptide of SEQ ID NO: 1 wherein the at least one mutation located at position Phe293 is selected from the group consisting of Phe293Asp, Phe293Ala, Phe293Ser, Phe293Gly and Phe293Glu.

In another embodiment, the mutant thiolase is the polypeptide of SEQ ID NO: 2 wherein the at least one mutation at position Met290 is selected from the group consisting of Met290Asp, Met290Ala, Met290Ser, Met290Gly and Met290Glu.

In another embodiment, the mutant thiolase is the polypeptide of SEQ ID NO: 3, wherein at least one mutation located at position Met289 is selected from the group consisting of Met289Asp, Met289Ala, Met289Ser, Met289Gly and Met289Glu.

In another disclosure, the mutant thiolase is the polypeptide of SEQ ID NO: 4 wherein the at least one mutation located at position Met294 is selected from the group consisting of Met294Asp, Met294Ala, Met294Ser, Met294Gly, and Met294Glu.

In another embodiment, the thiolase according to the invention further comprises at least one additional mutation at a position selected from the group consisting of the residues corresponding to positions 90 and 383 in the polypeptide of SEQ ID NO: 1.

In one embodiment, the mutant thiolase is the polypeptide of SEQ ID NO: 1, containing mutations at positions Val90 and Phe293.

In one embodiment, the mutant thiolase is the polypeptide of SEQ ID NO: 1, containing mutations at positions Phe293 and Ile383.

In one embodiment, the mutant thiolase is the polypeptide of SEQ ID NO: 1, containing mutations at positions Val90, Phe293 and Ile383.

In one embodiment, the mutant thiolase is the polypeptide of SEQ ID NO: 1, containing mutations at positions Val90 and Phe293, wherein the mutations are such that the amino acid at position 90 is not Val and the amino acid at position 293 is not Phe.

In one embodiment, the mutant thiolase is the polypeptide of SEQ ID NO: 1, containing mutations at positions Phe293 and Ile383, wherein the mutations are such that the amino acid at position 293 is not Phe and the amino acid at position 383 is not Ile.

In one embodiment, the mutant thiolase is the polypeptide of SEQ ID NO: 1, containing mutations at positions Val90, Phe293 and Ile383, wherein the mutations are such that the amino acid at position 90 is not Val, the amino acid at position 293 is not Phe and the amino acid at position 383 is not Ile.

In one embodiment, the mutant thiolase is the polypeptide of SEQ ID NO: 1, containing mutations at positions Val90 and Phe293, wherein the mutation located at position Phe293 is selected from the group consisting of Phe293Asp, Phe293Ala, Phe293Ser, Phe293Gly and Phe293Glu and/or wherein the mutation located at position Val90 is a Val90Ile mutation.

In one embodiment, the mutant thiolase is the polypeptide of SEQ ID NO: 1, containing mutations at positions Phe293 and Ile383 wherein the mutation located at position Phe293 is selected from the group consisting of Phe293Asp, Phe293Ala, Phe293Ser, Phe293Gly and Phe293Glu and/or wherein the mutation located at position Ile383 is a leI383Val mutation.

In one embodiment, the mutant thiolase is the polypeptide of SEQ ID NO: 1, containing mutations at positions Val90, Phe293 and Ile383, wherein the mutation located at position Phe293 is selected from the group consisting of Phe293Asp, Phe293Ala, Phe293Ser, Phe293Gly and Phe293Glu, wherein the mutation located at position Val90 is a Val90Ile mutation and/or wherein the mutation located at position Ile383 is a Ile383Val mutation.

In another embodiment, the mutant thiolase is selected from the group consisting of:
(i) The polypeptide of SEQ ID NO:1 containing the V90I and the F293A mutations,
(ii) The polypeptide of SEQ ID NO:1 containing the V90I and the F293D mutations,
(iii) The polypeptide of SEQ ID NO:1 containing the F293A and the I383V mutations,
(iv) The polypeptide of SEQ ID NO:1 containing the F293D and the I383V mutations,
(v) The polypeptide of SEQ ID NO:1 containing the V90I, the F293D and the I383V mutations and
(vi) The polypeptide of SEQ ID NO:1 containing the V90I, the F293A and the I383V mutations.

In another embodiment, the mutant thiolase is the thiolase of SEQ ID NO:2 containing mutations at positions Leu89 and Met290.

In another embodiment, the mutant thiolase is the thiolase of SEQ ID NO:2 containing mutations at positions Met290 and Met379.

In another embodiment, the mutant thiolase is the thiolase of SEQ ID NO:2 containing mutations at positions Leu89, Met290 and Met379.

In another embodiment, the mutant thiolase is the thiolase of SEQ ID NO:2 containing mutations at positions Leu89 and Met290, wherein the mutation is such that the amino acid at position 89 is not Leu and/or the amino acid at position 290 is not Met.

In another embodiment, the mutant thiolase is the thiolase of SEQ ID NO:2 containing mutations at positions Met290 and Met379, wherein the mutation is such that the amino acid at position 290 is not Met and the amino acid at position 379 is not Met.

In another embodiment, the mutant thiolase is the thiolase of SEQ ID NO:2 containing mutations at positions Leu89, Met290 and Met379, wherein the mutation is such that the amino acid at position 89 is not Leu, the amino acid at position 290 is not Met and/or the amino acid at position 379 is not Met.

In one embodiment, the mutant thiolase is the polypeptide of SEQ ID NO: 2, containing mutations at positions Leu89 and Met290, wherein the mutation located at position Met290 is selected from the group consisting of Met290Asp, Met290Ala, Met290Ser, Met290Gly and Met290Glu and/or the mutation at position Leu89 is a Leu89Ile mutation.

In one embodiment, the mutant thiolase is the polypeptide of SEQ ID NO: 2, containing mutations at positions Met290 and Met379, wherein the mutation located at position Met290 is selected from the group consisting of Met290Asp, Met290Ala, Met290Ser, Met290Gly and Met290Glu and/or the mutation at position Met379 is a Met379Val mutation.

In one embodiment, the mutant thiolase is the polypeptide of SEQ ID NO: 2, containing mutations at positions Leu89, Met290 and Met379, wherein the mutation located at position Met290 is selected from the group consisting of Met290Asp, Met290Ala, Met290Ser, Met290Gly and Met290Glu, the mutation at position Leu89 is a Leu89Ile mutation and/or the mutation at position Met379 is a Met379Val mutation.

In another embodiment, the mutant thiolase is selected from the group consisting of
(i) The polypeptide of SEQ ID NO:2 containing the L89I and the M290A mutations,
(ii) The polypeptide of SEQ ID NO:2 containing the L89I and the M290D mutations,
(iii) The polypeptide of SEQ ID NO:2 containing the M290D and the M379V mutations,
(iv) The polypeptide of SEQ ID NO:2 containing the M290A and the M379V mutations,
(v) The polypeptide of SEQ ID NO:2 containing the L89I, the M290A and the M379V mutations and
(vi) The polypeptide of SEQ ID NO:2 containing the L89I, the M290D and the M379V mutations.

In another embodiment, the mutant thiolase is the thiolase of SEQ ID NO:3 containing mutations at positions Val87 and Met289.

In another embodiment, the mutant thiolase is the thiolase of SEQ ID NO:3 containing mutations at positions Met289 and Leu378.

In another embodiment, the mutant thiolase is the thiolase of SEQ ID NO:3 containing mutations at positions Val87, Met289 and Leu378.

In another embodiment, the mutant thiolase is the thiolase of SEQ ID NO:3 containing mutations at positions Val87 and Met289, wherein the mutation is such that the amino acid at position 87 is not Val and/or the amino acid at position 289 is not Met.

In another embodiment, the mutant thiolase is the thiolase of SEQ ID NO:3 containing mutations at positions Met289 and Leu378, wherein the mutation is such that the amino acid at position 289 is not Met and the amino acid at position 378 is not Leu.

In another embodiment, the mutant thiolase is the thiolase of SEQ ID NO:3 containing mutations at positions Val87, Met289 and Met379, wherein the mutation is such that the amino acid at position 87 is not Val, the amino acid at position 289 is not Met and/or the amino acid at position 379 is not Met.

In another embodiment, the mutant thiolase is the thiolase of SEQ ID NO:3 containing mutations at positions Val87 and Met289, wherein the mutation at position 289 is Met289Asp, Met289Ala, Met289Ser, Met289Gly and Met289Glu and/or wherein the mutation at position Val87 is a Val87Ile mutation.

In another embodiment, the mutant thiolase is the thiolase of SEQ ID NO:3 containing mutations at positions Met289 and Leu378 wherein the mutation at position 289 is Met289Asp, Met289Ala, Met289Ser, Met289Gly and Met289Glu and/or wherein the mutation at position Leu378 is a Leu378Val mutation.

In another embodiment, the mutant thiolase is the thiolase of SEQ ID NO:3 containing mutations at positions Val87, Met289 and Leu378, wherein the mutation at position 289 is Met289Asp, Met289Ala, Met289Ser, Met289Gly and Met289Glu, wherein the mutation at position Val87 is a Val87Ile mutation and wherein the mutation at position Leu378 is a Leu378Val mutation.

In another embodiment, the mutant thiolase is selected from the group consisting of
(i) The polypeptide of SEQ ID NO:3 containing the V87I, the M289A and the L378V mutations and
(ii) the polypeptide of SEQ ID NO:3 containing the V87I, the M289D and the L378V mutations.

In another disclosure, the mutant thiolase is the thiolase of SEQ ID NO:4 containing mutations at positions Leu88 and Met294.

In another disclosure, the mutant thiolase is the thiolase of SEQ ID NO:4 containing mutations at positions Met294 and Met385.

In another disclosure, the mutant thiolase is the thiolase of SEQ ID NO:4 containing mutations at positions Leu88, Met294 and Met385.

In another disclosure, the mutant thiolase is the thiolase of SEQ ID NO:4 containing mutations at positions Leu88 and Met294, wherein the mutation is such that the amino acid at position 88 is not Leu and/or the amino acid at position 294 is not Met.

In another disclosure, the mutant thiolase is the thiolase of SEQ ID NO:4 containing mutations at positions Met294 and Met385, wherein the mutation is such that the amino acid at position 294 is not Met and the amino acid at position 385 is not Met.

In another disclosure, the mutant thiolase is the thiolase of SEQ ID NO:4 containing mutations at positions Leu88, Met294 and Met385, wherein the mutation is such that the amino acid at position 88 is not Leu, the amino acid at position 294 is not Met and the amino acid at position 385 is not Met.

In another disclosure, the mutant thiolase is the thiolase of SEQ ID NO:4 containing mutations at positions Leu88 and Met294, wherein the mutation located at position Met294 is selected from the group consisting of Met294Asp, Met294Ala, Met294Ser, Met294Gly, and Met294Glu and/or the mutation located at position Leu88 is a Leu88Ile mutation.

In another disclosure, the mutant thiolase is the thiolase of SEQ ID NO:4 containing mutations at positions Met294 and Met385, wherein the mutation located at position Met294 is selected from the group consisting of Met294Asp, Met294Ala, Met294Ser, Met294Gly, and Met294Glu and/or the mutation located at position Met385 is a Met385Val mutation.

In another disclosure, the mutant thiolase is the thiolase of SEQ ID NO:4 containing mutations at positions Leu88, Met294 and Met385, wherein the mutation located at position Met294 is selected from the group consisting of Met294Asp, Met294Ala, Met294Ser, Met294Gly, and Met294Glu, the mutation located at position Leu88 is a L88I mutation and/or the mutation located at position Met385 is a Met385Val mutation.

In another disclosuret, the mutant thiolase is selected from the group consisting of:
(i) The polypeptide of SEQ ID NO:4 containing the L88I, the M294A and the M385V mutations,
(ii) The polypeptide of SEQ ID NO:4 containing the L88I, the M294D and the M385V mutations,

The expression "contacting a compound of formula (II) and a compound of formula (III) in the presence of a thiolase", as used herein, refers to the incubation of said thiolase and said compounds of formula (II) and (III) for the interaction between said compounds and said thiolase; thereby leading to the formation of the compound of formula (I).

The conditions required for the thiolase to condense the compound of formula (II) and the compound of formula (III) producing the compound of formula (I) include an appropriate ratio between the thiolase and the substrates in terms of concentration and appropriate conditions of salts, cofactors, pH and temperature as well to an appropriate reaction time.

Suitable salt conditions for carrying the first process according to the invention include, without limitation, any buffer commonly known such as phosphate buffer, a bicarbonate buffer, a carbonate buffer, a Tris buffer, a borate buffer, a succinate buffer, a histidine buffer, a citrate buffer or a 2-(N-morpholino)ethanesulphonic acid buffer (MES) at concentrations of at least 10 mM, at least 20 mM, at least 30 mM, at least 40 mM, at least 50 mM, at least 60 mM, at least 70 mM, at least 80 mM, at least 90 mM, at least 100 mM, at least 200 mM, at least 300 mM or more. In a preferred embodiment, the process according to the present invention is carried out in 100 mM MES buffer.

Suitable pH values for carrying the first process according to the invention include, without limitation, values of 4.5, 5, 5.5, 6, 6.5, 7, 7.5, 8, 8.5, 9 and 9.5. In a preferred embodiment, the process is carried out at a pH of about 6.

Suitable temperature conditions for carrying the first process according to the invention include, without limitation, about 20°C, about 25°C, about 30°C, about 35°C, about 37°C. In a preferred embodiment, the process is carried out at 37°C.

Suitable reaction times for carrying the process according to the invention include, without limitation, at least 1 ms, at least 1 s, at least 15 s, at least 30 s, at least 1 min, at least 2 min, at least 3 min, at least 4 min, at least 5 min, at least 6 min, at least 7 min, at least 8 min, at least 9 min, at least 10 min, at least 15 min, at least 20 min, at least 25 min, at least 30 min, at least 35 min, at least 40 min, at least 45 min, at least 50 min, at least 55 min, at least 1 h, at least 2h, at least 3 h, at least 4h, at least 5h, at least 6h, at least 7h, at least 8h, at least 9h, at least 10 h or more. In a preferred embodiment, the reaction time is 1 hour.

The ratio of the concentration of compounds (II) and (III) in the first process according to the invention is not particularly limiting. Accordingly, suitable ratios of the concentration of compounds (II) to compounds (III) are 1000:1, 500:1, 100:1, 50:1, 40:1, 30:1, 20:1, 10:1, 9:1, 8:1, 7:1, 6:1, 5:1, 4:1, 3:1, 2:1, 1:1, 1:2, 1:3, 1:4, 1:4, 1:5, 1:6, 1:7, 1:8, 1:9, 1:10, 1:20, 1:30, 1:40, 1:50, 1:100, 1:500 or 1:1000. In one embodiment, both compounds are used at equimolar concentrations. Suitable concentrations of the compounds (II) and (III) include, without limitation, about 1 nM, about 5 nM, about 10 nM, about 20 nM, about 30 nM, about 40 nM, about 50 nM, about 100 nM, about 500 nM, about 1 µM, about 10 µM, about 20 µM, about 30 µM, about 40 µM, about 50 µM, about 100 µM, about 500 µM, about 1 mM, about 5 mM, about 10 mM, about 20 mM, about 30 mM, about 40 mM, about 50 mM, about 100 mM or more. In a preferred embodiment, the concentration of substrates (I) and (II) is between 0.1 and 1 mM.

Suitable concentrations of the thiolase for carrying the first process according to the invention include, without limitation, about 1 µg/mL, about 2 µg/mL, about 3 µg/mL, about 5 µg/mL, about 8 µg/mL, about 10 µg/mL, about 20 µg/mL, about 30 µg/mL, about 50 µg/mL, about 75 µg/mL, about 100 µg/mL, about 200 µg/mL, about 300 µg/mL, about 500 µg/mL, about 750 µg/mL, about 1 mg/mL, about 2 mg/mL, about 3 mg/mL, about 5 mg/mL, about 7.5 mg/mL, about 10 mg/mL, about 20 mg/mL, about 30 mg/mL, about 50 mg/mL, about 75 mg/mL, about 100 mg/mL. In a preferred embodiment, the concentration of the thiolase is between 0.05 and 1 mg/mL.

In a particular and preferred embodiment of the invention, the first process of the invention further comprises contacting the compound of formula (I) with a polypeptide having beta-ketoacyl-CoA reductase activity capable of catalyzing the formation of a compound of formula (IV) 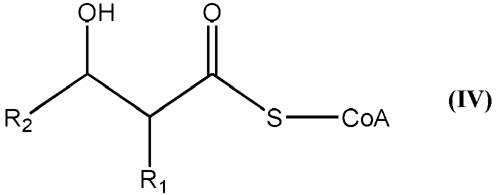 from the compound of formula (I) and wherein R₁ and R₂ are independently selected from the group consisting of linear or branched C₁-C₆ alkyl, linear or branched C₂-C₆ alkenyl, linear or branched C₂-C₆ alkynyl, aryl, aryl-alkyl and benzyl, and S-CoA is coenzyme A and wherein the polypeptide having beta-ketoacyl-CoA reductase activity consists of the sequence of SEQ ID NO: 6.

The term "polypeptide having beta-ketoacyl-CoA reductase activity", as used herein refers to a polypeptide which catalyzes the NADP(H) conversion of branched aldehydes or ketones to their respective alcohols, wherein the term NAD(P)H refers to either NADH or NADPH as the cofactor. The polypeptide having beta-ketoacyl-CoA reductase activity for use in the present invention is a biological catalyst; thus the polypeptide having beta-ketoacyl-CoA reductase activity for use in the invention is an enzyme.

The beta-ketoacyl-CoA reductase activity can be determined by any method known in the art suitable for the determination of the activity of NAD(P)H-dependent aldehyde reductase activity such as liquid chromatography coupled to mass spectrometry or spectrophotometrically by monitoring the oxidation of NAD(P)H at 240 nm.

Polypeptide having beta-ketoacyl-CoA reductase activity for use in the present invention include polypeptides having specific activities in the range of at least 0.001, at least 0.01, at least 0.1, at least 1, at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 75, at least 80, at least 85, at least 90, at least 95, at least 100 mU/mg, at least 200 mU/mg, at least 400 mU/mg, at least 800 mU/mg, at least 1000 mU/mg or more when using 3-keto-2-ethyl-butyryl-CoA as substrate.

According to the present disclosure, the sequence of a polypeptide having beta-ketoacyl-CoA reductase activity has an identity of at least 45% to the sequence of SEQ ID NO: 6, wherein SEQ ID NO: 6 corresponds to the amino acid sequence of the beta-ketoacyl-CoA reductase from *Pseudomonas putida.*

In a particular disclosure the sequence of a polypeptide having beta-ketoacyl-CoA reductase activity has an identity of at least 46%, at least 47%, at least 48%, at least 49%, at least 50%, at least 51%, at least 52%, at least 53%, at least 54%, at least 55%, at least 60%, at least 65%, at least 70%, at least 75%, at least 80%, at least 85%, at least 90%, at least 95%, at least 99% or more to the sequence SEQ ID NO:6. In a preferred embodiment, the above identity value is determined over the complete length of the polypeptides being compared. The term "identity" as well as suitable algorithms for determining the percentage of identity among two sequences have been detailed above.

In particular, the polypeptide having beta-ketoacyl-CoA reductase activity consists of the amino acid sequence of the beta-ketoacyl-CoA reductase protein from *Pseudomonas putida* shown in SEQ ID NO: 6.

As the person skilled in the art will understand, this particular embodiment includes suitable conditions for carrying out the reaction by which the compound (IV) is obtained from compound (I). Said conditions include an appropriate ratio between the polypeptide having beta-ketoacyl-CoA reductase activity and the substrate in terms of concentration and appropriate conditions of salts, cofactors, pH and temperature as well to an appropriate reaction time.

Suitable salt, pH, temperature conditions, suitable concentration of both cofactor and compound (I) and appropriate reaction time have been detailed above.

Suitable concentrations of the polypeptide having beta-ketoacyl-CoA reductase activity include, without limitation, about 1 µg/mL, about 2 µg/mL, about 3 µg/mL, about 5 µg/mL, about 8 µg/mL, about 10 µg/mL, about 20 µg/mL, about 30 µg/mL, about 50 µg/mL, about 75 µg/mL, about 100 µg/mL, about 200 µg/mL, about 300 µg/mL, about 500 µg/mL, about 750 µg/mL, about 1 mg/mL, about 2 mg/mL, about 3 mg/mL, about 5 mg/mL, about 7.5 mg/mL, about 10 mg/mL, about 20 mg/mL, about 30 mg/mL, about 50 mg/mL, about 75 mg/mL, about 100 mg/mL. In a preferred embodiment, the concentration of the polypeptide having beta-ketoacyl-CoA reductase activity is between 0.05 and 1 mg/mL.

In a particular and preferred embodiment of the first process of the invention, R₁ is an ethyl group. In another particular and preferred embodiment of the first process invention, R₂ is a methyl group.

### IN VITRO PROCESS FOR THE PRODUCTION OF THE COMPOUND OF FORMULA (IV)

In a disclosure, the invention relates to a process for the production of a compound of formula (IV), hereinafter "the third process of the invention", comprising contacting a compound of formula (I) with a polypeptide having beta-ketoacyl-CoA reductase activity capable of catalyzing the formation of a compound of formula (IV) 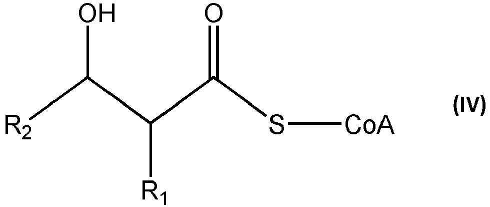 from the compound of formula (I), wherein R₁ and R₂ are independently selected from the group consisting of linear or branched C₂-C₆ alkyl, linear or branched C₂-C₆ alkenyl, linear or branched C₂-C₆ alkynyl, aryl, aryl-alkyl and benzyl, and S-CoA is coenzyme A.

The terms "polypeptide having beta-ketoacyl-CoA reductase activity", "compound of formula I", "compound of formula IV", "C₁-C₆ alkyl", "C₂-C₆ alkyl", "C₂-C₆ alkenyl", "C₂-C₆ alkynyl", "aryl", "aryl-alkyl", "benzyl" as the particulars thereof have been previously defined and are used herein with the same meaning.

The expression "contacting a compound of formula (I) with a polypeptide having beta-ketoacyl-CoA reductase activity", as used herein, refers to the incubation of said polypeptide and said compound of formula (I) for the interaction between said compound and said polypeptide; thereby leading the formation of the compound of formula (IV).

As the person skilled in the art will understand, this process is carried out under suitable conditions by which the compound (IV) is obtained from compound (I). Said conditions include an appropriate ratio between the polypeptide having beta-ketoacyl-CoA reductase activity and the substrate (i.e. the compound of formula I) in terms of concentration and appropriate conditions of salts, cofactors, pH and temperature as well to an appropriate reaction time.

Suitable salt conditions for carrying the third process according to the disclosure include, without limitation, any buffer commonly known such as phosphate buffer, a bicarbonate buffer, a carbonate buffer, Tris buffer, a borate buffer, a succinate buffer, a histidine buffer, a citrate buffer or a 2-(N-morpholino)ethanesulphonic acid buffer (MES) at concentrations of at least 10 mM, at least 20 mM, at least 30 mM, at least 40 mM, at least 50 mM, at least 60 mM, at least 70 mM, at least 80 mM, at least 90 mM, at least 100 mM, at least 200 mM, at least 300 mM or more. In a preferred embodiment, the third process according to the present invention is carried out in 100 mM MES buffer.

Suitable pH values for carrying the third process according to the disclosure include, without limitation, values of 4.5, 5, 5.5, 6, 6.5, 7, 7.5, 8, 8.5, 9 and 9.5. In a preferred embodiment, the process is carried out at a pH of about 6.

Suitable temperature conditions for carrying the third process according to the disclosure include, without limitation, about 20°C, about 25°C, about 30°C, about 35°C, about 37°C. In a preferred embodiment, the third process of the invention is carried out at 37°C.

Suitable reaction times for carrying the third process according to the disclosure include, without limitation, at least 1 ms, at least 1 s, at least 15 s, at least 30 s, at least 1 min, at least 2 min, at least 3 min, at least 4 min, at least 5 min, at least 6 min, at least 7 min, at least 8 min, at least 9 min, at least 10 min, at least 15 min, at least 20 min, at least 25 min, at least 30 min, at least 35 min, at least 40 min, at least 45 min, at least 50 min, at least 55 min, at least 1 h, at least 2h, at least 3 h, at least 4h, at least 5h, at least 6h, at least 7h, at least 8h, at least 9h, at least 10 h or more. In a preferred embodiment, the reaction time is 1 hour.

Suitable concentration of the compound (I) for carrying the third process according to the disclosure include, without limitation, about 1 nM, about 5 nM, about 10 nM, about 20 nM, about 30 nM, about 40 nM, about 50 nM, about 100 nM, about 500 nM, about 1 µM, about 10 µM, about 20 µM, about 30 µM, about 40 µM, about 50 µM, about 100 µM, about 500 µM, about 1 mM, about 5 mM, about 10 mM, about 20 mM, about 30 mM, about 40 mM, about 50 mM, about 100 mM or more. In a preferred embodiment, the concentration of the compound of formula (I) is between 0.1 and 0.5 mM.

Suitable concentrations of the polypeptide having beta-ketoacyl-CoA reductase activity include, without limitation, about 1 µg/mL, about 2 µg/mL, about 3 µg/mL, about 5 µg/mL, about 8 µg/mL, about 10 µg/mL, about 20 µg/mL, about 30 µg/mL, about 50 µg/mL, about 75 µg/mL, about 100 µg/mL, about 200 µg/mL, about 300 µg/mL, about 500 µg/mL, about 750 µg/mL, about 1 mg/mL, about 2 mg/mL, about 3 mg/mL, about 5 mg/mL, about 7.5 mg/mL, about 10 mg/mL, about 20 mg/mL, about 30 mg/mL, about 50 mg/mL, about 75 mg/mL, about 100 mg/mL. In a preferred embodiment, the concentration of the polypeptide having beta-ketoacyl-CoA reductase activity is between 0.05 and 1 mg/mL.

The term "cofactor" as used herein refers to a non-protein chemical compound that is required for the protein's biological activity. In one embodiment, the molecule NADPH acts as cofactor in the process for obtaining a compound of formula (IV) defined above. In another embodiment, the molecule NADH acts as cofactor in the process for obtaining compound of formula (IV) defined above. NADH acts as a reducing agent that donates electrons which are necessary for the formation of the hydroxyl group (-OH) from the carbonyl group (=O). Consequently, NADH is transformed to its oxidized form, NAD⁺. Suitable concentrations of the NADPH or NADH cofactor for carrying the first process according to the invention include, without limitation, about 10 nM, about 50 nM, about 0.1 mM, about 0.5 mM, about 1 mM, about 5 mM, about 10 mM or more. In a preferred embodiment, the cofactor concentration is 1 mM.

In a particular and disclosure of the third process of the invention, R₁ is an ethyl group. In another particular and preferred embodiment of the third process of the invention, R₂ is a methyl group.

Suitable polypeptides having beta-ketoacyl-CoA reductase activity for use according to the present invention have been detailed in the context of the first process of the invention and may be equally used in the context of the third process of the invention. In a particular and preferred embodiment of the third process of the invention, the polypeptide having beta-ketoacyl-CoA reductase activity consist of the amino acid sequence of the beta-ketoacyl-CoA reductase protein from *Pseudomonas putida* and which is shown in SEQ ID NO: 6.

### EXAMPLES

### Example 1. Alignment of thiolase sequences and identification of conserved residues.

The thiolases analyzed in the present study are shown in Table 1

**Table 1. List of selected thiolases**

| SEQ ID NO | Enzyme | Organism | Length | Sequence ID | Plasmid | Identity *(%) |
|---|---|---|---|---|---|---|
| 2 | BktB | *Ralstonia eutropha* H16 | 394 | WP_011615089.1 | pET28mut | 100 |
| 1 | Erg10 | *Saccharomyces cerevisiae* s288 | 398 | NP_015297.1 | pET28mut | 39 |
| 3 | AtoB | *Escherichia coli* K-12 MG1655 | 394 | WP_000786547.1 | pET32b | 50 |
| 4 | *Tt*Thl | *Thermus thermophilus* HB8 | 401 | WP_011228352.1 | pET28mut | 45 |

| | | | | | | |
|---|---|---|---|---|---|---|
| *Referred to BktB sequence. | | | | | | |

In order to indicate the degree of conservation of the selected residues, a multiple alignment was performed between the sequences of interest (SEQ ID NO: 1 to SEQ ID NO: 4). The alignment was performed using Clustal Omega web server from the European Bioinformatics Institute (EBI) using default parameters. The alignment is shown in **Figure 1****.**

### Example 2. Expression and purification of thiolases

Plasmids encoding natural or mutant thiolases were transformed into *E. coli* BL21 (DE3)-T1^{R}, expressed and purified as described below. The purified recombinant enzymes were then used as biocatalyst on the synthesis of 3-hydroxy-2-ethyl-butyryl-CoA (3H2EB-CoA).

Overnight culture in LB medium at 37°C was inoculated to an OD_{600 nm} of 0.1 into 50 ml of NZY Auto-Induction LB medium (NZYTech, Portugal). NZY medium was supplemented with 100 µg/mL Kan or Amp (according to the corresponding plasmid - Table 1). The cultures were grown at 37°C for 7 h and at 25°C until 24 h aerobically. Hereafter the cells were collected by centrifugation at 4500g for 10 minutes and kept at -20°C until further purification.

Pellets (50 ml) were homogenized with 50 mM phosphate pH 7.5 (10 ml) and placed in an ice bath for sonication. Sonication took place for 10 minutes using pulse mode (5 seconds ON/OFF) and 20% amplitude. Lysates were centrifuged at 4°C, 10,000g for 30 minutes. Supernatants containing the fusion protein were purified by His-affinity chromatography using empty PD-10 columns. Supernatants were incubated with 1 ml Talon® resin for 30 minutes at 4°C. Proteins were eluted with 1 ml elution buffer (300 mM imidazole, 50 mM phosphate pH 7.5), quantified and used for enzyme reaction.

### Example 3. High throughput expression and purification of thiolases

Plasmids encoding natural or mutant thiolases were transformed into *E. coli* BL21 (DE3)-T1^{R}, expressed and purified as described below. The purified recombinant enzymes were then used as biocatalyst on the synthesis of 3H2EB-CoA.

Overnight culture in LB medium at 37°C was inoculated to an OD₆₀₀ nm of 0.1 in 5 ml of NZY Auto-Induction LB medium (NZYTech, Portugal). NZY medium was supplemented with 100 µg/mL kanamycin or ampicillin (according to the corresponding plasmid - Table 1). The cultures were aerobically grown at 37°C for 7 h and at 25°C until 24 h. Hereafter, cells were aliquoted in 96-deep-well plates and centrifuged at 4500g for 10 minutes. Pellets were kept at -20°C until further purification.

Pellets in 96-deep-well plates were resuspended with 250 µl lysis buffer (50 mM phosphate buffer pH 7.5, Bugbuster®, 10 µg/ml DNase I, 20 mM MgSO₄) for cell lysis. Cell lysis took place for 20 minutes. Lysates were centrifuged at room temperature, 10,000g for 30 minutes. Supernatants containing the fusion protein were IMAC-purified. Supernatants were incubated with 100 µl Talon® resin for 15 minutes. Proteins were eluted with 50 µl elution buffer (300 mM imidazole, 50 mM phosphate buffer pH 7.5), quantified and used for enzyme reaction.

### Example 4. Assay for the acyl-CoA condensation by thiolases.

### a) Acetyl-CoA/butyryl-CoA condensation assay

The acetyl-CoA/butyryl-CoA condensation activity of thiolase variants was evaluated in the synthetic direction by an *in vitro* coupled enzymatic assay with product profiling by HPLC-MS. Since the condensation reaction catalyzed by thiolases is endergonic, it was coupled with 3-hydroxy-2-methylbutyryl-CoA dehydrogenase from *Pseudomonas putida* (SEQ ID NO:6, *Pp*FadB2) in order to drive the reaction in the forward direction (Thl/*Pp*FadB2 coupled assay). Three reaction products were possible: 3-hydroxybutyryl-CoA (3HB-CoA) (wild type product of the condensation of 2 acetyl-CoA molecules), 3-hydroxyhexanoyl-CoA (3HH-CoA) (acetyl-CoA/butyryl-CoA linear condensation product) and 3-hydroxy-2-ethylbutyryl-CoA (3H2EB-CoA) (acetyl-CoA/butyryl-CoA branched condensation product). All three products were analyzed by HPLC-MS (see below).

Reactions were carried out in 2-(N-morpholino)ethanesulfonate (MES) buffer (pH 6.0), 100 µM acetyl-CoA, 100 µM butyryl-CoA, 1 mM NADH, 0.15 mg/ml *Pp*FadB2 (SEQ ID NO:6) and 0.19 mg/ml of thiolase variant. Reaction mixtures were prepared in 96 well microplates in a 200 µL volume, and were incubated for 1 h at 37°C with agitation (300 rpm in Thermomixer). Once completed, the enzyme was eliminated by filtration through 10 kDa cut-off filtration plates (Millipore). Samples were stored at -20°C until further analysis.

### b) Acetyl-CoA/acetyl-CoA condensation assay

Reactions were carried out in 2-(N-morpholino)ethanesulfonate (MES) buffer (pH 6) with 200 µM acetyl-CoA, 1 mM NADH, 0.15 mg/ml *Pp*FadB2 and 0.19 mg/ml of thiolase variant. Reactions were processed as described in the previous section.

### Example 5. Expression and purification of 3-hydroxy-2-methylbutyryl-CoA-dehydrogenase from Pseudomonas putida (SEQ ID NO:6, PpFadB2)

Overnight culture in LB medium at 37°C was inoculated to an OD_{600 nm} of 0.1 into 500 ml of NZY Auto-Induction LB medium (NZYTech, Portugal). NZY medium was supplemented with 100 µg/mL ampicillin. The cultures were grown at 37°C for 7 h and at 25°C until 24 h aerobically. Hereafter the cells were aliquoted into 50 ml and collected by centrifugation at 4500g for 10 minutes and kept at -20°C until further purification.

Pellets (50 ml) were homogenized with 50 mM phosphate pH 7.5 (20 ml) and placed in ice bath for sonication. Sonication took place for 10 minutes using pulse mode (5 seconds ON/OFF) and 20% amplitude. Lysates were centrifuged at 4°C, 10,000g for 30 minutes. Supernatants containing the fusion protein were purified by His-Affinity chromatography using empty PD-10 columns. Supernatants were incubated with 3 ml Talon® resin for 30 minutes at 4°C. Proteins were eluted with 3 ml elution buffer (300 mM imidazole, 50 mM phosphate pH 7.5). Eluted solution was dialysed and concentrated by 10,000 MWCO ultrafiltration membrane (Amicon) to a final volume of approx. 0.5 ml. Purified protein was then quantified and kept at 4°C until being used for enzyme reaction.

### Example 6. Characterization of the dehydrogenase activity of PpFadB2 protein on 3-keto-2-ethylbutyryl-CoA.

The FadB2 protein from *Pseudomonas putida* (*Pp*FadB2; Accession Number: WP_064492895.1) exhibits a high activity on 3-keto-2-ethylbutyryl-CoA. To further confirm the biocatalytic potential of the enzyme, *in vitro* transformation assays were carried out using 1 mM NADH and 100 µM 3-keto-2-ethylbutyryl-CoA. Purified *Pp*FadB2 was added to the assays to a final concentration of 0.0001-1 mg/mL. At all protein concentrations, the consumption of 3-keto-2-ethylbutyryl-CoA was total, and product yields were in the range of 76-95% as assessed by HPLC **(****Fig. 2****).**

The reduction of the keto group in 3-keto-2-ethylbutyryl-CoA generates a new chiral carbon in the molecule. Dehydrogenases are highly stereoselective, and preferential synthesis of one isomer is expected.

### LC-MS, LC-MS/MS and NMR analysis of 3-hydroxy-2-ethyl-butyryl-CoA and 3-hydroxyhexanoyl-CoA

3-hydroxy-2-ethylbutyryl-CoA and 3-hydroxyhexanoyl-CoA were purchased from Sigma-Aldrich and analyzed by LC-MS. Chemically synthesized 3-hydroxy-2-ethylbutyryl-CoA eluted as two peaks with m/z ratio of 882.2. Chemically synthesized 3-hydroxyhexanoyl-CoA eluted as a single peak with m/z ratio of 882.2. All three peaks were well resolved chromatographically.

The MS/MS spectra of the two peaks from 3-hydroxy-2-ethylbutyryl-CoA were indistinguishable, and differed significantly from the MS/MS spectrum of the single peak of 3-hydroxy-2-ethylbutyryl-CoA **(Table 2)**.

**Table 2. MS/MS spectra of 3-hydroxy-2-ethylbutyryl-CoA (eluting as Peak 1 and Peak 2) and 3-hydroxyhexanoyl-CoA (eluting as a single peak). MS/MS spectra were recorded in the negative mode.**

| **3-hydroxy-2-ethylbutyryl-CoA** | | | | | | **3-hydroxyhexanoyl-CoA** | | |
|---|---|---|---|---|---|---|---|---|
| ***Peak 1*** | | | ***Peak 2*** | | | | | |
| ***m*/*z*** | ***Abund***¹ | ***Norm***² | ***m*/*z*** | ***Abund***¹ | ***Norm***² | ***m*/*z*** | ***Abund***¹ | ***Norm***² |
| 158,93 | 234289,31 | 2 | 158,93 | 97214,29 | 2 | 158,93 | 299574,63 | 5 |
| 408,01 | 1864911,63 | 16 | 408,01 | 750274,81 | 15 | 408,01 | 2362461,75 | 17 |
| ***409,02*** | ***223195,11*** | ***2*** | ***409,01*** | ***84708,17*** | ***2*** | | | |
| 426,02 | 1443406,88 | 12 | 426,02 | 570374,19 | 12 | 426,02 | 1848262,50 | 13 |
| | | | | | | ***453,15*** | ***292756,75*** | ***2*** |
| 533,11 | 823315,06 | 7 | 533,11 | 317675,25 | 6 | 533,11 | 934316,13 | 7 |
| ***551,12*** | ***287170,13*** | ***2*** | ***551,12*** | ***93560,85*** | ***2*** | | | |
| | | | | | | **782,20** | **670456,69** | ***5*** |
| 800,21 | 549242,69 | ***5*** | 800,21 | 199489,63 | ***4*** | 800,21 | 668001,81 | 5 |
| 880,18 | 3875833,50 | 33 | 880,18 | 1817105,25 | 37 | 880,18 | 4211549,50 | 30 |
| 881,18 | 1724722,75 | 15 | 881,18 | 717834,38 | 15 | 881,18 | 1956559,88 | 14 |
| 882,18 | 669350,13 | 6 | 882,18 | 267766,75 | 5 | 882,18 | 755951,38 | 5 |

| | | | | | | | | |
|---|---|---|---|---|---|---|---|---|
| ¹Abund: absolute value of peak area integration. ²Norm: relative value of peak area integration, normalized by the sum of all peaks integration areas in MS/MS spectrum. | | | | | | | | |

Chemically synthesized 3-hydroxy-2-ethylbutyryl-CoA was further analyzed by ¹H-NMR, which further confirmed the presence of two isomers **(****Fig. 3****).** Both LC-MS and ¹H-NMR confirmed that Peak 1 and Peak 2 isomers were present in a 7:3 ratio in chemically synthesized 3-hydroxy-2-ethylbutyryl-CoA.

Thus, it was concluded that chemically synthesized 3-hydroxy-2-ethylbutyryl-CoA consisted of a mixture of two diasteroisomers in a 7:3 ratio.

### Experimental assessment of the stereoselectivity of PpFadB2.

In the *in vitro* assays, the stereoselectivity of the enzyme was highly affected by its concentration. At 1 mg/mL *Pp*FadB2, reduction of 3-keto-2-ethyl-butyryl-CoA to 3-hydroxy-2-ethylbutyryl-CoA yields a mixture of two diasteroisomers which are resolved by HPLC. At lower enzyme concentrations, the ratio of isomers changed, with increasing yield in the Peak 2 isomer **(****Figure 2****).**

### Example 7. Acetyl-CoA/butyryl-CoA condensation activity of thiolase variants.

BktB_L89I_M290A_M379L (Var10) variant was constructed, expressed and purified to be used as biocatalyst in the synthesis of 3H2EB-CoA.

This protein variant was able to catalyze the synthesis of the desired compound (see chromatogram in **Figure. 4****).**

Once VarlO was identified as a biocatalyst able to perform the synthesis of 3H2EB-CoA, we decided to investigate the effect of those mutations on the other studied thiolases (Table 3). The selected thiolases show sequence identities lower than 50% (Table 3).

**Table 3. List of thiolase variants homologue to BktB VarlO mutant.**

| **Thiolase** | **Name** |
|---|---|
| BktB_L89I_M290A_M379V | BktB_Var10 |
| AtoB_V87I_M289_L378V | AtoB_Var10 |
| Erg10_V90I_F293A_I383V | Erg10_Var10 |
| *Tt*Thl_L88I_M294A_M385V | *Tt*Thl_Var10 |

The four modified thiolases were constructed, purified and used as biocatalysts for the *in vitro* acetyl-CoA/butyryl-CoA condensation. **Figure** 5 displays yield of products A) 3H2EB-CoA, B) 3HHB-CoA , C) 3HB-CoA and D) product selectivity for wild type thiolases and their triple mutants.

Regarding acetyl-CoA condensation activity, BktB_Var10, AtoB_Var10 and Erg10_Var10 showed worse activity than their wild type enzymes for 3HB-CoA synthesis **(****Fig. 5****).**

Remarkably AtoB_Var10 and Erg10_Var10 improved acetyl-CoA/butyryl-CoA branched condensation product yields, exhibiting comparable activity (ca. 0.6% product yield), 4-fold higher than that of BtkB_Var10. Also, Erg10_Var10 showed a remarkable selectivity towards the synthesis of the branched compound (3HB-CoA/3H2EB-CoA 60:40).

In the case of the synthesis of 3HH-CoA, BktB and its VarlO were the best performers, having a comparable synthetic activity. Also AtoB_Var10 was able to catalyse that reaction, although giving rise to lower yields. Mutations on *Tt*Thl killed the linear condensation activity. Interestingly, Erg10_VarlO produced traces of the linear product that were close to the detection limits (activity not observed in the wild type enzyme). Comparing, Erg10_Var10 and AtoB_Var10 showed 43 and 3 times higher yields for the branched compound than for the linear one, respectively.

Therefore, Erg10_Var10 was the best variant since it accepts substrates other than acetyl-CoA in the second step of the Claisen condensation reaction, while being selective towards the synthesis of branched compounds.

### Example 8. Analysis of single and double mutants of Erg10 thiolase.

We generated all possible combinations of the variations at these three positions. The three single variants (Erg10_V90I, Erg10_F293A and Erg10_I383V) and the three double variants (Erg10_V90I_F293A, Erg10_V90I_I383V and Erg10_F293A_I383V) were evaluated for the synthesis of 3H2EB-CoA.

Double variants having the mutation F293A on their sequences were able to catalyse the condensation of acetyl-CoA and butyryl-CoA, giving rise to 3H2EB-CoA **(****Fig. 6****).** Of those having activity, F293A_I383V displayed higher performance than V90I_F293A. Concerning single mutants, only F293A was capable of carrying out the desired reaction, showing 3-fold increase of activity compared to Erg10_Var10. In the case of single variant I383V, the branched compound synthesised was close to the detection limits. Acetyl-CoA/butyryl-CoA condensation products were not found for variant V90I **(****Fig. 6****).**

### Example 9. Analysis of Erg10 thiolase mutants at Phe293.

The role of Phe293 residue for thiolase activity of **Erg10** was explored by saturation mutagenesis (changing it for the remaining 19 natural amino acids). Surprisingly, the F293D mutant outperformed **Erg10_**VarlO 17 times and 120 times the initial activity observed in BktB. **(****Fig. 7****).**

When looking at product selectivity **(****Fig. 7A** **insert),** F293D came up as the most active and selective variant for the production of 3H2EB-CoA. Remarkably, this mutant yielded 1.5- and 35-times more 3H2EB-CoA than 3HB-CoA **(****Fig. 7B****)** and 3HH-CoA. **(****Fig. 7C****),** respectively.

### Example 10. Kinetic analysis of thiolase variants.

For the kinetic analysis of the acetyl-CoA/butyryl-CoA condensation activity of the best thiolase variants, the concentration of enzyme in the assay was adjusted in order to ensure capturing the linear phase of product synthesis profile. Two different enzyme concentrations were assayed for each protein (0.04 and 0.02 mg/ml for Erg10_F293D; 0.19 and 0.09 mg/ml for Erg10_F293A). Reactions were followed during 24 h. Specific activity was calculated for the first 20 minutes of reaction.

Assays were carried out in triplicate and at least at two protein concentrations in order to ensure the reliability of the activity determined.

Reactions were stopped by fast separation of the enzyme by filtration through 10 kDa cut-off filtration plates. Samples were stored at -20°C before until further analysis. Specific activity for Erg10_F293D, was measured and compared to the one for Erg10_F293A. Results showed Erg_F293D was 12 times more active for the synthesis of 3H2EB-CoA than Erg_F293A, displaying a specific activity of 700 ± 140 mU/g protein and 54 ± 10 mU/g protein, respectively.

We carried out a time course of 3H2EB-CoA formation. Both enzymes showed a typical saturation curve **(****Figure 8****).** After 24 h, we observed a maximum production yields for Erg10_F293D and Erg10_F293A ca. 3000 nM and 1800 nM, respectively (Fig. 8).

### Example 11: Characterization of substrate flexibility of Erg10 thiolase variants

We have analyzed the capability of Erg10 variants to catalyze several condensation reactions *in vitro.* The enzyme assays were carried out as described for the acetyl-CoA/butyryl-CoA condensation reaction but with alternative substrates.

We performed Cross-Claisen condensation reactions using acetyl-CoA/propionyl-CoA and acetyl-CoA/hexanoyl-CoA as substrate pairs. Reactions were analyzed by LC-MS by monitoring chromatograms for compounds with m/z of 868.1 or 910.2, respectively.

### Acetyl-CoA/propionyl-CoA Cross Claisen condensation.

Chromatograms were monitored for compounds with m/z of 868.1. Several peaks were detected in samples, two of them (peaks 2 and 3) were significantly more intense (13 and 1.5-fold higher in reaction samples with Erg10_F293A compared to enzyme-free controls; 122 and 5-fold higher in reaction samples with Erg10_F293D compared to enzyme-free controls) (Fig. 9). These two peaks therefore correspond to products generated by the action of Erg10 variants on substrates.

As in the case of the acetyl-CoA/butyryl-CoA condensation, linear (3-hydroxypentanoyl-CoA) and branched (3-hydroxy-2-methylbutyryl-CoA) condensation products are possible. The retention time of peak 2 differs to that of the commercial standard 3-hydroxypentanoyl-CoA (Sigma), which suggests that the major product generated in the reaction is likely to correspond to the branched condensation product (3-hydroxy-2-methylbutyryl-CoA, peak 2), although the linear condensation product is also formed (3-hydroxypentanoyl-CoA, peak 3) **(****Fig. 9****).**

### Acetyl-CoA/hexanoyl-CoA Cross Claisen condensation.

Chromatograms were monitored for compounds with m/z of 910.2. Several peaks were detected in samples, one of them (retention time 12.5 min) was significantly more intense in reaction samples compared to enzyme-free controls (22-fold higher in reaction samples with Erg10_F293D compared to enzyme-free controls). This peak therefore corresponds to a product generated by the action of Erg10 variant on substrates **(****Fig. 10****).**

Linear (3-hydroxyoctanoyl-CoA) and branched (3-hydroxy-2-butylbutyryl-CoA) condensation products are possible but its identity could not be confirmed, due to the lack of a commercial standard.

Thus, the results from acetyl-CoA/propionyl-CoA and acetyl-CoA/hexanoyl-CoA Cross-Claisen condensation reactions suggest that:
- Erg10_F293D variant can accept a wide range of acyl-CoAs as substrate in the second step of the Claisen condensation.
- Erg10_F293D variant is highly specific towards acetyl-CoA as substrate in the first step of the Claisen condensation.
- Altogether, these two observations further support the higher selectivity of Erg10_F293 variants towards the synthesis of branched condensation compounds.

### EXAMPLE 12. Acetyl-CoA/acetyl-CoA condensation activity of Erg10 thiolase variants.

Wild type reaction was also carried out with these enzymes. Erg10_F293D and Erg10_F293A retained 60% and 30%, respectively, of the acetyl-CoA/acetyl-CoA condensation activity of the wild type enzyme **(****Fig. 11****).**

### Example 13: Effect of acetyl-CoA/butyryl-CoA ratio on the product formation profile in acetyl-CoA/butyryl-CoA condensation assays.

Different ratios acetyl-CoA:butyryl-CoA were assayed. We evaluated acetyl-CoA:butyryl-CoA ratios from 1:1 to 1:10. An increase of 3-hydroxy-2-ethylbutyryl-CoA product formation was observed when increasing butyryl-CoA concentration. Contrarily, 3HB-CoA production was significantly reduced under the same reaction conditions **(****Figure 12****).** Regarding 3HH-CoA, a clear tendency was not observed due to the low concentration near to detection limits in all cases.

### Example 14: Analysis of single and double mutants of BktB thiolase (SEQ. ID NO. 2)

*Ralstonia eutropha* thiolase (BktB, SEQ. ID. NO:2) is able to condense two acetyl-CoA molecules (yielding acetoacetyl-CoA) or one acetyl-CoA and one butyryl-CoA molecules (yielding 3-ketohexanoyl-CoA). A small amount of 3-keto-2-ethylbutyryl-CoA is produced. In the presence of *Pp*FadB2 these compounds are transformed to 3-hydroxybutyryl-CoA, 3-hydroxy-2-ethylbutyryl-CoA and 3-hydroxyhexanoyl-CoA, respectively.

Single, double and triple mutants at Leu89, Met290 and Met379 were constructed. Seven protein variants were constructed: BktB Leu89Ile, BktB Met290Ala, BktB Met290Asp, BktB Met379Val, BktB Leu89Ile Met290Ala, BktB Leu89Ile Met379Val, BktB Met290Ala Met379Val or BktB Leu89Ile Met290Ala Met379Val. The ability to conduct a Claisen condensation activity on a mixture of acetyl-CoA and butyryl-CoA of the resulting protein variants was assessed as explained before.

Reaction products identified were: 3-hydroxybutyryl-CoA, 3-hydroxy-2-ethylbutyryl-CoA and 3-hydroxyhexanoyl-CoA. Two different isomers of 3-hydroxy-2-ethylbutyryl-CoA were detected, which eluted at different retention times **(****Fig 13****).**

### Example 15: Analysis of single and triple mutants of AtoB thiolase (SEQ. ID NO.3)

*Escherichia coli* thiolase (AtoB, SEQ. ID. NO:3) is able to condense two acetyl-CoA molecules (yielding acetoacetyl-CoA) or one acetyl-CoA and one butyryl-CoA molecules (yielding 3-ketohexanoyl-CoA). In the presence of *Pp*FadB2 these compounds are transformed to 3-hydroxybutyryl-CoA and 3-hydroxyhexanoyl-CoA, respectively.

Single and triple mutants at Val87, Met289 and Leu378 were constructed. Three protein variants were constructed: AtoB Met289Ala, AtoB Met289Asp and AtoB Val87Ile Met289Ala Leu378Val (VarlO). The ability to conduct a Claisen condensation activity on a mixture of acetyl-CoA and butyryl-CoA of the resulting protein variants was assessed as explained before.

Reaction products identified were: 3-hydroxybutyryl-CoA, 3-hydroxy-2-ethylbutyryl-CoA and 3-hydroxyhexanoyl-CoA. Two different isomers of 3-hydroxy-2-ethylbutyryl-CoA were detected, which eluted at different retention times **(Table 4)**.

**Table 4. Concentrations of the products of the acetyl-CoA/butyryl-CoA condensation reaction (3-hydroxybutyryl-CoA, 3-hydroxy-2-ethylbutyryl-CoA and 3-hydroxyhexanoyl-CoA) catalyzed by AtoB thiolase and its mutants. Reactions were carried out with a 1:1 acetyl-CoA:butyryl-CoA ratio.**

| | 3HB-CoA (µM) | 3H2EB-CoA (nM) | 3HHB-CoA (nM) |
|---|---|---|---|
| AtoB | 4,12 | 0 | 425 |
| M289A | 5,62 | 33,62 | 17,22 |
| M289D | 12,51 | 2491,38 | 34,57 |
| VarlO | 11,63 | 2166,47 | 30,92 |

### Example 16. UPLC-MS analysis of coenzyme A thioesters.

Standard stock solutions were prepared in dH₂O. Then, calibration curve samples containing different amounts of each analyte were obtained after dilution with 100 mM ammonium formate buffer. The final concentrations were 40, 30, 20, 10, 4, 1, 0.1 mM for acetyl-CoA, butyryl-CoA and 3-hydroxybutyryl-CoA and 200, 150, 100, 50, 20, 5, 2, 0.5 µM for 3-hydroxyhexanoyl-CoA and 3-hydroxy-2-ethylbutyryl-CoA. Blank samples were also included before and after the calibration curve samples in order to control the absence of carry-over effect.
Chromatography was performed in an Acquity UPLC system using an Acquity BEH C18 column (100x 2.1 mm, 1.7 µm) from Waters (Milford, MA, USA) and equipped with photodiode array detector (PDA). The gradient elution buffers were A (water and 100 mM of ammonium formate) and B (acetonitrile). The gradient method was as follows: 0-1 min at 95% A, 1-14 min to 80% A, 14-15 min to 10% A 15-17 min at 10% A, 17-17.5 min to 95% A, 17.5-20 min at 95% A. The UV detector wavelength was set at 254 nm and the injection volume was 10 µL. Total run time was 20 min and the flow rate was set at 300 µLmin-1.
The mass spectrometry detection was carried out using a time-of-flight mass spectrometer (ESI-TOF) LCT Premier XE from Waters (Milford, MA, USA) with an electrospray ionization source, working in positive /V mode. The MS range acquired was between m/z 100-1000. The capillary and cone voltages were set at 3000 and 100 V, respectively. For other parameters, desolvation gas temperature was 220°C and source temperature was 120°C. The desolvation gas flow was set at 600 L·h⁻¹ and cone gas flow was set at 50 L·h⁻¹.
Masslynx v4.1 software was used to analyze chromatograms and spectra (Waters, Milford, MA, USA). All the analytes were identified by mass spectrometry (Table 5). The quantification of 3-hydroxyhexanoyl-CoA and 3-hydroxy-2-ethylbutyryl-CoA was performed by ESI-TOF MS. On the other hand, in order to avoid the signal saturation, the quantification of acetyl-CoA, butyryl-CoA and 3-hydroxybutyryl-CoA was performed by UV absorbance at 254 nm.

**Table 5. Analytes, retention time, adduct and detected ion.**

| Analyte | Retention time (min) | Adduct | m/z |
|---|---|---|---|
| **Acetyl-CoA** | 2.9-3.2 | [M+H]+ | 810.12 |
| **3-hydroxybutyryl-CoA** | 3.2-3.5 | [M+H]+ | 854.16 |
| **3-hydroxy-2-ethylbutyryl-CoA isomer 1** | 5.9-6.2 | [M+H]+ | 882.19 |
| **3-hydroxy-2-ethylbutyryl-CoA isomer 2** | 6.6-6.9 | [M+H]+ | 882.19 |
| **3-hydroxyhexanoyl- CoA** | 7.2-7.5 | [M+H]+ | 882.19 |
| **Butyryl-CoA** | 7.4-7.7 | [M+H]+ | 838.16 |

## Claims

1. A mutant thiolase capable of catalyzing the formation of a compound of formula (I) 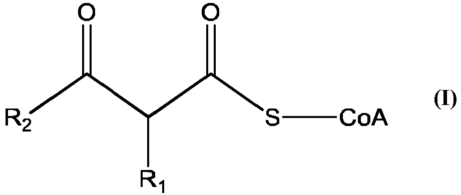 by the condensation of a compound of formula (II) 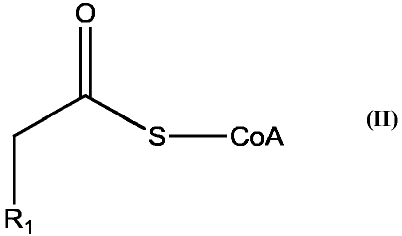 with a compound of formula (III) 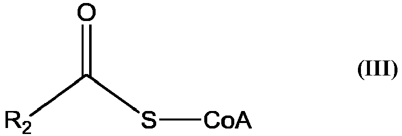
wherein R₁ and R₂ are independently selected from the group consisting of linear or branched C₁-C₆ alkyl, linear or branched C₂-C₆ alkenyl, linear or branched C₂-C₆ alkynyl, aryl, aryl-alkyl and benzyl, and S-CoA is coenzyme A,
said mutant thiolase having at least an amino acid substitution at a position corresponding to position 293 in the polypeptide of SEQ ID NO: 1 wherein the thiolase shows at least a 75% sequence identity with a thiolase selected from the group consisting of SEQ ID NO:1, 2, or 3, with the proviso that said thiolase is not a thiolase consisting of the sequence SEQ ID NO: 7 or SEQ ID NO: 8, and wherein said mutant thiolase displays a specific thiolase activity of at least 0,05mU/mg for the catalysis of a branched product of formula (I) when compound of formula (II) is butyryl-CoA and compound of formula (III) is acetyl-CoA,

2. The thiolase according to claim 1, wherein the sequence in said thiolase corresponding to the thiolase signature having the accession number PS00098 in the Prosite database has an identity of at least 35% to the sequence corresponding to said signature in the polypeptide of SEQ ID NO:1, wherein the sequence in said thiolase corresponding to the thiolase signature having the accession number PS00737 in the Prosite database has an identity of at least 50 % to the sequence corresponding to said signature in the polypeptide of SEQ ID NO:1 and/or wherein the sequence in said thiolase corresponding to the thiolase signature having the accession number PS00099 in the Prosite database has an identity of at least 25% to the sequence corresponding to said signature in the polypeptide of SEQ ID NO:1.

3. The thiolase according to claim 2, wherein the mutant thiolase is selected from the group consisting of:
(i) The polypeptide of SEQ ID NO: 1, wherein the at least one mutation is located at position Phe293;
(ii) The polypeptide of SEQ ID NO: 2 wherein the at least one mutation is located at position Met290,
(iii)The polypeptide of SEQ ID NO: 3, wherein the at least one mutation is located at position Met289.

4. The thiolase according to claim 3, wherein the mutant thiolase is the polypeptide of SEQ ID NO: 1 wherein the at least one mutation located at position Phe293 is selected from the group consisting of Phe293Asp, Phe293Ala, Phe293Ser, Phe293Gly and Phe293Glu.

5. The thiolase according to claim 3, wherein the mutant thiolase is the polypeptide of SEQ ID NO: 2 wherein the at least one mutation at position Met290 is selected from the group consisting of Met290Asp, Met290Ala, Met290Ser, Met290Gly and Met290Glu.

6. The thiolase according to claim 3, wherein the mutant thiolase is the polypeptide of SEQ ID NO: 3, wherein at least one mutation located at position Met289 is selected from the group consisting of Met289Asp, Met289Ala, Met289Ser, Met289Gly and Met289Glu.

7. The thiolase according to any of claims 1 to 6 further comprising at least one additional mutation at a position selected from the group consisting of the residues corresponding to positions 90 and 383 in the polypeptide of SEQ ID NO: 1.

8. The thiolase according to any of claims 1 to 7 wherein R₁ is an ethyl group and/or R₂ is a methyl group.

9. A nucleic acid encoding a thiolase according to any of claims 1 to 8, or the vector comprising the nucleic acid encoding a thiolase according to any of claims 1 to 8, or a host cell comprising the nucleic acid encoding a thiolase according to any of claims 1 to 8, or the host cell comprising vector comprising the nucleic acid encoding a thiolase according to any of claims 1 to 8.

10. A process for the production of a compound of formula (I) comprising contacting a compound of formula (II) and a compound of formula (III) in the presence of a mutant thiolase capable of catalyzing the formation of a compound of formula (I) 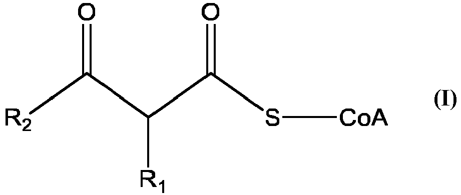 by the condensation of a compound of formula (II) 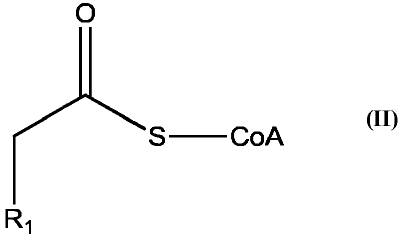 with a compound of formula (III) 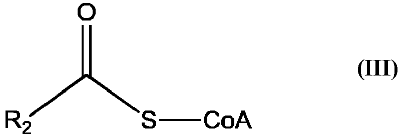
wherein R₁ and R₂ are independently selected from the group consisting of linear or branched C₁-C₆ alkyl, linear or branched C₂-C₆ alkenyl, linear or branched C₂-C₆ alkynyl, aryl, aryl-alkyl and benzyl, and S-CoA is coenzyme A,
said mutant thiolase having at least an amino acid substitution with respect to the naturally-occurring thiolase, wherein said amino acid substitution is located at a position corresponding to position 293 in the polypeptide of SEQ ID NO: 1, wherein said thiolase has at least a 75% sequence identity with a thiolase selected from the group consisting of of SEQ ID NO:1, 2, or 3 and displays a specific thiolase activity of at least 0,05mU/mg for the catalysis of a branched product of formula (I) when compound of formula (II) is butyryl-CoA and compound of formula (III) is acetyl-CoA; and wherein said contacting is carried out under conditions adequate for the condensation of compounds of formula (II) and (III) into a compound of formula (I).

11. The process according t claim 10 wherein the thiolase is not a thiolase consisting of the sequence SEQ ID NO: 7 or SEQ ID NO: 8.

12. The process according to claim 10, wherein:
the sequence in said thiolase corresponding to the thiolase signature having the accession number PS00098 in the Prosite database has an identity of at least 35% to the sequence corresponding to said signature in the thiolase of SEQ ID NO:1, wherein the sequence in said thiolase corresponding to the thiolase signature having the accession number PS00737 in the Prosite database has an identity of at least 50% to the sequence corresponding to said signature in the thiolase of SEQ ID NO:1 and/or wherein the sequence in said thiolase corresponding to the thiolase signature having the accession number PS00099 in the Prosite database has an identity of at least 25% to the sequence corresponding to said signature in the thiolase of SEQ ID NO:1.

13. The process according to any of claims 10 to 12, wherein the mutant thiolase is selected from the group consisting of:
(i) The polypeptide of SEQ ID NO: 1 wherein the at least one mutation is located at position Phe293;
(ii) The polypeptide of SEQ ID NO: 2 wherein the at least one mutation is located at position Met290;
(iii)The polypeptide of SEQ ID NO: 3, wherein the at least one mutation is located at position Met289.

14. The process according to claim 13, wherein the mutant thiolase is the polypeptide of SEQ ID NO: 1 wherein the at least one mutation located at position Phe293 is selected from the group consisting of Phe293Asp, Phe293Ala, Phe293Ser, Phe293Gly and Phe293Glu.

15. The process according to claim 13, wherein the mutant thiolase is the polypeptide of SEQ ID NO: 2 wherein the at least one mutation at position Met290 is selected from the group consisting of Met290Asp, Met290Ala, Met290Ser, Met290Gly and Met290Glu.

16. The process according to claim 13, wherein the mutant thiolase is the polypeptide of SEQ ID NO: 3, wherein at least one mutation located at position Met289 is selected from the group consisting of Met289Asp, Met289Ala, Met289Ser, Met289Gly and Met289Glu.

17. The process according to any of claims 10 to 16 wherein the thiolase further comprises at least one additional mutation at a position selected from the group consisting of the residues corresponding to positions 90 and 383 in the polypeptide of SEQ ID NO: 1.

18. The process according to any of claims 10 to 17 wherein R₁ is an ethyl group and/or wherein R2 is a methyl group.

19. The process according to any of claims 10 to 17 further comprising contacting the compound of formula (I) with a polypeptide having beta-ketoacyl-CoA reductase activity capable of catalyzing the formation of a compound of formula (IV) 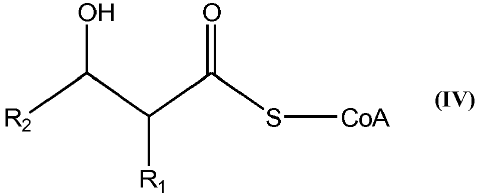 from the compound of formula (I), wherein R₁, R₂ and S-CoA are as defined in claim 1, and wherein the polypeptide having beta-ketoacyl-CoA reductase activity consists of the sequence of SEQ ID NO: 6.

## Patentansprüche

1. Eine mutierte Thiolase, die in der Lage ist, die Bildung einer Verbindung der Formel (I) 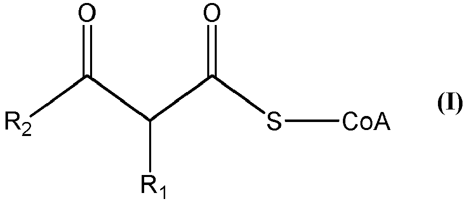 zu katalysieren, durch die Kondensation einer Verbindung der Formel (II) 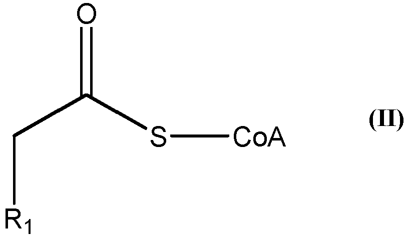 mit einer Verbindung der Formel (III) 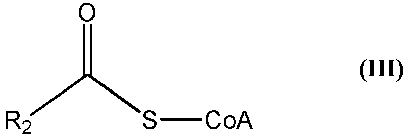
wobei R₁ und R₂ unabhängig voneinander ausgewählt sind aus der Gruppe, die aus linearem oder verzweigtem C₁-C₆-Alkyl, linearem oder verzweigtem C₂-C₆-Alkenyl, linearem oder verzweigtem C₂-C₆-Alkinyl, Aryl, Arylalkyl und Benzyl besteht, und S-CoA Coenzym A ist,
die mutierte Thiolase mindestens eine Aminosäuresubstitution an einer Position aufweist, die der Position 293 in dem Polypeptid von SEQ ID Nr.: 1 entspricht, wobei die Thiolase mindestens 75 % Sequenzidentität zu einer Thiolase aufweist, die ausgewählt ist aus der Gruppe, die aus SEQ ID Nr.: 1, 2 oder 3 besteht, mit der Bedingung, dass die Thiolase keine Thiolase ist, die aus der Sequenz SEQ ID Nr.: 7 oder SEQ ID Nr.: 8 besteht, und
wobei die mutierte Thiolase eine spezifische Thiolaseaktivität von mindestens 0,05 mU/mg für die Katalyse eines verzweigten Produkts der Formel (I) zeigt, wenn die Verbindung der Formel (II) Butyryl-CoA ist, und die Verbindung der Formel (III) Acetyl-CoA ist.

2. Die Thiolase nach Anspruch 1, wobei die Sequenz in der Thiolase, die der Thiolase Signatur mit der Zugriffsnummer PS00098 in der Prosite-Datenbank entspricht, eine Identität von mindestens 35 % zu der Sequenz aufweist, die der Signatur des Polypeptids der SEQ ID Nr.: 1 entspricht,
wobei die Sequenz in der Thiolase, die der Thiolase Signatur mit der Zugriffsnummer PS00737 in der Prosite-Datenbank entspricht, eine Identität von mindestens 50 % zu der Sequenz aufweist, die der Signatur des Polypeptids der SEQ ID Nr.: 1 entspricht, und/oder
wobei die Sequenz in der Thiolase, der Thiolase Signatur mit der Zugriffsnummer PS00099 in der Prosite-Datenbank entspricht, eine Identität von mindestens 25 % zu der Sequenz aufweist, die der Signatur in dem Polypeptid der SEQ ID Nr.: 1 entspricht.

3. Die Thiolase nach Anspruch 2, wobei die mutierte Thiolase ausgewählt ist aus der Gruppe bestehend aus:
(i) dem Polypeptid von SEQ ID Nr.: 1, wobei sich mindestens eine Mutation an der Position Phe293 befindet;
(ii) dem Polypeptid von SEQ ID Nr.: 2, wobei sich mindestens eine Mutation an der Position Met290 befindet;
(iii) Das Polypeptid von SEQ ID Nr.: 3, wobei sich mindestens eine Mutation an der Position Met289 befindet.

4. Die Thiolase nach Anspruch 3, wobei die mutierte Thiolase das Polypeptid von SEQ ID Nr.: 1 ist, wobei die mindestens eine Mutation, die sich an der Position Phe293 befindet, aus der Gruppe ausgewählt ist, die aus Phe293Asp, Phe293Ala, Phe293Ser, Phe293Gly und Phe293Glu besteht.

5. Die Thiolase nach Anspruch 3, wobei die mutierte Thiolase das Polypeptid von SEQ ID Nr.: 2 ist, wobei die mindestens eine Mutation an der Position Met290 aus der Gruppe ausgewählt ist, die aus Met290Asp, Met290Ala, Met290Ser, Met290Gly und Met290Glu besteht

6. Die Thiolase nach Anspruch 3, wobei die mutierte Thiolase das Polypeptid von SEQ ID Nr.: 3 ist, wobei mindestens eine Mutation, die sich an der Position Met289 befindet, aus der Gruppe ausgewählt ist, die aus Met289Asp, Met289Ala, Met289Ser, Met289Gly und Met289Glu besteht.

7. Die Thiolase nach einem der Ansprüche 1 bis 6, ferner umfassend mindestens eine zusätzliche Mutation an einer Position, die aus der Gruppe ausgewählt ist, die aus den Resten, die den Positionen 90 und 383 im Polypeptid der SEQ ID Nr.: 1 entsprechen, besteht.

8. Die Thiolase nach einem der Ansprüche 1 bis 7, wobei R₁ eine Ethylgruppe und/oder R₂ eine Methylgruppe ist.

9. Eine Nukleinsäure, die eine Thiolase nach einem der Ansprüche 1 bis 8 kodiert, oder ein Vektor der die Nukleinsäure umfasst, die eine Thiolase nach einem der Ansprüche 1 bis 8 kodiert, oder eine Wirtszelle, die die Nukleinsäure umfasst, die eine Thiolase nach einem der Ansprüche 1 bis 8 kodiert, oder eine Wirtszelle, die den Vektor umfasst, der die Nukleinsäure umfasst, die eine Thiolase nach einem der Ansprüche 1 bis 8 kodiert.

10. Ein Verfahren zur Herstellung einer Verbindung der Formel (I), umfassend das Kontaktieren einer Verbindung der Formel (II) und einer Verbindung der Formel (III) in Gegenwart einer mutierten Thiolase, die in der Lage ist, die Bildung einer Verbindung der Formel (I) zu katalysieren 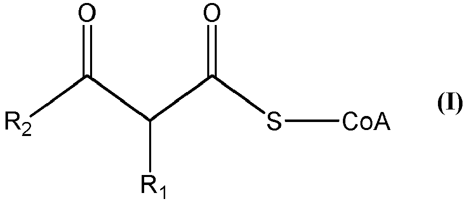 durch die Kondensation einer Verbindung der Formel (II) 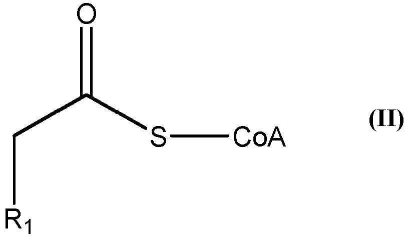 mit einer Verbindung der Formel (III) 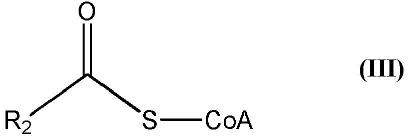
wobei R₁ und R₂ unabhängig voneinander ausgewählt sind aus der Gruppe, die aus linearem oder verzweigtem C₁-C₆-Alkyl, linearem oder verzweigtem C₂-C₆-Alkenyl, linearem oder verzweigtem C₂-C₆-Alkinyl, Aryl, Arylalkyl und Benzyl besteht, und S-CoA Coenzym A ist,
die mutierte Thiolase mindestens eine Aminosäuresubstitution in Bezug auf eine natürlich vorkommende Thiolase aufweist,
wobei sich die Aminosäuresubstitution an einer Position befindet, die der Position 293 in dem Polypeptid von SEQ ID Nr.: 1 entspricht,
wobei die Thiolase mindestens 75 % Sequenzidentität zu einer Thiolase aufweist, die ausgewählt ist aus der Gruppe, bestehend aus SEQ ID Nr.: 1, 2 oder 3, und eine spezifische Thiolaseaktivität von mindestens 0,05 mU/mg für die Katalyse eines verzweigten Produkts der Formel (I) zeigt, wenn die Verbindung der Formel (II) Butyryl-CoA ist, und die Verbindung der Formel (III) Acetyl-CoA ist; und
wobei das Kontaktieren unter Bedingungen durchgeführt wird, die für die Kondensation der Verbindungen der Formel (II) und (III) zu einer Verbindung der Formel (I) geeignet sind.

11. Das Verfahren nach Anspruch 10, wobei die Thiolase keine Thiolase ist, die aus der Sequenz SEQ ID Nr.: 7 oder SEQ ID Nr.: 8 besteht.

12. Das Verfahren nach Anspruch 10, wobei:
die Sequenz in der Thiolase, die der Thiolase Signatur mit der Zugriffsnummer PS00098 in der Prosite-Datenbank entspricht, eine Identität von mindestens 35 % zu der Sequenz aufweist, die der Signatur der Thiolase der SEQ ID Nr.: 1 entspricht,
wobei die Sequenz in der Thiolase, die der Thiolase Signatur mit der Zugriffsnummer PS00737 in der Prosite-Datenbank entspricht, eine Identität von mindestens 50 % zu der Sequenz aufweist, die der Signatur der Thiolase der SEQ ID Nr.: 1 entspricht, und/oder wobei die Sequenz der Thiolase, die der Thiolase Signatur mit der Zugriffsnummer PS00099 in der Prosite-Datenbank entspricht, eine Identität von mindestens 25 % zu der Sequenz aufweist, die der Signatur der Thiolase der SEQ ID Nr.: 1 entspricht.

13. Das Verfahren nach einem der Ansprüche 10 bis 12, wobei die mutierte Thiolase ausgewählt ist aus der Gruppe bestehend aus:
(i) dem Polypeptid von SEQ ID Nr.: 1, wobei sich mindestens eine Mutation an der Position Phe293 befindet;
(ii) dem Polypeptid von SEQ ID Nr.: 2, wobei sich mindestens eine Mutation an der Position Met290 befindet;
(iii) das Polypeptid von SEQ ID Nr.: 3, wobei sich mindestens eine Mutation an der Position Met289 befindet.

14. Das Verfahren nach Anspruch 13, wobei die mutierte Thiolase das Polypeptid von SEQ ID Nr.: 1 ist, wobei die mindestens eine Mutation, die sich an der Position Phe293 befindet, aus der Gruppe ausgewählt ist, die aus Phe293Asp, Phe293Ala, Phe293Ser, Phe293Gly und Phe293Glu besteht.

15. Das Verfahren nach Anspruch 13, wobei die mutierte Thiolase das Polypeptid von SEQ ID Nr.: 2 ist, wobei die mindestens eine Mutation an der Position Met290 aus der Gruppe ausgewählt ist, die aus Met290Asp, Met290Ala, Met290Ser, Met290Gly und Met290Glu besteht.

16. Das Verfahren nach Anspruch 13, wobei die mutierte Thiolase das Polypeptid von SEQ ID Nr.: 3 ist, wobei mindestens eine Mutation an der Position Met289 aus der Gruppe ausgewählt ist, die aus Met289Asp, Met289Ala, Met289Ser, Met289Gly und Met289Glu besteht,

17. Das Verfahren nach einem der Ansprüche 10 bis 16, wobei die Thiolase mindestens eine zusätzliche Mutation an einer Position umfasst, die ausgewählt ist aus der Gruppe, die aus den Resten, die den Positionen 90 und 383 im Polypeptid von SEQ ID Nr.: 1 entsprechen, besteht.

18. Das Verfahren nach einem der Ansprüche 10 bis 17, wobei R₁ eine Ethylgruppe ist und/oder wobei R₂ eine Methylgruppe ist.

19. Das Verfahren nach einem der Ansprüche 10 bis 17, umfasst weiterhin das Kontaktieren der Verbindung der Formel (I) mit einem Polypeptid mit Beta-Ketoacyl-CoA-Reduktase-Aktivität, das in der Lage ist, die Bildung einer Verbindung der Formel (IV) 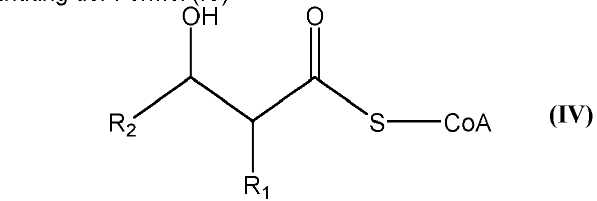 zu katalysieren aus der Verbindung der Formel (I), wobei R₁, R₂ und S-CoA wie in Anspruch 1 definiert sind, und wobei das Polypeptid mit Beta-Ketoacyl-CoA-Reduktase-Aktivität aus der Sequenz von SEQ ID Nr.: 6 besteht.

## Revendications

1. Thiolase mutante capable de catalyser la formation d'un composé de formule (I) 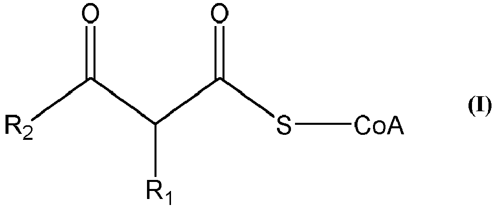 par condensation d'un composé de formule (II) 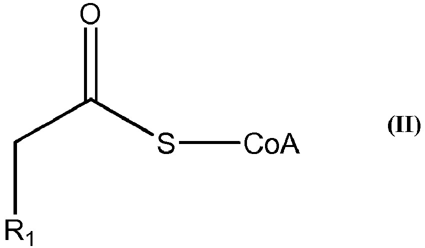 avec un composé de formule (III) 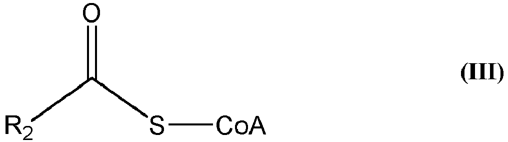
formules dans lesquelles R₁ et R₂ sont indépendamment choisis dans le groupe constitué par alkyle en C₁ à C₆ linéaire ou ramifié, alcényle en C₂ à C₆ linéaire ou ramifié, alcynyle en C₂ à C₆ linéaire ou ramifié, aryle, aryl-alkyle et benzyle, et S-CoA est la coenzyme A,
ladite thiolase mutante ayant au moins une substitution d'acide aminé à une position correspondant à la position 293 dans le polypeptide de la SEQ ID NO : 1, où la thiolase présente une identité de séquence d'au moins 75 % avec une thiolase choisie dans le groupe constitué par les SEQ ID NO : 1, 2 et 3, sous réserve que ladite thiolase ne soit pas une thiolase consistant en la séquence de la SEQ ID NO : 7 ou de la SEQ ID NO : 8, et où ladite thiolase mutante présente une activité de thiolase spécifique d'au moins 0,05 mU/mg pour la catalyse d'un produit ramifié de formule (I) quand le composé de formule (II) est la butyryl-CoA et le composé de formule (III) est l'acétyl-CoA.

2. Thiolase selon la revendication 1, où la séquence dans ladite thiolase correspondant à la signature de thiolase ayant le numéro d'accès PS00098 dans la base de données Prosite a une identité d'au moins 35 % avec la séquence correspondant à ladite signature dans le polypeptide de la SEQ ID NO : 1, où la séquence dans ladite thiolase correspondant à la signature de thiolase ayant le numéro d'accès PS00737 dans la base de données Prosite a une identité d'au moins 50 % avec la séquence correspondant à ladite signature dans le polypeptide de la SEQ ID NO : 1, et/ou où la séquence dans ladite thiolase correspondant à la signature de thiolase ayant le numéro d'accès PS00099 dans la base de données Prosite a une identité d'au moins 25 % avec la séquence correspondant à ladite signature dans le polypeptide de la SEQ ID NO : 1.

3. Thiolase selon la revendication 2, où la thiolase mutante est choisie dans le groupe constitué par :
(i) le polypeptide de la SEQ ID NO : 1, où l'au moins une mutation est située à la position Phe293 ;
(ii) le polypeptide de la SEQ ID NO : 2, où l'au moins une mutation est située à la position Met290 ;
(iii) le polypeptide de la SEQ ID NO : 3, où l'au moins une mutation est située à la position Met289.

4. Thiolase selon la revendication 3, où la thiolase mutante est le polypeptide de la SEQ ID NO : 1 où l'au moins une mutation située à la position Phe293 est choisie dans le groupe constitué par Phe293Asp, Phe293Ala, Phe293Ser, Phe293Gly et Phe293Glu.

5. Thiolase selon la revendication 3, où la thiolase mutante est le polypeptide de la SEQ ID NO : 2 où l'au moins une mutation située à la position Met290 est choisie dans le groupe constitué par Met290Asp, Met290Ala, Met290Ser, Met290Gly et Met290Glu.

6. Thiolase selon la revendication 3, où la thiolase mutante est le polypeptide de la SEQ ID NO : 3 où l'au moins une mutation située à la position Met289 est choisie dans le groupe constitué par Met289Asp, Met289Ala, Met289Ser, Met289Gly et Met289Glu.

7. Thiolase selon l'une quelconque des revendications 1 à 6, comprenant en outre au moins une mutation additionnelle à une position choisie dans le groupe constitué par les résidus correspondant aux positions 90 et 383 dans le polypeptide de la SEQ ID NO : 1.

8. Thiolase selon l'une quelconque des revendications 1 à 7, dans laquelle R₁ est un groupe éthyle et/ou R₂ est un groupe méthyle.

9. Acide nucléique codant pour une thiolase de l'une quelconque des revendications 1 à 8, ou vecteur comprenant l'acide nucléique codant pour une thiolase de l'une quelconque des revendications 1 à 8, ou cellule hôte comprenant l'acide nucléique codant pour une thiolase de l'une quelconque des revendications 1 à 8, ou cellule hôte comprenant un vecteur comprenant l'acide nucléique codant pour une thiolase de l'une quelconque des revendications 1 à 8.

10. Procédé pour la production d'un composé de formule (I), comprenant la mise en contact d'un composé de formule (II) et d'un composé de formule (III) en présence d'une thiolase mutante capable de catalyser la formation d'un composé de formule (I) 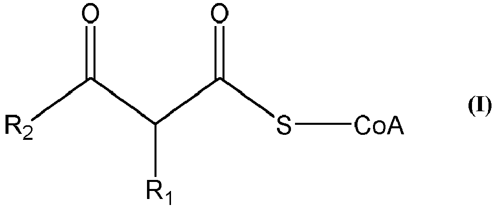 par condensation d'un composé de formule (II) 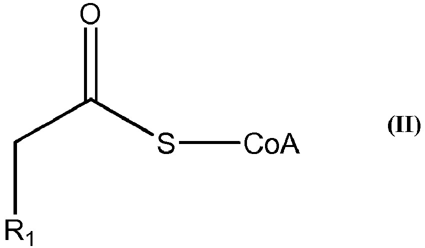 avec un composé de formule (III) 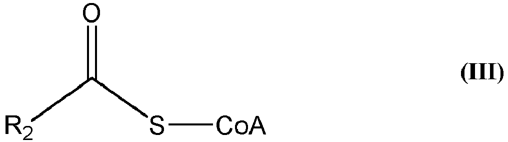
formules dans lesquelles R₁ et R₂ sont indépendamment choisis dans le groupe constitué par alkyle en C₁ à C₆ linéaire ou ramifié, alcényle en C₂ à C₆ linéaire ou ramifié, alcynyle en C₂ à C₆ linéaire ou ramifié, aryle, aryl-alkyle et benzyle, et S-CoA est la coenzyme A,
ladite thiolase mutante ayant au moins une substitution d'acide aminé par rapport à la thiolase naturelle, où ladite substitution d'acide aminé est située à une position correspondant à la position 293 dans le polypeptide de la SEQ ID NO : 1, où ladite thiolase a une identité de séquence d'au moins 75 % avec une thiolase choisie dans le groupe constitué par les SEQ ID NO : 1, 2 et 3 et présente une activité de thiolase spécifique d'au moins 0,05 mU/mg pour la catalyse d'un produit ramifié de formule (I) quand le composé de formule (II) est la butyryl-CoA et le composé de formule (III) est l'acétyl-CoA ; et dans lequel ladite mise en contact est effectuée dans des conditions adéquates pour la condensation de composés de formules (II) et (III) en un composé de formule (I) .

11. Procédé selon la revendication 10, dans lequel la thiolase n'est pas une thiolase consistant en la séquence de la SEQ ID NO : 7 ou de la SEQ ID NO : 8.

12. Procédé selon la revendication 10, dans lequel :
la séquence dans ladite thiolase correspondant à la signature de thiolase ayant le numéro d'accès PS00098 dans la base de données Prosite a une identité d'au moins 35 % avec la séquence correspondant à ladite signature dans la thiolase de la SEQ ID NO : 1, où la séquence dans ladite thiolase correspondant à la signature de thiolase ayant le numéro d'accès PS00737 dans la base de données Prosite a une identité d'au moins 50 % avec la séquence correspondant à ladite signature dans la thiolase de la SEQ ID NO : 1, et/ou où la séquence dans ladite thiolase correspondant à la signature de thiolase ayant le numéro d'accès PS00099 dans la base de données Prosite a une identité d'au moins 25 % avec la séquence correspondant à ladite signature dans la thiolase de la SEQ ID NO : 1.

13. Procédé selon l'une quelconque des revendications 10 à 12, dans lequel la thiolase mutante est choisie dans le groupe constitué par :
(i) le polypeptide de la SEQ ID NO : 1, où l'au moins une mutation est située à la position Phe293 ;
(ii) le polypeptide de la SEQ ID NO : 2, où l'au moins une mutation est située à la position Met290 ;
(iii) le polypeptide de la SEQ ID NO : 3, où l'au moins une mutation est située à la position Met289.

14. Procédé selon la revendication 13, dans lequel la thiolase mutante est le polypeptide de la SEQ ID NO : 1 où l'au moins une mutation située à la position Phe293 est choisie dans le groupe constitué par Phe293Asp, Phe293Ala, Phe293Ser, Phe293Gly et Phe293Glu.

15. Procédé selon la revendication 13, dans lequel la thiolase mutante est le polypeptide de la SEQ ID NO : 2 où l'au moins une mutation située à la position Met290 est choisie dans le groupe constitué par Met290Asp, Met290Ala, Met290Ser, Met290Gly et Met290Glu.

16. Procédé selon la revendication 13, dans lequel la thiolase mutante est le polypeptide de la SEQ ID NO : 3 où l'au moins une mutation située à la position Met289 est choisie dans le groupe constitué par Met289Asp, Met289Ala, Met289Ser, Met289Gly et Met289Glu.

17. Procédé selon l'une quelconque des revendications 10 à 16, dans lequel la thiolase comprend en outre au moins une mutation additionnelle à une position choisie dans le groupe constitué par les résidus correspondant aux positions 90 et 383 dans le polypeptide de la SEQ ID NO : 1.

18. Procédé selon l'une quelconque des revendications 10 à 17, dans lequel R₁ est un groupe éthyle et/ou dans lequel R₂ est un groupe méthyle.

19. Procédé selon l'une quelconque des revendications 10 à 17, comprenant en outre la mise en contact du composé de formule (I) avec un polypeptide ayant une activité de bêta-cétoacyl-CoA réductase capable de catalyser la formation d'un composé de formule (IV) 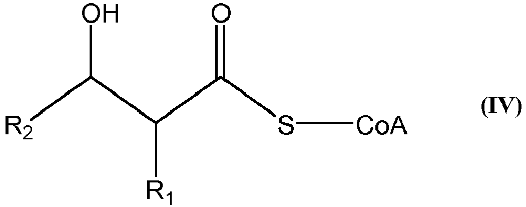 à partir du composé de formule (I), où R₁, R₂ et S-CoA sont tels que définis dans la revendication 1, et dans lequel le polypeptide ayant une activité de bêta-cétoacyl-CoA réductase consiste en la séquence de la SEQ ID NO : 6.


### SEQUENCE LISTING

<110> REPSOL, S.A.
<120> MODIFIED THIOLASES CAPABLE OF PRODUCING BRANCHED COMPOUNDS AND USES THEREOF
<130> P12331PC00
<150> EP15382676
   <151> 2015-12-29
<160> 17
<170> PatentIn version 3.5
<210> 1
   <211> 398
   <212> PRT
   <213> Saccharomyces cerevisiae
<400> 1
   Met Ser Gln Asn Val Tyr Ile Val Ser Thr Ala Arg Thr Pro Ile Gly
   1               5                   10                  15
   Ser Phe Gln Gly Ser Leu Ser Ser Lys Thr Ala Val Glu Leu Gly Ala
               20                  25                  30
   Val Ala Leu Lys Gly Ala Leu Ala Lys Val Pro Glu Leu Asp Ala Ser
           35                  40                  45
   Lys Asp Phe Asp Glu Ile Ile Phe Gly Asn Val Leu Ser Ala Asn Leu
       50                  55                  60
   Gly Gln Ala Pro Ala Arg Gln Val Ala Leu Ala Ala Gly Leu Ser Asn
   65                  70                  75                  80
   His Ile Val Ala Ser Thr Val Asn Lys Val Cys Ala Ser Ala Met Lys
                   85                  90                  95
   Ala Ile Ile Leu Gly Ala Gln Ser Ile Lys Cys Gly Asn Ala Asp Val
               100                 105                 110
   Val Val Ala Gly Gly Cys Glu Ser Met Thr Asn Ala Pro Tyr Tyr Met
           115                 120                 125
   Pro Ala Ala Arg Ala Gly Ala Lys Phe Gly Gln Thr Val Leu Val Asp
       130                 135                 140
   Gly Val Glu Arg Asp Gly Leu Asn Asp Ala Tyr Asp Gly Leu Ala Met
   145                 150                 155                 160
   Gly Val His Ala Glu Lys Cys Ala Arg Asp Trp Asp Ile Thr Arg Glu
                   165                 170                 175
   Gln Gln Asp Asn Phe Ala Ile Glu Ser Tyr Gln Lys Ser Gln Lys Ser
               180                 185                 190
   Gln Lys Glu Gly Lys Phe Asp Asn Glu Ile Val Pro Val Thr Ile Lys
           195                 200                 205
   Gly Phe Arg Gly Lys Pro Asp Thr Gln Val Thr Lys Asp Glu Glu Pro
       210                 215                 220
   Ala Arg Leu His Val Glu Lys Leu Arg Ser Ala Arg Thr Val Phe Gln
   225                 230                 235                 240
   Lys Glu Asn Gly Thr Val Thr Ala Ala Asn Ala Ser Pro Ile Asn Asp
                   245                 250                 255
   Gly Ala Ala Ala Val Ile Leu Val Ser Glu Lys Val Leu Lys Glu Lys
               260                 265                 270
   Asn Leu Lys Pro Leu Ala Ile Ile Lys Gly Trp Gly Glu Ala Ala His
           275                 280                 285
   Gln Pro Ala Asp Phe Thr Trp Ala Pro Ser Leu Ala Val Pro Lys Ala
       290                 295                 300
   Leu Lys His Ala Gly Ile Glu Asp Ile Asn Ser Val Asp Tyr Phe Glu
   305                 310                 315                 320
   Phe Asn Glu Ala Phe Ser Val Val Gly Leu Val Asn Thr Lys Ile Leu
                   325                 330                 335
   Lys Leu Asp Pro Ser Lys Val Asn Val Tyr Gly Gly Ala Val Ala Leu
               340                 345                 350
   Gly His Pro Leu Gly Cys Ser Gly Ala Arg Val Val Val Thr Leu Leu
           355                 360                 365
   Ser Ile Leu Gln Gln Glu Gly Gly Lys Ile Gly Val Ala Ala Ile Cys
       370                 375                 380
   Asn Gly Gly Gly Gly Ala Ser Ser Ile Val Ile Glu Lys Ile
   385                 390                 395
<210> 2
   <211> 394
   <212> PRT
   <213> Ralstonia eutropha
<400> 2
   Met Thr Arg Glu Val Val Val Val Ser Gly Val Arg Thr Ala Ile Gly
   1               5                   10                  15
   Thr Phe Gly Gly Ser Leu Lys Asp Val Ala Pro Ala Glu Leu Gly Ala
               20                  25                  30
   Leu Val Val Arg Glu Ala Leu Ala Arg Ala Gln Val Ser Gly Asp Asp
           35                  40                  45
   Val Gly His Val Val Phe Gly Asn Val Ile Gln Thr Glu Pro Arg Asp
       50                  55                  60
   Met Tyr Leu Gly Arg Val Ala Ala Val Asn Gly Gly Val Thr Ile Asn
   65                  70                  75                  80
   Ala Pro Ala Leu Thr Val Asn Arg Leu Cys Gly Ser Gly Leu Gln Ala
                   85                  90                  95
   Ile Val Ser Ala Ala Gln Thr Ile Leu Leu Gly Asp Thr Asp Val Ala
               100                 105                 110
   Ile Gly Gly Gly Ala Glu Ser Met Ser Arg Ala Pro Tyr Leu Ala Pro
           115                 120                 125
   Ala Ala Arg Trp Gly Ala Arg Met Gly Asp Ala Gly Leu Val Asp Met
       130                 135                 140
   Met Leu Gly Ala Leu His Asp Pro Phe His Arg Ile His Met Gly Val
   145                 150                 155                 160
   Thr Ala Glu Asn Val Ala Lys Glu Tyr Asp Ile Ser Arg Ala Gln Gln
                   165                 170                 175
   Asp Glu Ala Ala Leu Glu Ser His Arg Arg Ala Ser Ala Ala Ile Lys
               180                 185                 190
   Ala Gly Tyr Phe Lys Asp Gln Ile Val Pro Val Val Ser Lys Gly Arg
           195                 200                 205
   Lys Gly Asp Val Thr Phe Asp Thr Asp Glu His Val Arg His Asp Ala
       210                 215                 220
   Thr Ile Asp Asp Met Thr Lys Leu Arg Pro Val Phe Val Lys Glu Asn
   225                 230                 235                 240
   Gly Thr Val Thr Ala Gly Asn Ala Ser Gly Leu Asn Asp Ala Ala Ala
   245 250 255
   Ala Val Val Met Met Glu Arg Ala Glu Ala Glu Arg Arg Gly Leu Lys
   260 265 270
   Pro Leu Ala Arg Leu Val Ser Tyr Gly His Ala Gly Val Asp Pro Lys
   275 280 285
   Ala Met Gly Ile Gly Pro Val Pro Ala Thr Lys Ile Ala Leu Glu Arg
   290 295 300
   Ala Gly Leu Gln Val Ser Asp Leu Asp Val Ile Glu Ala Asn Glu Ala
   305 310 315 320
   Phe Ala Ala Gln Ala Cys Ala Val Thr Lys Ala Leu Gly Leu Asp Pro
   325 330 335
   Ala Lys Val Asn Pro Asn Gly Ser Gly Ile Ser Leu Gly His Pro Ile
   340 345 350
   Gly Ala Thr Gly Ala Leu Ile Thr Val Lys Ala Leu His Glu Leu Asn
   355 360 365
   Arg Val Gln Gly Arg Tyr Ala Leu Val Thr Met Cys Ile Gly Gly Gly
   370 375 380
   Gln Gly Ile Ala Ala Ile Phe Glu Arg Ile
   385 390
<210> 3
   <211> 394
   <212> PRT
   <213> Escherichia coli
<400> 3
   Met Lys Asn Cys Val Ile Val Ser Ala Val Arg Thr Ala Ile Gly Ser
   1 5 10 15
   Phe Asn Gly Ser Leu Ala Ser Thr Ser Ala Ile Asp Leu Gly Ala Thr
   20 25 30
   Val Ile Lys Ala Ala Ile Glu Arg Ala Lys Ile Asp Ser Gln His Val
   35 40 45
   Asp Glu Val Ile Met Gly Asn Val Leu Gln Ala Gly Leu Gly Gln Asn
   50 55 60
   Pro Ala Arg Gln Ala Leu Leu Lys Ser Gly Leu Ala Glu Thr Val Cys
   65                  70                  75                  80
   Gly Phe Thr Val Asn Lys Val Cys Gly Ser Gly Leu Lys Ser Val Ala
                   85                  90                  95
   Leu Ala Ala Gln Ala Ile Gln Ala Gly Gln Ala Gln Ser Ile Val Ala
               100                 105                 110
   Gly Gly Met Glu Asn Met Ser Leu Ala Pro Tyr Leu Leu Asp Ala Lys
           115                 120                 125
   Ala Arg Ser Gly Tyr Arg Leu Gly Asp Gly Gln Val Tyr Asp Val Ile
       130                 135                 140
   Leu Arg Asp Gly Leu Met Cys Ala Thr His Gly Tyr His Met Gly Ile
   145                 150                 155                 160
   Thr Ala Glu Asn Val Ala Lys Glu Tyr Gly Ile Thr Arg Glu Met Gln
                   165                 170                 175
   Asp Glu Leu Ala Leu His Ser Gln Arg Lys Ala Ala Ala Ala Ile Glu
               180                 185                 190
   Ser Gly Ala Phe Thr Ala Glu Ile Val Pro Val Asn Val Val Thr Arg
           195                 200                 205
   Lys Lys Thr Phe Val Phe Ser Gln Asp Glu Phe Pro Lys Ala Asn Ser
       210                 215                 220
   Thr Ala Glu Ala Leu Gly Ala Leu Arg Pro Ala Phe Asp Lys Ala Gly
   225                 230                 235                 240
   Thr Val Thr Ala Gly Asn Ala Ser Gly Ile Asn Asp Gly Ala Ala Ala
                   245                 250                 255
   Leu Val Ile Met Glu Glu Ser Ala Ala Leu Ala Ala Gly Leu Thr Pro
               260                 265                 270
   Leu Ala Arg Ile Lys Ser Tyr Ala Ser Gly Gly Val Pro Pro Ala Leu
           275                 280                 285
   Met Gly Met Gly Pro Val Pro Ala Thr Gln Lys Ala Leu Gln Leu Ala
       290                 295                 300
   Gly Leu Gln Leu Ala Asp Ile Asp Leu Ile Glu Ala Asn Glu Ala Phe
   305                 310                 315                 320
   Ala Ala Gln Phe Leu Ala Val Gly Lys Asn Leu Gly Phe Asp Ser Glu
   325 330 335
   Lys Val Asn Val Asn Gly Gly Ala Ile Ala Leu Gly His Pro Ile Gly
   340 345 350
   Ala Ser Gly Ala Arg Ile Leu Val Thr Leu Leu His Ala Met Gln Ala
   355 360 365
   Arg Asp Lys Thr Leu Gly Leu Ala Thr Leu Cys Ile Gly Gly Gly Gln
   370 375 380
   Gly Ile Ala Met Val Ile Glu Arg Leu Asn
   385 390
<210> 4
   <211> 401
   <212> PRT
   <213> Thermus thermophilus
<400> 4
   Met Pro Glu Ala Trp Ile Val Glu Ala Val Arg Thr Pro Ile Gly Lys
   1 5 10 15
   His Gly Gly Ala Leu Ala Ser Val Arg Pro Asp Asp Leu Leu Ala His
   20 25 30
   Ala Leu Ser Val Leu Val Asp Arg Ser Gly Val Pro Lys Glu Glu Val
   35 40 45
   Glu Asp Val Tyr Ala Gly Cys Ala Asn Gln Ala Gly Glu Asp Asn Arg
   50 55 60
   Asn Val Ala Arg Met Ala Leu Leu Leu Ala Gly Phe Pro Val Glu Val
   65 70 75 80
   Ala Gly Cys Thr Val Asn Arg Leu Cys Gly Ser Gly Leu Glu Ala Val
   85 90 95
   Ala Gln Ala Ala Arg Ala Ile Trp Ala Gly Glu Gly Lys Val Tyr Ile
   100 105 110
   Gly Ser Gly Val Glu Ser Met Ser Arg Ala Pro Tyr Ala Val Pro Lys
   115 120 125
   Pro Glu Arg Gly Phe Pro Thr Gly Asn Leu Val Met Tyr Asp Thr Thr
   130 135 140
   Leu Gly Trp Arg Phe Val Asn Pro Lys Met Gln Ala Leu Tyr Gly Thr
   145                 150                 155                 160
   Glu Ser Met Gly Glu Thr Ala Glu Asn Leu Ala Glu Met Tyr Gly Ile
                   165                 170                 175
   Arg Arg Glu Glu Gln Asp Arg Phe Ala Leu Leu Ser His Gln Lys Ala
               180                 185                 190
   Val Arg Ala Trp Glu Glu Gly Arg Phe Gln Asp Glu Val Val Pro Val
           195                 200                 205
   Pro Val Lys Arg Gly Lys Glu Glu Ile Leu Val Glu Gln Asp Glu Gly
       210                 215                 220
   Pro Arg Arg Asp Thr Ser Leu Glu Lys Leu Ala Ala Leu Arg Pro Val
   225                 230                 235                 240
   Phe Arg Glu Gly Gly Thr Val Thr Ala Gly Asn Ser Ser Pro Leu Asn
                   245                 250                 255
   Asp Gly Ala Ala Ala Val Leu Leu Val Ser Asp Asp Tyr Ala Lys Ala
               260                 265                 270
   His Gly Leu Arg Pro Leu Ala Arg Val Arg Ala Ile Ala Val Ala Gly
           275                 280                 285
   Val Pro Pro Arg Ile Met Gly Ile Gly Pro Val Pro Ala Thr Arg Lys
       290                 295                 300
   Ala Leu Glu Arg Ala Gly Leu Ser Phe Ser Asp Leu Gly Leu Ile Glu
   305                 310                 315                 320
   Leu Asn Glu Ala Phe Ala Ala Gln Ala Leu Ala Val Leu Arg Glu Trp
                   325                 330                 335
   Ser Leu Ser Met Glu Asp Gln Arg Leu Asn Pro Asn Gly Gly Ala Ile
               340                 345                 350
   Ala Leu Gly His Pro Leu Gly Ala Ser Gly Ala Arg Ile Leu Thr Thr
           355                 360                 365
   Leu Val His Glu Met Arg Arg Arg Lys Val Gln Phe Gly Leu Ala Thr
       370                 375                 380
   Met Cys Ile Gly Val Gly Gln Gly Ile Ala Val Val Val Glu Gly Met
   385 390 395 400
   Gly
<210> 5
   <211> 402
   <212> PRT
   <213> Flavobacteriaceae bacterium
<400> 5
   Met Pro Tyr Gln Lys Thr Val Lys Tyr Ile Met Gln Glu Val Phe Ile
   1 5 10 15
   Val Ser Ala Val Arg Thr Pro Ile Gly Ser Phe Leu Gly Ser Leu Ala
   20 25 30
   Gly Val Pro Ala Thr Lys Leu Gly Ser Ala Ala Ile Lys Gly Ala Leu
   35 40 45
   Glu Lys Ile Asn Leu Asp Pro Asn Gln Val Gln Glu Val Tyr Met Gly
   50 55 60
   Asn Val Leu Gln Ala Gly Glu Gly Gln Ala Pro Ala Arg Gln Ala Ala
   65 70 75 80
   Leu Gly Ala Gly Leu Ser Asn Thr Thr Pro Ala Thr Thr Val Asn Lys
   85 90 95
   Val Cys Ala Ser Gly Met Lys Ala Val Met Met Ala Ser Gln Ala Ile
   100 105 110
   Lys Cys Gly Asp Gln Asp Val Val Val Ala Gly Gly Met Glu Asn Met
   115 120 125
   Ser Leu Val Pro His Tyr Phe Asn Ala Arg Ser Ser Thr Lys Leu Gly
   130 135 140
   Glu Val Lys Met Gln Asp Gly Met Ile Leu Asp Gly Leu Thr Asp Val
   145 150 155 160
   Tyr Gly Lys Val His Met Gly Val Cys Ala Glu Lys Cys Ala Ala Glu
   165 170 175
   Asn Gly Phe Ser Arg Glu Asp Gln Asp Asn Phe Ala Val Glu Ser Tyr
   180 185 190
   Lys Arg Ser Ala Lys Ala Trp Ala Asp Gly Lys Phe Lys Asp Glu Val
                   195                 200                 205
   Val Ala Val Glu Ile Pro Gln Arg Lys Gly Asp Pro Val Ile Phe Ala
       210                 215                 220
   Glu Asp Glu Glu Tyr Lys Ser Val Asn Phe Asp Arg Ile Ser Thr Leu
   225                 230                 235                 240
   Pro Thr Val Phe Gln Lys Glu Asn Gly Thr Val Thr Ala Ala Asn Ala
                   245                 250                 255
   Ser Thr Ile Asn Asp Gly Ala Ser Ala Leu Ile Leu Met Ser Lys Asp
               260                 265                 270
   Lys Met Glu Glu Leu Gly Leu Lys Pro Leu Ala Lys Ile Val Ser Tyr
           275                 280                 285
   Ala Asp Ala Ala His Glu Pro Glu Trp Phe Thr Thr Ala Pro Ser Lys
       290                 295                 300
   Ala Leu Pro Ile Ala Leu Lys Lys Ala Gly Leu Glu Val Ser Asp Ile
   305                 310                 315                 320
   Asp Tyr Phe Glu Phe Asn Glu Ala Phe Ser Val Val Gly Leu Ala Asn
                   325                 330                 335
   Asn Lys Ile Leu Gly Leu Asp Ala Ser Lys Val Asn Val Asn Gly Gly
               340                 345                 350
   Ala Val Ser Leu Gly His Pro Leu Gly Ser Ser Gly Ser Arg Ile Ile
           355                 360                 365
   Val Thr Leu Ile Asn Ile Leu Lys Gln Asn Asn Gly Lys Tyr Gly Ala
       370                 375                 380
   Ala Ala Ile Cys Asn Gly Gly Gly Gly Ala Ser Ala Ile Val Ile Glu
   385                 390                 395                 400
   Asn Val
<210> 6
   <211> 255
   <212> PRT
   <213> Pseudomonas putida
<400> 6
   Met His Ile Ala Asn Lys His Phe Ile Val Ser Gly Ala Ala Ser Gly
   1               5                   10                  15
   Leu Gly Ala Ala Thr Ala Gln Met Leu Val Glu Ala Gly Ala Lys Val
               20                  25                  30
   Met Leu Val Asp Leu Asn Ala Gln Ala Val Glu Ala Lys Ala Arg Glu
           35                  40                  45
   Leu Gly Asp Asn Ala Arg Phe Ala Val Ala Asp Ile Ser Asp Glu Gln
       50                  55                  60
   Ala Ala Gln Ser Ala Val Asp Ala Ala Val Ser Ala Phe Gly Ser Leu
   65                  70                  75                  80
   His Gly Leu Val Asn Cys Ala Gly Ile Val Gly Ala Glu Lys Val Leu
                   85                  90                  95
   Gly Lys Gln Gly Pro His Gly Leu Ala Ser Phe Ala Lys Val Ile Asn
               100                 105                 110
   Val Asn Leu Ile Gly Ser Phe Asn Leu Leu Arg Leu Ala Ala Ala Ala
           115                 120                 125
   Met Ala Glu Gly Ala Ala Asp Glu Ser Gly Glu Arg Gly Val Ile Ile
       130                 135                 140
   Asn Thr Ala Ser Ile Ala Ala Tyr Asp Gly Gln Ile Gly Gln Ala Ala
   145                 150                 155                 160
   Tyr Ala Ala Ser Lys Gly Ala Ile Ala Ser Leu Thr Leu Pro Ala Ala
                   165                 170                 175
   Arg Glu Leu Ala Arg Phe Gly Ile Arg Val Met Thr Ile Ala Pro Gly
               180                 185                 190
   Ile Phe Glu Thr Pro Met Met Ala Gly Met Ser Glu Glu Val Arg Ala
           195                 200                 205
   Ser Leu Ala Ala Gly Val Pro Phe Pro Pro Arg Leu Gly Arg Pro Gln
       210                 215                 220
   Glu Tyr Ala Ala Leu Ala Arg His Ile Ile Glu Asn Ser Met Leu Asn
   225                 230                 235                 240
   Gly Glu Val Ile Arg Leu Asp Gly Ala Leu Arg Met Ala Ala Lys
                   245                 250                 255
<210> 7
   <211> 394
   <212> PRT
   <213> Ralstonia eutropha
<400> 7
   Met Thr Arg Glu Val Val Val Val Ser Gly Val Arg Thr Ala Ile Gly
   1               5                   10                  15
   Thr Phe Gly Gly Ser Leu Lys Asp Val Ala Pro Ala Glu Leu Gly Ala
               20                  25                  30
   Leu Val Val Arg Glu Ala Leu Ala Arg Ala Gln Val Ser Gly Asp Asp
           35                  40                  45
   Val Gly His Val Val Phe Gly Asn Val Ile Gln Thr Glu Pro Arg Asp
       50                  55                  60
   Met Tyr Leu Gly Arg Val Ala Ala Val Asn Gly Gly Val Thr Ile Asn
   65                  70                  75                  80
   Ala Pro Ala Leu Thr Val Asn Arg Leu Cys Gly Ser Gly Leu Gln Ala
                   85                  90                  95
   Ile Val Ser Ala Ala Gln Thr Ile Leu Leu Gly Asp Thr Asp Val Ala
               100                 105                 110
   Ile Gly Gly Gly Ala Glu Ser Met Ser Arg Ala Pro Tyr Leu Ala Pro
           115                 120                 125
   Ala Ala Arg Trp Gly Ala Arg Met Gly Asp Ala Gly Leu Val Asp Met
       130                 135                 140
   Met Leu Gly Ala Leu His Asp Pro Phe His Arg Ile His Met Gly Val
   145                 150                 155                 160
   Thr Ala Glu Asn Val Ala Lys Glu Tyr Asp Ile Ser Arg Ala Gln Gln
                   165                 170                 175
   Asp Glu Ala Ala Leu Glu Ser His Arg Arg Ala Ser Ala Ala Ile Lys
               180                 185                 190
   Ala Gly Tyr Phe Lys Asp Gln Ile Val Pro Val Val Ser Lys Gly Arg
           195                 200                 205
   Lys Gly Asp Val Thr Phe Asp Thr Asp Glu His Val Arg His Asp Ala
       210                 215                 220
   Thr Ile Asp Asp Met Thr Lys Leu Arg Pro Val Phe Val Lys Glu Asn
   225 230 235 240
   Gly Thr Val Thr Ala Gly Asn Ala Ser Gly Leu Asn Asp Ala Ala Ala
   245 250 255
   Ala Val Val Met Met Glu Arg Ala Glu Ala Glu Arg Arg Gly Leu Lys
   260 265 270
   Pro Leu Ala Arg Leu Val Ser Tyr Gly His Ala Gly Val Asp Pro Lys
   275 280 285
   Ala Ala Gly Ile Gly Pro Val Pro Ala Thr Lys Ile Ala Leu Glu Arg
   290 295 300
   Ala Gly Leu Gln Val Ser Asp Leu Asp Val Ile Glu Ala Asn Glu Ala
   305 310 315 320
   Phe Ala Ala Gln Ala Cys Ala Val Thr Lys Ala Leu Gly Leu Asp Pro
   325 330 335
   Ala Lys Val Asn Pro Asn Gly Ser Gly Ile Ser Leu Gly His Pro Ile
   340 345 350
   Gly Ala Thr Gly Ala Leu Ile Thr Val Lys Ala Leu His Glu Leu Asn
   355 360 365
   Arg Val Gln Gly Arg Tyr Ala Leu Val Thr Met Cys Ile Gly Gly Gly
   370 375 380
   Gln Gly Ile Ala Ala Ile Phe Glu Arg Ile
   385 390
<210> 8
   <211> 394
   <212> PRT
   <213> Ralstonia eutropha
<400> 8
   Met Thr Arg Glu Val Val Val Val Ser Gly Val Arg Thr Ala Ile Gly
   1 5 10 15
   Thr Phe Gly Gly Ser Leu Lys Asp Val Ala Pro Ala Glu Leu Gly Ala
   20 25 30
   Leu Val Val Arg Glu Ala Leu Ala Arg Ala Gln Val Ser Gly Asp Asp
   35 40 45
   Val Gly His Val Val Phe Gly Asn Val Ile Gln Thr Glu Pro Arg Asp
       50                  55                  60
   Met Tyr Leu Gly Arg Val Ala Ala Val Asn Gly Gly Val Thr Ile Asn
   65                  70                  75                  80
   Ala Pro Ala Leu Thr Val Asn Arg Leu Cys Gly Ser Gly Leu Gln Ala
                   85                  90                  95
   Ile Val Ser Ala Ala Gln Thr Ile Leu Leu Gly Asp Thr Asp Val Ala
               100                 105                 110
   Ile Gly Gly Gly Ala Glu Ser Met Ser Arg Ala Pro Tyr Leu Ala Pro
           115                 120                 125
   Ala Ala Arg Trp Gly Ala Arg Met Gly Asp Ala Gly Leu Val Asp Met
       130                 135                 140
   Met Leu Gly Ala Leu His Asp Pro Phe His Arg Ile His Met Gly Val
   145                 150                 155                 160
   Thr Ala Glu Asn Val Ala Lys Glu Tyr Asp Ile Ser Arg Ala Gln Gln
                   165                 170                 175
   Asp Glu Ala Ala Leu Glu Ser His Arg Arg Ala Ser Ala Ala Ile Lys
               180                 185                 190
   Ala Gly Tyr Phe Lys Asp Gln Ile Val Pro Val Val Ser Lys Gly Arg
           195                 200                 205
   Lys Gly Asp Val Thr Phe Asp Thr Asp Glu His Val Arg His Asp Ala
       210                 215                 220
   Thr Ile Asp Asp Met Thr Lys Leu Arg Pro Val Phe Val Lys Glu Asn
   225                 230                 235                 240
   Gly Thr Val Thr Ala Gly Asn Ala Ser Gly Leu Asn Asp Ala Ala Ala
                   245                 250                 255
   Ala Val Val Met Met Glu Arg Ala Glu Ala Glu Arg Arg Gly Leu Lys
               260                 265                 270
   Pro Leu Ala Arg Leu Val Ser Tyr Gly His Ala Gly Val Asp Pro Lys
           275                 280                 285
   Ala Leu Gly Ile Gly Pro Val Pro Ala Thr Lys Ile Ala Leu Glu Arg
       290                 295                 300
   Ala Gly Leu Gln Val Ser Asp Leu Asp Val Ile Glu Ala Asn Glu Ala
   305 310 315 320
   Phe Ala Ala Gln Ala Cys Ala Val Thr Lys Ala Leu Gly Leu Asp Pro
   325 330 335
   Ala Lys Val Asn Pro Asn Gly Ser Gly Ile Ser Leu Gly His Pro Ile
   340 345 350
   Gly Ala Thr Gly Ala Leu Ile Thr Val Lys Ala Leu His Glu Leu Asn
   355 360 365
   Arg Val Gln Gly Arg Tyr Ala Leu Val Thr Met Cys Ile Gly Gly Gly
   370 375 380
   Gln Gly Ile Ala Ala Ile Phe Glu Arg Ile
   385 390
<210> 9
   <211> 364
   <212> PRT
   <213> Ascaris suum
<400> 9
   Met Ala Thr Ser Arg Leu Val Cys Ser Asn Leu Thr Lys Gln Cys Phe
   1 5 10 15
   Thr Ile Ser Ser Arg Ala Ala Ser Gln Phe Thr Val Phe Phe Gly Cys
   20 25 30
   Val Val Pro Ser Asn Cys Gly Gln Gly Pro Ala Arg Gln Ala Thr Leu
   35 40 45
   Gly Ala Gly Cys Asp Pro Ser Thr Ile Val Thr Thr Leu Asn Lys Leu
   50 55 60
   Cys Ala Ser Gly Met Lys Ser Ile Ala Cys Ala Ala Ser Leu Leu Gln
   65 70 75 80
   Leu Gly Leu Gln Glu Val Thr Val Gly Gly Gly Met Glu Ser Met Ser
   85 90 95
   Leu Val Pro Tyr Tyr Leu Glu Arg Gly Glu Thr Thr Tyr Gly Gly Met
   100 105 110
   Lys Leu Ile Asp Gly Ile Pro Arg Asp Gly Leu Thr Asp Ala Tyr Ser
   115 120 125
   Asn Gln Leu Met Gly Ala Cys Ala Asp Asn Val Ala Lys Arg Phe Asn
   130 135 140
   Ile Thr Arg Glu Glu Gln Asp Lys Phe Ala Ile Glu Ser Tyr Lys Arg
   145 150 155 160
   Ser Ala Ala Ala Trp Glu Ser Gly Ala Cys Lys Ala Glu Val Val Pro
   165 170 175
   Ile Glu Val Thr Lys Gly Lys Lys Thr Tyr Ile Val Asp Lys Asp Glu
   180 185 190
   Glu Tyr Thr Lys Val Asn Phe Glu Lys Leu Pro Lys Leu Lys Pro Ala
   195 200 205
   Phe Leu Lys Asp Gly Thr Ile Thr Ala Gly Asn Ala Ser Thr Leu Asn
   210 215 220
   Asp Gly Ala Ala Ala Val Val Met Thr Thr Val Glu Gly Ala Lys Lys
   225 230 235 240
   Tyr Gly Val Lys Pro Leu Ala Arg Leu Leu Ser Tyr Gly Asp Ala Ala
   245 250 255
   Thr Asn Pro Val Asp Phe Ala Ile Ala Pro Ser Met Val Ile Pro Lys
   260 265 270
   Val Leu Lys Leu Ala Asn Leu Glu Ile Lys Asp Ile Asp Leu Trp Glu
   275 280 285
   Ile Asn Glu Ala Phe Ala Val Val Pro Leu His Ser Met Lys Thr Leu
   290 295 300
   Gly Ile Asp His Ser Lys Val Asn Ile His Gly Gly Gly Val Ser Leu
   305 310 315 320
   Gly His Pro Ile Gly Met Ser Gly Ala Arg Ile Ile Val His Leu Ile
   325 330 335
   His Ala Leu Lys Pro Gly Gln Lys Gly Cys Ala Ala Ile Cys Asn Gly
   340 345 350
   Gly Gly Gly Ala Gly Gly Met Val Ile Glu Lys Leu
   355 360
<210> 10
   <211> 392
   <212> PRT
   <213> Chryseobacterium aquaticum
<400> 10
   Met Lys Glu Val Phe Ile Val Ser Ala Val Arg Thr Pro Met Gly Ser
   1               5                   10                  15
   Phe Met Gly Ser Leu Ser Thr Val Pro Ala Thr Lys Leu Gly Ala Thr
               20                  25                  30
   Ala Val Lys Gly Ala Leu Asp Lys Ile Ser Leu Asp Pro Lys Asn Val
           35                  40                  45
   Gln Glu Ile Tyr Met Gly Asn Val Leu Gln Ala Gly Glu Gly Gln Ala
       50                  55                  60
   Pro Ala Arg Gln Val Ala Leu Gly Ala Gly Leu Ser Asn Glu Thr Pro
   65                  70                  75                  80
   Ser Thr Thr Ile Asn Lys Val Cys Ala Ser Gly Met Lys Ala Val Thr
                   85                  90                  95
   Met Ala Ala Gln Ala Ile Lys Ala Gly Asp Ala Asp Val Ile Val Ala
               100                 105                 110
   Gly Gly Met Glu Asn Met Ser Met Val Pro His Tyr Tyr Asn Ala Arg
           115                 120                 125
   Asn Ala Thr Lys Leu Gly Asp Val Lys Met Gln Asp Gly Met Val Leu
       130                 135                 140
   Asp Gly Leu Thr Asp Val Tyr Asn Lys Val His Met Gly Val Cys Ala
   145                 150                 155                 160
   Glu Lys Cys Ala Thr Asp Tyr Ser Phe Ser Arg Glu Asp Gln Asp Asn
                   165                 170                 175
   Phe Ala Ile Glu Ser Tyr Lys Arg Ser Ala Lys Ala Trp Ser Glu Gly
               180                 185                 190
   Lys Phe Ala Glu Glu Val Val Pro Val Thr Ile Pro Gln Arg Lys Gly
           195                 200                 205
   Asp Pro Ile Ile Phe Ala Glu Asp Glu Glu Tyr Lys Ala Val Asn Phe
       210                 215                 220
   Asp Arg Leu Pro Thr Leu Pro Thr Val Phe Lys Lys Glu Glu Gly Thr
   225                 230                 235                 240
   Val Thr Ala Ala Asn Ala Ser Thr Leu Asn Asp Gly Ala Ser Ala Leu
   245 250 255
   Val Leu Val Ser Lys Glu Lys Met Glu Glu Leu Gly Leu Lys Pro Leu
   260 265 270
   Ala Lys Ile Ile Ser Tyr Ala Asp Ala Ala His Glu Pro Glu Asn Phe
   275 280 285
   Thr Thr Ala Pro Ser Lys Ala Leu Pro Ile Ala Leu Lys Lys Ala Gly
   290 295 300
   Leu Glu Val Ser Asp Ile Asp Phe Phe Glu Phe Asn Glu Ala Phe Ser
   305 310 315 320
   Val Val Gly Leu Ala Asn Asn Lys Ile Leu Gly Leu Asp Ala Ala Lys
   325 330 335
   Val Asn Val Asn Gly Gly Ala Val Ala Leu Gly His Pro Leu Gly Ser
   340 345 350
   Ser Gly Ser Arg Ile Ile Val Thr Leu Ile Asn Val Leu Lys Gln Asn
   355 360 365
   Asn Gly Lys Tyr Gly Ala Ala Ala Ile Cys Asn Gly Gly Gly Gly Ala
   370 375 380
   Ser Ala Ile Val Ile Glu Asn Ile
   385 390
<210> 11
   <211> 398
   <212> PRT
   <213> Zygosaccharomyces rouxii
<400> 11
   Met Ser Asp Asn Val Tyr Val Val Ala Ser Cys Arg Thr Pro Val Gly
   1 5 10 15
   Ser Phe Gln Gly Ser Leu Ala Ser Lys Thr Ala Ile Glu Leu Gly Thr
   20 25 30
   Ala Ala Val Lys Gly Ala Ile Ser Lys Val Pro Gln Leu Lys Pro Glu
   35 40 45
   Thr Asp Tyr Asp Glu Ile Ile Phe Gly Asn Val Leu Ser Ala Asn Ser
   50 55 60
   Gly Gln Ala Pro Ala Arg Gln Val Ala Leu Gly Ala Gly Leu His Asn
   65                  70                  75                  80
   His Ile Val Asp Thr Thr Val Asn Lys Val Cys Ala Ser Ala Leu Arg
                   85                  90                  95
   Ser Ile Ile Leu Gly Ala Gln Ser Ile Lys Cys Gly Thr Ser Asp Val
               100                 105                 110
   Val Val Ala Gly Gly Cys Glu Ser Met Thr Asn Thr Pro Tyr Tyr Met
           115                 120                 125
   Pro Ala Ala Arg Ser Gly Ala Arg Phe Gly Glu Thr Lys Leu Val Asp
       130                 135                 140
   Gly Ile Gln Arg Asp Gly Leu Asn Asp Ala Tyr Asp Gly Leu Ala Met
   145                 150                 155                 160
   Gly Val His Ala Glu Lys Cys Ala Arg Asp Trp Asp Cys Ser Arg Gln
                   165                 170                 175
   Asp Gln Asp Glu Phe Ala Ile Ser Ser Tyr Gln Lys Ala Gln Lys Ala
               180                 185                 190
   Gln Asn Glu Gly Lys Phe Asp Lys Glu Ile Val Pro Ile Thr Ile Lys
           195                 200                 205
   Gly Phe Arg Gly Lys Pro Asp Thr Gln Val Thr Lys Asp Glu Glu Pro
       210                 215                 220
   Ser Lys Leu Asn Val Glu Lys Leu Lys Ser Ala Arg Thr Val Phe Gln
   225                 230                 235                 240
   Lys Glu Asn Gly Thr Val Thr Ala Pro Asn Ala Ser Pro Leu Asn Asp
                   245                 250                 255
   Gly Ala Ala Ala Val Ile Leu Val Ser Glu Lys Lys Leu Lys Gln Leu
               260                 265                 270
   Gly Leu Lys Pro Ile Ala Val Ile Arg Gly Trp Gly Glu Ala Ala His
           275                 280                 285
   Glu Pro Ala Asp Phe Thr Trp Ala Pro Ser Leu Ala Val Pro Lys Ala
       290                 295                 300
   Leu Thr His Ala Gly Ile Lys Asp Ile Gly Gln Ile Asp Phe Phe Glu
   305 310 315 320
   Phe Asn Glu Ala Phe Ser Val Val Gly Leu Ala Asn Thr Lys Ile Leu
   325 330 335
   Lys Ile Asp Pro Ser Lys Val Asn Val Tyr Gly Gly Ala Val Ala Leu
   340 345 350
   Gly His Pro Leu Gly Cys Ser Gly Ala Arg Ile Val Val Thr Leu Leu
   355 360 365
   Ser Val Leu Glu Gln Glu Asn Gly Lys Leu Gly Ala Ala Ala Ile Cys
   370 375 380
   Asn Gly Gly Gly Gly Ala Ser Ser Ile Val Leu Glu Lys Leu
   385 390 395
<210> 12
   <211> 391
   <212> PRT
   <213> Pseudomonas pelagia
<400> 12
   Met Ser Leu Asn Pro Arg Asp Val Val Ile Val Asp Phe Gly Arg Thr
   1               5                   10                  15
   Pro Met Gly Arg Ser Lys Gly Gly Met Tyr Arg Asn Val Arg Ala Glu
               20                  25                  30
   Asn Leu Ser Ala Asp Leu Ile Thr Gly Leu Leu Ala Arg Asn Pro Lys
           35                  40                  45
   Val Asp Pro Ala Glu Val Glu Asp Val Ile Trp Gly Cys Val Asn Gln
       50                  55                  60
   Thr Leu Glu Gln Gly Trp Asn Val Ala Arg Met Ile Ser Leu Leu Thr
   65                  70                  75                  80
   Pro Ile Pro His Thr Ser Ser Ala Gln Thr Val Ser Arg Leu Cys Gly
                   85                  90                  95
   Ser Ser Met Ser Ala Leu His Thr Ala Ala Gln Ala Ile Gln Thr Gly
               100                 105                 110
   Asn Gly Asp Val Phe Val Val Gly Gly Val Glu His Met Gly His Val
           115                 120                 125
   Gly Met Met His Gly Val Asp Pro Asn Pro Ser Leu Ser Leu Tyr Ala
           130                 135                 140
   Ala Lys Ala Ser Gly Met Met Gly Leu Thr Ala Glu Met Leu Gly Lys
   145                 150                 155                 160
   Met His Gly Ile Ser Arg Glu Gln Gln Asp Gln Phe Ala Leu Arg Ser
                   165                 170                 175
   His Gln Leu Ala His Lys Ala Thr Leu Asn Gly Gly Phe Lys Asp Glu
               180                 185                 190
   Ile Ile Pro Met His Gly His Asp Ala Asp Gly Phe Leu Arg Val Phe
           195                 200                 205
   Ser Glu Asp Glu Thr Ile Arg Pro Glu Thr Thr Leu Glu Ser Leu Ala
       210                 215                 220
   Ala Leu Arg Pro Ala Phe Asn Pro Lys Gly Gly Thr Val Thr Ala Gly
   225                 230                 235                 240
   Ser Ser Ser Gln Ile Thr Asp Gly Ala Ser Cys Met Ile Val Met Ser
                   245                 250                 255
   Ala Glu Arg Ala Lys Ala Leu Gly Leu Glu Pro Met Ala Val Ile Arg
               260                 265                 270
   Ser Met Ala Val Ala Gly Val Asp Pro Ala Ile Met Gly Tyr Gly Pro
           275                 280                 285
   Val Pro Ala Thr His Lys Ala Leu Lys Arg Ala Gly Met Thr Met Asp
       290                 295                 300
   Asn Val Asp Phe Val Glu Leu Asn Glu Ala Phe Ala Ala Gln Ala Leu
   305                 310                 315                 320
   Pro Val Leu Lys Asp Leu Lys Leu Leu Asp Lys Met Glu Ala Lys Val
                   325                 330                 335
   Asn Leu His Gly Gly Ala Ile Ala Leu Gly His Pro Phe Gly Cys Ser
               340                 345                 350
   Gly Ala Arg Ile Ser Gly Thr Leu Leu Asn Val Met Lys Gln Asn Gly
           355                 360                 365
   Gly Thr Val Gly Val Ser Thr Met Cys Ile Gly Leu Gly Gln Gly Ile
       370                 375                 380
   Thr Thr Val Phe Glu Arg Val
   385 390
<210> 13
   <211> 400
   <212> PRT
   <213> Thermus igniterrae
<400> 13
   Met Pro Glu Ala Trp Ile Ile Gln Ala Val Arg Thr Pro Ile Gly Lys
   1 5 10 15
   His Gly Gly Ala Leu Ala Pro Val Arg Pro Asp Asp Leu Leu Ala His
   20 25 30
   Ala Leu Ser Ala Leu Met Glu Arg Ala Gly Val Pro Lys Glu Glu Val
   35 40 45
   Glu Asp Val Tyr Ala Gly Cys Ala Asn Gln Ala Gly Glu Asp Asn Arg
   50 55 60
   Asn Val Ala Arg Met Ala Leu Leu Leu Ala Gly Phe Pro Val Glu Val
   65 70 75 80
   Ala Gly Cys Thr Val Asn Arg Leu Cys Gly Ser Gly Leu Glu Ala Val
   85 90 95
   Ala Gln Ala Ala Arg Ala Ile Trp Ala Gly Glu Gly Gln Val Tyr Val
   100 105 110
   Gly Ala Gly Val Glu Ser Met Ser Arg Ala Pro Phe Val Val Pro Lys
   115 120 125
   Ala Glu Arg Pro Phe Pro Thr Gly Asn Leu Val Met Tyr Asp Thr Thr
   130 135 140
   Leu Gly Trp Arg Leu Val Asn Pro Arg Met Gln Ala Leu Tyr Gly Thr
   145 150 155 160
   Glu Ser Met Gly Glu Thr Ala Glu Asn Leu Ala Glu Met Tyr Gly Ile
   165 170 175
   Pro Arg Glu Glu Gln Asp Arg Phe Ala Leu Leu Ser His Gln Lys Ala
   180 185 190
   Val Arg Ala Trp Asp Glu Gly Arg Phe Pro Glu Glu Val Val Pro Val
   195 200 205
   Pro Val Arg Arg Gly Lys Glu Glu Val Leu Val Ala Val Asp Glu Gly
       210                 215                 220
   Pro Arg Arg Asp Thr Ser Leu Glu Ala Leu Ala Gln Leu Arg Pro Val
   225                 230                 235                 240
   Phe Arg Gln Gly Gly Thr Val Thr Ala Gly Asn Ser Ser Pro Leu Asn
                   245                 250                 255
   Asp Gly Ala Ala Ala Leu Leu Leu Val Ser Asp Ala Tyr Ala Arg Ala
               260                 265                 270
   His Gly Leu Arg Pro Leu Ala Arg Val Arg Ser Val Ala Val Ala Gly
           275                 280                 285
   Val Pro Pro Arg Ile Met Gly Ile Gly Pro Val Pro Ala Thr Arg Lys
       290                 295                 300
   Ala Leu Glu Arg Ala Gly Leu Thr Leu Gly Asp Met Gly Leu Ile Glu
   305                 310                 315                 320
   Leu Asn Glu Ala Phe Ala Ala Gln Ser Leu Ala Val Leu Arg Glu Trp
                   325                 330                 335
   Gly Leu Ser Pro Glu Asp Pro Arg Leu Asn Pro Asn Gly Gly Ala Ile
               340                 345                 350
   Ala Leu Gly His Pro Leu Gly Ala Ser Gly Ala Arg Ile Leu Thr Thr
           355                 360                 365
   Leu Val Tyr Glu Met Arg Arg Arg Gly Val Pro Leu Gly Leu Ala Thr
       370                 375                 380
   Met Cys Ile Gly Val Gly Gln Gly Ile Ala Met Val Val Glu Ala Val
   385                 390                 395                 400
<210> 14
   <211> 394
   <212> PRT
   <213> Ralstonia solanacearum
<400> 14
   Met Ala Arg Glu Val Val Val Val Ser Gly Val Arg Thr Ala Ile Gly
   1               5                   10                  15
   Thr Phe Gly Gly Ser Leu Lys Asp Val Ala Pro Cys Glu Leu Gly Ala
               20                  25                  30
   Leu Val Val Arg Glu Ala Leu Ala Arg Ala Gln Val Lys Gly Glu Glu
           35                  40                  45
   Val Gly His Val Val Phe Gly His Val Ile Asn Thr Glu Pro Arg Asp
       50                  55                  60
   Met Tyr Leu Ser Arg Val Ala Ala Leu Asp Gly Gly Val Pro Ala Glu
   65                  70                  75                  80
   Thr Pro Ala Phe Asn Val Asn Arg Leu Cys Gly Ser Gly Leu Gln Ala
                   85                  90                  95
   Val Val Ser Ala Ala Gln Thr Ile Leu Leu Gly Asp Val Asp Val Ala
               100                 105                 110
   Ile Gly Gly Gly Ala Glu Ser Met Ser Arg Ala Pro Tyr Leu Ala Pro
           115                 120                 125
   Ala Ala Arg Trp Gly Ser Arg Met Gly Asp Ala Lys Leu Val Asp Met
       130                 135                 140
   Met Leu Gly Ala Leu His Asp Pro Phe His Ala Ile His Met Gly Val
   145                 150                 155                 160
   Thr Ala Glu Asn Val Ala Arg Glu Tyr Gly Ile Ser Arg Glu Leu Gln
                   165                 170                 175
   Asp Gln Thr Ala Leu Glu Ser His Arg Arg Ala Ser Arg Ala Ile Gln
               180                 185                 190
   Ala Gly Tyr Phe Lys Glu Gln Ile Val Pro Val Thr Ile Lys Ser Arg
           195                 200                 205
   Lys Gly Asp Val Gln Phe Asp Thr Asp Glu His Val Arg His Asp Val
       210                 215                 220
   Thr Ile Glu Asp Met Thr Arg Leu Lys Pro Val Phe Ala Lys Glu Asn
   225                 230                 235                 240
   Gly Thr Val Thr Ala Gly Asn Ala Ser Gly Leu Asn Asp Ala Ala Ala
                   245                 250                 255
   Ala Leu Val Leu Met Glu Arg Ser Leu Ala Glu Lys Arg Gly Leu Lys
               260                 265                 270
   Pro Leu Ala Arg Leu Val Ser Tyr Gly His Ala Gly Val Asp Pro Asn
           275                 280                 285
   Met Met Gly Ile Gly Pro Val Pro Ala Thr Arg Lys Ala Leu Glu Arg
       290                 295                 300
   Ala Gly Leu Ser Val Asn Asp Leu Asp Val Val Glu Ala Asn Glu Ala
   305                 310                 315                 320
   Phe Ala Ala Gln Ala Cys Ala Val Thr Gln Gln Leu Gly Leu Asp Pro
                   325                 330                 335
   Ala Lys Val Asn Pro Asn Gly Ser Gly Ile Ser Leu Gly His Pro Ile
               340                 345                 350
   Gly Ala Thr Gly Ala Leu Ile Thr Val Lys Ala Leu His Glu Leu Gln
           355                 360                 365
   Arg Ile Gly Gly Arg Tyr Ala Leu Val Thr Met Cys Ile Gly Gly Gly
       370                 375                 380
   Gln Gly Ile Ala Ala Val Phe Glu Arg Leu
   385                 390
<210> 15
   <211> 394
   <212> PRT
   <213> Citrobacter braakii
<400> 15
   Met Lys Asn Cys Val Ile Val Ser Ala Ala Arg Thr Ala Ile Gly Ser
   1               5                   10                  15
   Phe Asn Gly Ala Leu Ala Thr Thr Gly Ala Val Glu Leu Gly Ser Ile
               20                  25                  30
   Val Ile Arg Ala Ala Leu Glu Arg Ala His Leu Asp Pro Gln Arg Val
           35                  40                  45
   Asp Glu Val Ile Met Gly Asn Val Leu Gln Ser Gly Leu Gly Gln Asn
       50                  55                  60
   Pro Ala Arg Gln Ala Leu Leu Lys Ser Gly Leu Ser Asp Thr Val Cys
   65                  70                  75                  80
   Gly Phe Thr Val Asn Lys Val Cys Gly Ser Gly Leu Lys Ser Val Ala
                   85                  90                  95
   Leu Ala Ala Leu Ala Ile Lys Ala Gly Gln Ala Gln Ala Ile Val Ala
               100                 105                 110
   Gly Gly Met Glu Asn Met Ser Leu Ala Pro Tyr Leu Leu Asp Ala Lys
           115                 120                 125
   Ala Arg Trp Gly Tyr Arg Leu Gly Asp Gly Gln Leu Ser Asp Val Ile
       130                 135                 140
   Leu Arg Asp Gly Leu Ile Cys Ala Glu His Gly Tyr His Met Gly Ile
   145                 150                 155                 160
   Thr Ala Glu Asn Val Ala Lys Glu Tyr Gly Ile Ser Arg Glu Met Gln
                   165                 170                 175
   Asp Glu Leu Ala Leu Gln Ser Gln Arg Lys Ala Val Ala Ala Ile Glu
               180                 185                 190
   Ser Gly Ala Phe Glu Ala Glu Ile Val Pro Val Ser Val Ser Ser Arg
           195                 200                 205
   Lys Lys Thr Ile Ile Val Glu Arg Asp Glu Phe Pro Lys Ala Asp Ser
       210                 215                 220
   Thr Thr Glu Gly Leu Ala Ala Leu Arg Pro Ala Phe Asp Lys Ala Gly
   225                 230                 235                 240
   Thr Val Thr Ala Gly Asn Ala Ser Gly Ile Asn Asp Gly Ala Ala Ala
                   245                 250                 255
   Leu Val Ile Met Glu Glu Ser Ala Ala Leu Glu Ala Gly Leu Lys Pro
               260                 265                 270
   Leu Ala Arg Ile Lys Ala Trp Ala Ser Gly Gly Val Ala Pro Ala Met
           275                 280                 285
   Met Gly Met Gly Pro Val Pro Ala Thr Leu Lys Ala Leu Gln Leu Ser
       290                 295                 300
   Gly Leu Gln Leu Ser Asp Ile Asp Leu Ile Glu Ala Asn Glu Ala Phe
   305                 310                 315                 320
   Ala Ala Gln Phe Leu Ala Val Gly Lys Thr Leu Gly Phe Asp Pro Gln
                   325                 330                 335
   Lys Val Asn Val Asn Gly Gly Ala Ile Ala Leu Gly His Pro Ile Gly
               340                 345                 350
   Ala Ser Gly Ala Arg Ile Leu Val Thr Leu Leu His Ala Leu Gln Ala
           355                 360                 365
   Arg Asp Gln Thr Leu Gly Leu Ala Thr Leu Cys Ile Gly Gly Gly Gln
   370 375 380
   Gly Ile Ala Met Ile Val Glu Arg Met Asn
   385 390
<210> 16
   <211> 392
   <212> PRT
   <213> Zoogloea ramigera
<400> 16
   Met Ser Thr Pro Ser Ile Val Ile Ala Ser Ala Arg Thr Ala Val Gly
   1 5 10 15
   Ser Phe Asn Gly Ala Phe Ala Asn Thr Pro Ala His Glu Leu Gly Ala
   20 25 30
   Thr Val Ile Ser Ala Val Leu Glu Arg Ala Gly Val Ala Ala Gly Glu
   35 40 45
   Val Asn Glu Val Ile Leu Gly Gln Val Leu Pro Ala Gly Glu Gly Gln
   50 55 60
   Asn Pro Ala Arg Gln Ala Ala Met Lys Ala Gly Val Pro Gln Glu Ala
   65 70 75 80
   Thr Ala Trp Gly Met Asn Gln Leu Cys Gly Ser Gly Leu Arg Ala Val
   85 90 95
   Ala Leu Gly Met Gln Gln Ile Ala Thr Gly Asp Ala Ser Ile Ile Val
   100 105 110
   Ala Gly Gly Met Glu Ser Met Ser Met Ala Pro His Cys Ala His Leu
   115 120 125
   Ala Gly Gly Val Lys Met Gly Asp Phe Lys Met Ile Asp Thr Met Ile
   130 135 140
   Lys Asp Gly Leu Thr Asp Ala Phe Tyr Gly Tyr His Met Gly Thr Thr
   145 150 155 160
   Ala Glu Asn Val Ala Lys Gln Trp Gln Leu Ser Arg Asp Glu Gln Asp
   165 170 175
   Ala Phe Ala Val Ala Ser Gln Asn Lys Ala Glu Ala Ala Gln Lys Asp
   180 185 190
   Gly Arg Phe Lys Asp Glu Ile Val Pro Phe Ile Val Lys Gly Arg Lys
   195 200 205
   Gly Asp Ile Thr Val Asp Ala Asp Glu Tyr Ile Arg His Gly Ala Thr
   210 215 220
   Leu Asp Ser Met Ala Lys Leu Arg Pro Ala Phe Asp Lys Glu Gly Thr
   225 230 235 240
   Val Thr Ala Gly Asn Ala Ser Gly Leu Asn Asp Gly Ala Ala Ala Ala
   245 250 255
   Leu Leu Met Ser Glu Ala Glu Ala Ser Arg Arg Gly Ile Gln Pro Leu
   260 265 270
   Gly Arg Ile Val Ser Trp Ala Thr Val Gly Val Asp Pro Lys Val Met
   275 280 285
   Gly Thr Gly Pro Ile Pro Ala Ser Arg Lys Ala Leu Glu Arg Ala Gly
   290 295 300
   Trp Lys Ile Gly Asp Leu Asp Leu Val Glu Ala Asn Glu Ala Phe Ala
   305 310 315 320
   Ala Gln Ala Cys Ala Val Asn Lys Asp Leu Gly Trp Asp Pro Ser Ile
   325 330 335
   Val Asn Val Asn Gly Gly Ala Ile Ala Ile Gly His Pro Ile Gly Ala
   340 345 350
   Ser Gly Ala Arg Ile Leu Asn Thr Leu Leu Phe Glu Met Lys Arg Arg
   355 360 365
   Gly Ala Arg Lys Gly Leu Ala Thr Leu Cys Ile Gly Gly Gly Met Gly
   370 375 380
   Val Ala Met Cys Ile Glu Ser Leu
   385 390
<210> 17
   <211> 393
   <212> PRT
   <213> Neorhizobium galegae
<400> 17
   Met Ser Asn Pro Ser Ile Val Ile Ala Ala Ala Gly Arg Thr Ala Val
   1 5 10 15
   Gly Ser Phe Asn Gly Ala Phe Ala Asn Val Pro Ala His Glu Leu Gly
               20                  25                  30
   Ala Ala Ala Ile Lys Gly Ala Leu Ala Arg Ala Gly Val Glu Ala Ala
           35                  40                  45
   Glu Val Asp Glu Val Ile Leu Gly Gln Val Leu Pro Ala Gly Glu Gly
       50                  55                  60
   Gln Asn Pro Ala Arg Gln Ala Ala Met Lys Ala Gly Val Pro Lys Glu
   65                  70                  75                  80
   Ala Thr Ala Trp Gly Ile Asn Gln Leu Cys Gly Ser Gly Leu Arg Ala
                   85                  90                  95
   Val Ala Leu Gly Met Gln Gln Ile Ala Leu Gly Asp Ala Lys Ile Ile
               100                 105                 110
   Val Ala Gly Gly Gln Glu Ser Met Ser Met Ala Pro His Cys Gly His
           115                 120                 125
   Leu Arg Gly Gly Val Lys Met Gly Asp Phe Lys Met Ile Asp Thr Met
       130                 135                 140
   Ile Lys Asp Gly Leu Thr Asp Ala Phe Tyr Gly Tyr His Met Gly Ile
   145                 150                 155                 160
   Thr Ala Glu Asn Ile Ala Arg Gln Trp Gln Leu Ser Arg Glu Glu Gln
                   165                 170                 175
   Asp Gln Phe Ala Val Ser Ser Gln Asn Lys Ala Glu Ala Ala Gln Asn
               180                 185                 190
   Ala Gly Arg Phe Lys Asp Glu Ile Ile Pro Tyr Ile Ile Gln Thr Arg
           195                 200                 205
   Lys Gly Asp Val Thr Val Glu Asn Asp Glu Tyr Ile Arg Ala Gly Ala
       210                 215                 220
   Thr Leu Asp Ser Met Ala Lys Leu Arg Pro Ala Phe Asp Lys Glu Gly
   225                 230                 235                 240
   Thr Val Thr Ala Gly Asn Ala Ser Gly Leu Asn Asp Gly Ala Ala Ala
                   245                 250                 255
   Ala Val Leu Met Thr Glu Ala Glu Ala Ala Arg Arg Gly Ile Thr Pro
                   260                 265                 270
   Met Val Arg Ile Val Ser Trp Ala Thr Ala Gly Val Asp Pro Gln Ile
           275                 280                 285
   Met Gly Thr Gly Pro Ile Pro Ala Ser Arg Lys Ala Leu Glu Lys Ala
       290                 295                 300
   Gly Trp Lys Val Gly Asp Leu Asp Leu Ile Glu Ala Asn Glu Ala Phe
   305                 310                 315                 320
   Ala Ala Gln Ser Cys Ala Val Thr Lys Asp Leu Gly Phe Asp Pro Ser
                   325                 330                 335
   Leu Val Asn Val Asn Gly Gly Ala Ile Ala Ile Gly His Pro Ile Gly
               340                 345                 350
   Ala Ser Gly Ala Arg Ile Leu Asn Thr Leu Leu Phe Glu Met Lys Arg
           355                 360                 365
   Arg Gly Ala Lys Lys Gly Leu Ala Thr Leu Cys Ile Gly Gly Gly Met
       370                 375                 380
   Gly Val Ala Met Cys Leu Glu Ala Met
   385                 390